# EUROPEAN PATENT SPECIFICATION

(11) **EP 1 862 557 B1**
(45) Date of publication and mention of the grant of the patent: **18.05.2011**
(21) Application number: 06290862.9
(22) Date of filing: 29.05.2006
(51) Int. Cl.: C12Q 1/68

(54) **Real-time multiplex detection of three bacterial species responsible for sexually-transmitted diseases**
Echtzeit-Multiplex-Nachweis von drei Bakterienarten verantwortlich für sexuell übertragbare Krankheiten
Détection multiplex en temps réel de trois espèces bactériennes responsables de maladies à transmission sexuelle

(43) Date of publication of application: 05.12.2007
(73) Proprietor: Bio-Rad Pasteur, 92430 Marnes-la-Coquette (FR)
(72) Inventor: Clarebout, Gervais, 14550 Blainville sur Orne (FR)
(74) Representative: Paris, Fabienne

(56) References cited:
- WO-A-2004/040013
- WO-A2-98/11259
- WO-A2-2004/024944
- US-A- 5 310 650
- JALAL H. ET AL: "Development and validation of a rotor-gene real-time PCR assay for detection, identification, and quantification of Chlamydia trachomatis in a single reaction" JOURNAL OF CLINICAL MICROBIOLOGY, vol. 44, no. 1, January 2006 (2006-01), pages 206-213, XP002404059
- BLAYLOCK M W ET AL: "Determination of Infectious Load of Mycoplasma genitalium in Clinical Samples of Human Vaginal Cells" JOURNAL OF CLINICAL MICROBIOLOGY, WASHINGTON, DC, US, vol. 42, no. 2, February 2004 (2004-02), pages 746-752, XP002360337 ISSN: 0095-1137
- GERAATS-PETERS C W M ET AL: "SPECIFIC AND SENSITIVE DETECTION OF NEISSERIA GONORRHOEA IN CLINICAL SPECIMENS BY REAL-TIME PCR" JOURNAL OF CLINICAL MICROBIOLOGY, WASHINGTON, DC, US, vol. 43, no. 11, November 2005 (2005-11), pages 5653-5659, XP008058231 ISSN: 0095-1137

## Description

### TECHNICAL FIELD:

The present invention relates to the detection of three different bacterial species which are responsible for sexually-transmitted diseases, i.e., *Chlamydia trachomatis* (CT), *Neisseria gonorrhoeae* (NG) and *Mycoplasma genitalium* (MG).

The present invention more particularly relates to the detection of these three species in real-time PCR, in multiplex PCR or in real-time multiplex PCR.

### BACKGROUND OF THE INVENTION:

*Chlamydia trachomatis* (CT) is a species of the chlamydiae, a group of obligately intracellular bacteria. It causes sexually transmitted diseases, such as chlamydia and lymphogranuloma venereum, as well as trachoma, an eye infection that is a frequent cause of blindness.

*Neisseria gonorrhoeae* (NG) is a species of Gram-negative bacteria responsible for the disease gonorrhoea.

CT and NG co-infections are frequent.

*Mycoplasma genitalium* (MG) is a parasitic bacterium which lives in the primate genital and respiratory tracts. MG is thought to be involved in urethritis.

Traditional diagnosis of the presence of CT and/or MG and/or NG involves the culture of samples collected from patients, such as urethral specimen, on species-specific culture media. Such cultures are time-consuming and fastidious. They are all the most time-consuming and fastidious in the case of CT, MG and NG, because the culture of CT and MG requires a high level of technicality, and because NG is very sensitive to temperature and humidity variations.

Diagnosis methods based on nucleic acid amplification have therefore been developed.

For example, the FDA-approved Amplicor^{™} kit available from Roche Diagnostic enables the detection of CT or NG. However, it does not enable to detect MG.

WO 98/11259 in the name of Visible Genetics Inc. discloses a method for co-amplification and detection of CT, MG and NG. This method involves the amplification of CT, MG and NG targets by amplification primers. The detection of the amplicons produced by said primers is carried out either by direct labelling of the primers, or by agarose gel techniques. The method of WO 98/11259 does however not involve the use of amplicon-annealing probes (*cf*. pages 9-10 of WO 98/11259). Hence, the method of WO 98/11259 is not a real-time technique.

The present invention enables the detection of CT, MG and NG in real-time amplification, and provides primers as well as probes, preferably beacon probes, which can be used together in multiplex in the same tube to detect the three bacterial species in real-time amplification.

As an advantageous feature, the invention may thus be implemented in real-time PCR, in multiplex PCR, or in multiplex real-time PCR.

### SUMMARY OF THE INVENTION:

The invention relates to the detection of three different bacterial species which are responsible for sexually-transmitted diseases, i.e., *Chlamydia trachomatis* (CT), *Neisseria gonorrhoeae* (NG) and *Mycoplasma genitalium* (MG).

The invention more particularly relates to the detection of these three species in real-time PCR, in multiplex PCR and in real-time multiplex PCR.

The application provides reference templates sequences, which are especially adapted to the design of primers and probes, which can be used together in the same tube to detect CT and/or MG and/or NG, advantageously CT and MG and NG, by real-time multiplex amplification, and the invention provides a method of production of such primers and probes from said reference template sequences.

The invention also relates to these primers and probes, as well as to pharmaceutical compositions, to biological compositions, to detection kits and to diagnostic kits, which comprise at least one of primers and/or probes of the invention.

Table 1, which is at the end of Example 1 below, lists SEQ ID sequences, which are representative of said reference template polynucleotides,and of primers and probes of the invention.

The invention further relates to a process for the detection of CT, MG and NG, which involves the use of at least one primer pair and/or at least one probe of the invention, as well as to amplification compositions.

To the best of the inventors' knowledge, the invention provides the first description of a detection of CT and MG and NG in real-time multiplex amplification.

### BRIEF DESCRIPTION OF THE FIGURES:

Figure 1 shows the sequence, which is available under accession number J03321 (SEQ ID NO: 1), which is the pCHL1 plasmid sequence of CT.
Figure 2 shows the sequence, which is available under accession number X91074 (SEQ ID NO: 2), which is the sequence of the 5' region of the adhesin gene of MG.
Figure 3 shows the sequence, which is available under accession number M31431 (SEQ ID NO: 3), which is the sequence of the adhesin gene of MG.
Figure 4 shows the sequence, which is available under accession number AF042097 (SEQ ID NO: 4), which is the sequence of the *pil*E gene of NG.

### DETAILED DESCRIPTION:

The invention relates to the detection of three sexually-transmittable bacterial species, namely: *Chlamydia trachomatis* (CT), *Mycoplasma genitalium* (MG) and *Neisseria gonorrhoeae* (NG).

The application provides nucleic acid reference template sequences, which allow for the construction and production of primers and probes, which are suitable for the detection of CT and/or MG and/or NG in real-time multiplex amplification.

The invention thus provides CT primers, CT primer pairs, CT probes, MG primers, MG primer pairs, MG probes, NG primers, NG primer pairs and NG probes, which are suitable for the detection of CT and/or MG and/or NG in real-time multiplex amplification.

The primers of the invention can be mixed together in multiplex in the same tube to amplify CT and MG and NG in said same tube.

Advantageously, the invention provides probes, which are suitable for real-time detection in such multiplex operative conditions.

Hence, the primers and probes of the invention can be mixed together in multiplex in the same tube to amplify and detect CT and MG and NG in real-time in said same tube. Hence, the invention enables the detection of CT and MG and NG in one single (amplification+detection) operative step.

To the best of the inventors' knowledge, the invention is the first description of a real-time multiplex detection of the three bacterial species (CT, MG, NG).

In addition to detecting the three bacterial species within the same sample, the invention has the advantage of allowing checking for the absence of Taq polymerase inhibitors, by the use of an Internal Control (IC). IC, as well as IC primers and probes, are described in more details below.

The invention therefore allows for a quadruplex amplification (CT, MG, NG and IC).

The invention provides for a detection of said three bacterial species, which is much faster than any prior art technique, as well as much reliable as it heavily reduces the risk of having contaminated samples or cultures for analysis.

The means of the invention further are very sensitive and reproducible (see example 4 below).

At present time, there is no systemic detection of MG, whereas it is now acknowledged that MG is responsible for non-gonococcal urethritis, and is often associated to cervicitis and endometritis.

The invention provides the first means that allow for a systemic detection of MG in a routine test allowing the simultaneous detection of CT and NG.

The invention thereby allows the physician to avoid the prescription of inappropriate antibiotics.

More particularly, the invention provides:
- CT real-time amplification systems, wherein each CT real-time amplification system comprises at least two CT primers of the invention and at least one CT probe of the invention,
- MG real-time amplification systems, wherein each MG real-time amplification system comprises at least two MG primers of the invention and at least one MG probe of the invention, and
- NG real-time amplification systems, wherein each NG real-time amplification system comprises at least two NG primers and at least one NG probe.

Still more particularly, the invention provides CT real-time amplification systems, MG real-time amplification systems, and NG real-time amplification systems, which allow for a detection of CT, MG and NG, respectively, which is specific of the bacterial species to which the real-time amplification system is intended.

Advantageously, the invention provides CT real-time amplification systems, MG real-time amplification systems, and NG real-time amplification systems, which can be used together in multiplex in the same tube, without any significant loss in specificity: even when used together in multiplex in the same tube,
- such a CT real-time amplification system of the invention still specifically detects CT, without any significant cross-reactivity with the real-time amplification systems of MG and NG, or with the amplicons that may possibly be produced by these MG and NG systems,
- such a MG real-time amplification system of the invention still specifically detects MG, without any significant cross-reactivity with the real-time amplification systems of CT and NG, or with the amplicons that may possibly be produced by these CT and NG systems,
- such a NG real-time amplification system of the invention still specifically detects NG, without any significant cross-reactivity with the real-time amplification systems of CT and MG, or with the amplicons that may possibly be produced by these CT and MG systems.

To the best of the inventors' knowledge, none of the real-time amplification systems of the invention detects human DNA (no cross-hybridization).

The application also relates to pharmaceutical compositions, to biological compositions, to detection kits and to diagnostic kits, which comprise at least one of the primers and/or probes of the invention, preferably at least two primers and at least one probe of the invention, more preferably at least one real-time amplification system of the invention, most preferably at least one CT and at least one MG and at least one NG real-time amplification systems of the invention.

The invention also relates to a process for the detection of at least one *Chlamydia trachomatis* (CT) and/or at least one *Mycoplasma genitalium* (MG) and/or at least one *Neisseria gonorrhoeae* (NG).

Said detection is usually performed in a sample.

By "sample containing nucleic acid material", it is meant any sample, which contains at least one nucleic acid, e.g., a biological sample, such as a sample which has been collected from a cell culture, or from an animal or a human being, preferably a sample which has been collected from a tissue or fluid that is suspected of containing CT and/or MG and/or NG, such as e.g., a sample of uterine cervix, most preferably a urine sample (such as a first void urine sample).

Advantageously, the invention enables a reliable detection of CT and/or MG and/or NG is a urine sample.

Said sample may optionally have been further treated and/or purified according to any technique known by the skilled person, to improve the amplification efficiency and/or qualitative accuracy and/or quantitative accuracy. The sample may thus exclusively, or essentially, consist of nucleic acid(s), whether obtained by purification, isolation, or by chemical synthesis. Means are available to the skilled person, who would like to isolate or purify nucleic acids, such as DNA, from a biological sample, for example to isolate or purify DNA from cervical scrapes (e.g., QIAamp-DNA Mini-Kit; Qiagen, Hilden, Germany).

Said detection comprises the determination of whether at least one amplicon has been, or is, produced from said sample, or from nucleic acid material thereof, by amplification by means of amplification primers,
whereby a positive determination indicates that at least one CT and/or at least one MG and/or at least one NG is(are) present in said sample.

Said determination can be carried out by means of at least one probe, which is intended to anneal to said at least one amplicon.

The detection process of the invention may thus comprise:
- contacting said sample, or nucleic acid material thereof, with at least two amplification primers, under conditions suitable for the production of at least one amplicon by said primers (i.e., under conditions, which would be suitable for the production by said primers of at least one amplicon from said at least one CT and/or MG and/or NG to be detected, if this CT and/or MG and/or NG were present in said sample),
- determining whether at least one amplicon has been produced, or is produced, by said primers, e.g., by means of at least one detection probe, which anneals to the amplicon produced by said at least two primers, preferably in real-time amplification involving at least one probe of the invention.

In the process of the application, said amplification primers comprise:
- at least two primers, which are intended for targeting CT, and which are suitable for the detection of CT in real-time multiplex amplification,
   and/or
- at least two primers, which are intended for targeting MG, and which are suitable for the detection of MG in real-time multiplex amplification,
   and/or
- at least two primers, which are intended for targeting NG, and which are suitable for the detection of NG in real-time multiplex amplification.

In the process of the invention, said amplification primers comprise said at least two CT-targeted primers and said at least two MG-targeted primers and said at least two NG-targeted primers.

In the process of the application, said at least one probe preferably comprise:
- at least one CT-targeted probe, which is suitable for the detection of CT in real-time multiplex amplification,
   and/or
- at least one MG-targeted probe, which is suitable for the detection of MG in real-time multiplex amplification,
   and/or
- at least one NG-targeted probe, which is suitable for the detection of NG in real-time multiplex amplification.

In the process of the invention, said at least one probe preferably comprises at least one CT-targeted probe and at least one MG-targeted probe and at least one NG-targeted probe.

Whereas the reference template sequences of the application, the primers and the probes of the invention have been optimized so as to allow for the detection of CT, MG and NG in real-time multiplex amplification, the simplex embodiments thereof are of course also encompassed by the application (e.g., real-time, or non real-time simplex embodiments, involving one primer pair of the invention).

Quite similarly, whereas the invention provides the special feature of allowing a multiplex detection of CT, MG and NG in real-time, the implementations of the primers of the invention without a probe of the invention, or with at least one probe of the invention but not in real-time, are of course also encompassed by the present application.

Also, whereas the primers of the invention have been designed as primer pairs, each primer is individually encompassed as such by the present application.

Table 1, which is at the end of Example 1 below, lists SEQ ID sequences, which are representative of reference template polynucleotides of the application, and of primers and probes of the invention.

Table 1 thereby shows two CT real-time amplification systems, five MG real-time amplification systems, and one real-time amplification system of the invention. These systems can be used together in the same tube to detect CT and MG and NG in real-time multiplex amplification.

Said at least two primers, which are intended for targeting CT, and which are suitable for the detection of CT in real-time multiplex amplification, are oligonucleotides, the sequences of which are suitable for use as forward and reverse primers, respectively, in the amplification of at least one CT reference template sequence, wherein said at least one CT reference template sequence is a fragment consisting of positions 5571-5760 (SEQ ID NO: 5) of the CT sequence of SEQ ID NO: 1 (CT pCHL1 plasmid; J03321), or of a conservative sub-fragment thereof, which has retained the property of being a suitable reference template sequence, to construct and produce CT-targeted primers, which allow for a real-time multiplex detection of CT. Said at least two primers, which are intended for targeting CT, preferably consist of 14-30 nucleotides (each independently from each other).

Said at least one CT reference template sequence advantageously is the fragment consisting of positions 5580-5754 (SEQ ID NO: 6) of the CT sequence of SEQ ID NO: 1 or the sequence that is fully complementary to said fragment over the entire length of said fragment.

Said at least two primers, which are intended for targeting MG, and which are suitable for the detection of MG in real-time multiplex amplification, are oligonucleotides, the sequences of which are suitable for use as forward and reverse primers, respectively, in the amplification of at least one MG reference template sequence, wherein said at least one MG reference template sequence is a fragment consisting of:
- positions 1-270 (SEQ ID NO: 13) of the MG sequence of SEQ ID NO: 2 (5'region of MG adhesin gene; X91074), or of a conservative sub-fragment thereof, which has retained the property of being a suitable reference template sequence, to construct and produce MG-targeted primers, which allow for a real-time multiplex detection of MG,
- positions 1140-1290 (SEQ ID NO: 19) of the MG sequence of SEQ ID NO: 3 (MG adhesion gene; M31431), or of a conservative sub-fragment thereof, which has retained the property of being a suitable reference template sequence, to construct and produce MG-targeted primers, which allow for a real-time multiplex detection of MG,
- positions 1060-1250 (SEQ ID NO: 25) of the MG sequence of SEQ ID NO: 3, or of a conservative sub-fragment thereof, which has retained the property of being a suitable reference template sequence, to construct and produce MG-targeted primers, which allow for a real-time multiplex detection of MG,
- positions 1520-1710 (SEQ ID NO: 31) of the MG sequence of SEQ ID NO: 3, or of a conservative sub-fragment thereof, which has retained the property of being a suitable reference template sequence, to construct and produce MG-targeted primers, which allow for a real-time multiplex detection of MG,
- positions 1500-1710 (SEQ ID NO: 37) of the MG sequence of SEQ ID NO: 3, or of a conservative sub-fragment thereof, which has retained the property of being a suitable reference template sequence, to construct and produce MG-targeted primers, which allow for a real-time multiplex detection of MG.

Said at least two primers, which are intended for targeting MG, preferably consist of 14-30 nucleotides (each independently from each other).

Said at least one MG reference template sequence advantageously is:
- the fragment consisting of positions 2-259 (SEQ ID NO: 14) of the MG sequence of SEQ ID NO:2, or the sequence that is fully complementary to said fragment over the entire length of said fragment, or
- the fragment consisting of positions 1144-1283 (SEQ ID NO:20) of the MG sequence of SEQ ID NO:3, or the sequence that is fully complementary to said fragment over the entire length of said fragment, or
- the fragment consisting of positions 1064-1249 (SEQ ID NO:26) of the MG sequence of SEQ ID NO:3, or the sequence that is fully complementary to said fragment over the entire length of said fragment, or
- the fragment consisting of positions 1527-1704 (SEQ ID NO:32) of the MG sequence of SEQ ID NO:3, or the sequence that is fully complementary to said fragment over the entire length of said fragment, or
- the fragment consisting of positions 1501-1704 (SEQ ID NO:38) of the MG sequence of SEQ ID NO:3, or the sequence that is fully complementary to said fragment over the entire length of said fragment.

Said MG reference template sequences share the specific technical feature of being suitable references to construct and produce MG-targeted primers, as well as MG-specific probes, which can be used together in multiplex in the same tube to specifically detect CT and/or MG and/or NG, advantageously CT and MG and NG, in real-time time in said same tube.

Said at least two primers, which are intended for targeting NG, and which are suitable for the detection of NG in real-time multiplex amplification, are oligonucleotides, the sequences of which are suitable for use as forward and reverse primers, respectively, in the amplification of at least one NG reference template sequence, wherein said at least one NG reference template sequence is a fragment consisting of positions 101-380 (SEQ ID NO: 42) of the NG sequence of SEQ ID NO: 4 (NG *pil*E gene; AF042097), or of a conservative sub-fragment thereof, which has retained the property of being a suitable reference template sequence, to construct and produce NG-targeted primers, which allow for a real-time multiplex detection of NG.

Said at least two primers, which are intended for targeting NG, preferably consist of 14-30 nucleotides (each independently from each other).

Said at least one NG reference template sequence advantageously is the fragment consisting of positions 114-365 (SEQ ID NO: 43) of the NG sequence of SEQ ID NO: 4, or the sequence that is fully complementary to said fragment over the entire length of said fragment.

By "consisting of 14-30 nucleotides", it is meant "consisting of 14, 15, 16, 17, 18, 19, 20, 21, 22, 23, 24, 25, 26, 27, 28, 29 or 30 nucleotides".

The same applies mutatis mutandis to any range, which is recited in the present application.

The nucleotide lengths of the primers can be chosen independently from each other.

By "suitable for use as forward and reverse primers, respectively, in the amplification of" a reference template sequence "consisting of" an indicated sequence or SEQ ID, it is herein meant that these forward and reverse primers do not flank the reference template sequence, but that they anneal in the exact terminal positions that allow for the sequence that would be amplified to consist of the indicated reference template sequence.

More preferably, a primer of the invention consists of 14-30 nucleotides, the sequence of which has an identity of at least 80%, preferably of at least 85%, more preferably of at least 90%, even more preferably of at least 92%, still even more preferably of at least 95%, most preferably of at least 97%, with a sequence of the same length contained at the very 3' end or at the very 5' end of its reference template sequence or conservative sub-fragment thereof, or of the sequence that is fully complementary to said reference template sequence or conservative sub-fragment thereof over the entire length of this reference template sequence or complementary sequence thereof.

For example, a primer pair of the invention may consist of a forward primer and a reverse primer, which each independently consists of 14-30 nucleotides,
wherein the sequence of the forward primer has an identity of at least 80%, preferably of at least 85%, more preferably of at least 90%, even more preferably of at least 92%, still even more preferably of at least 95%, most preferably of at least 97%, with a sequence of the same length contained at the very 5' end of its reference template sequence or conservative sub-fragment thereof, and
wherein the sequence of the reverse primer has an identity of at least 80%, preferably of at least 85%, more preferably of at least 90%, even more preferably of at least 92%, still even more preferably of at least 95%, most preferably of at least 97%, with a sequence of the same length contained at the very 5' end of the sequence that is fully complementary to said reference template sequence or conservative sub-fragment thereof, over the entire length of said reference template sequence or complementary sequence thereof.

A primer of the invention preferably consists of 14-28, more preferably of 15-28, even more preferably of 16-27, still even more preferably of 16-26, most preferably of 17-25 nucleotides, e.g., of 17, 18, 19, 20, 21, 22, 23, 24 or 25 nucleotides.

Said at least two CT-targeted primers preferably are one oligonucleotide of SEQ ID NO: 7 (forward primer), or a conservative variant thereof, and one oligonucleotide of SEQ ID NO: 12 (reverse primer), or a conservative variant thereof.

Said at least two MG-targeted primers preferably are:
- one oligonucleotide of SEQ ID NO: 15 (forward primer), or a conservative variant thereof, and one oligonucleotide of SEQ ID NO:18 (reverse primer), or a conservative variant thereof, or are
- at least one oligonucleotide selected from the group consisting of SEQ ID NO: 21; 27; 33; 39, or a conservative variant thereof, and at least one oligonucleotide selected from the group consisting of SEQ ID NO: 24; 30; 36, or a conservative variant thereof.

Said at least two MG-targeted primers can for example be at least one oligonucleotide selected from the group consisting of SEQ ID NO: 21; 27, or a conservative variant thereof, and at least one oligonucleotide selected from the group consisting of SEQ ID NO: 24; 30; 36, or a conservative variant thereof.

Said at least two MG-targeted primers preferably are one oligonucleotide of SEQ ID NO: 21, or a conservative variant thereof, and at least one oligonucleotide selected from the group consisting of SEQ ID NO: 24; 36, or a conservative variant thereof.

More preferably, said at least two MG-targeted primers are one oligonucleotide of SEQ ID NO: 21, or a conservative variant thereof and one oligonucleotide of SEQ ID NO: 24, or a conservative variant thereof.

More preferably, said at least two MG-targeted primers are one oligonucleotide of SEQ ID NO: 27, or a conservative variant thereof, and at least one oligonucleotide selected from the group consisting of SEQ ID NO: 24; 30; 36, or a conservative variant thereof. Most preferably, said at least two MG-targeted primers are one oligonucleotide of SEQ ID NO: 27, or a conservative variant thereof, and one oligonucleotide of SEQ ID NO: 30, or a conservative variant thereof.

Said at least two MG-targeted primers preferably are one oligonucleotide of SEQ ID NO: 36, or a conservative variant thereof, and at least one oligonucleotide selected from the group consisting of SEQ ID NO: 21; 27; 33 and 39, or a conservative variant thereof.

Most preferably, said at least two MG-targeted primers are one oligonucleotide of SEQ ID NO: 33 and one oligonucleotide of SEQ ID NO: 36; or are one of SEQ ID NO: 39 and one oligonucleotide of SEQ ID NO: 36.

Said at least two NG-targeted primers preferably are one oligonucleotide of SEQ ID NO: 44, or a conservative variant thereof, and one oligonucleotide of SEQ ID NO: 47, or a conservative variant thereof.

Conservative variants of such primers more particularly comprise those variant primers, the sequence of which has an identity of at least 80%, preferably of at least 85%, more preferably of at least 90%, even more preferably of at least 92%, still even more preferably of at least 95%, most preferably of at least 97%, with at least one of the above-mentioned SEQ ID primer sequences.

Said at least one CT- or MG- or NG-targeted probe, which is suitable for the detection of CT or MG or NG, respectively, in real-time multiplex amplification is an oligonucleotide, the sequence of which is suitable for use as a probe for the detection of at least one amplicon produced from CT or MG or NG, respectively, by said at least two CT- or MG- or NG-targeted primers.

The sequence of such a CT- or MG- or NG-targeted probe hence necessarily differs from the sequence of the CT-, MG-, NG-targeted primers.

Advantageously, said at least one CT-targeted probe is a CT-specific probe that anneals to CT amplicons, without annealing to NG or MG amplicons under conditions of at least moderate stringency.

Advantageously, said at least one MG-targeted probe is a MG -specific probe that anneals to MG amplicons, without annealing to CT or NG amplicons under conditions of at least moderate stringency.

Advantageously, said at least one NG-targeted probe is a NG-specific probe that anneals to NG amplicons, without annealing to CT or MG amplicons under conditions of at least moderate stringency.

Said at least one CT-specific probe preferably is an oligonucleotide of 15-60 nucleotides, which is sufficiently complementary to a fragment of the same size of said CT reference template sequence, or to a fragment of the same size of the sequence that is fully complementary to said CT reference template sequence over the entire length of said reference template sequence, to anneal to said CT reference template sequence or complementary sequence thereof, under conditions of at least moderate stringency, but which is not sufficiently complementary to any fragment of the same size of said MG or NG reference template sequence, or of the sequence that is fully complementary to said MG or NG reference template sequence over the entire length of said MG or NG reference template sequence, to anneal to said MG or NG reference template sequence or complementary sequence thereof, under the same stringency conditions.

Said at least one MG-specific probe preferably is an oligonucleotide of 15-60 nucleotides, which is sufficiently complementary to a fragment of the same size of said MG reference template sequence, or to a fragment of the same size of the sequence that is fully complementary to said MG reference template sequence over the entire length of said reference template sequence, to anneal to said MG reference template sequence or complementary sequence thereof, under conditions of at least moderate stringency, but which is not sufficiently complementary to any fragment of the same size of said CT or NG reference template sequence, or of the sequence that is fully complementary to said CT or NG reference template sequence over the entire length of said CT or NG reference template sequence, to anneal to said CT or NG reference template sequence or complementary sequence thereof, under the same stringency conditions.

Said at least one NG-specific probe preferably is an oligonucleotide of 15-60 nucleotides, which is sufficiently complementary to a fragment of the same size of said NG reference template sequence, or to a fragment of the same size of the sequence that is fully complementary to said NG reference template sequence over the entire length of said reference template sequence, to anneal to said NG reference template sequence or complementary sequence thereof, under conditions of at least moderate stringency, but which is not sufficiently complementary to any fragment of the same size of said CT or MG reference template sequence, or of the sequence that is fully complementary to said MG or NG reference template sequence over the entire length of said CT or MG reference template sequence, to anneal to said CT or MG reference template sequence or complementary sequence thereof, under the same stringency conditions.

More preferably, said at least one CT-specific probe is a fragment of at least 15 nucleotides of said CT reference template sequence, or of the sequence that is fully complementary to said CT reference template sequence over the entire length of said reference template sequence,
wherein said fragment does not anneal to said MG or NG reference template sequences under conditions of at least moderate stringency.

More preferably, said at least one MG-specific probe is a fragment of at least 15 nucleotides of said MG reference template sequence, or of the sequence that is fully complementary to said MG reference template sequence over the entire length of said reference template sequence,
wherein said fragment does not anneal to said CT or NG reference template sequences under conditions of at least moderate stringency.

More preferably, said at least one NG-specific probe is a fragment of at least 15 nucleotides of said NG reference template sequence, or of the sequence that is fully complementary to said NG reference template sequence over the entire length of said reference template sequence,
wherein said fragment does not anneal to said CT or MG reference template sequences under conditions of at least moderate stringency.

The expression "conditions of at least moderate stringency" is intended to mean conditions of moderate, high or very high stringency.

The expressions "moderate stringency", "high stringency" and "very high stringency" are given their ordinary meaning in the field.

Stringency refers to hybridization conditions chosen to optimize binding of polynucleotide sequences with different degrees of complementarity. Stringency is affected by factors such as temperature, salt conditions, the presence of organic solvents in the hybridization mixtures, and the lengths and base compositions of the sequences to be hybridized and the extent of base mismatching, and the combination of parameters is more important than the absolute measure of any one factor.

Illustrative conditions of moderate stringency comprise:
- hybridization to filter-bound DNA in 5xSSC, 2% sodium dodecyl sulfate (SDS), 100 microgrammes/mL single stranded DNA at 55-65°C for 8 hours, and washing in 0.2xSSC and 0.2% SDS at 50-55°C for thirty minutes.

Illustrative conditions of high stringency comprise:
- hybridization to filter-bound DNA in 5xSSC, 2% sodium dodecyl sulfate (SDS), 100 microgrammes/mL single stranded DNA at 55-65°C for 8 hours, and washing in 0.2xSSC and 0.2% SDS at 60-65°C for thirty minutes.

Illustrative conditions of very high stringency comprise:
- hybridization to filter-bound DNA in 5xSSC, 2% sodium dodecyl sulfate (SDS), 100 microgrammes /mL single stranded DNA at 55-65°C for 8 hours, and washing in 0.1xSSC and 0.1% SDS at 60-65°C for thirty minutes.

Most preferably, said at least one CT-specific probe, which is suitable for the detection of CT in real-time multiplex amplification, preferably is:
i. a fragment of at least 15 nucleotides of said CT reference template sequence, or of the sequence that is fully complementary to said CT reference template sequence over the entire length of said reference template sequence,or
ii. a conservative variant thereof, which derives from a fragment of i. by deletion and/or substitution and/or addition of at least one nucleotide, but which has retained the capacity of being a CT-specific probe, wherein said fragment does not anneal to said NG or MG reference template sequences, e.g., a conservative variant of a fragment of i., the sequence of which has at least 90% identity with a fragment of i. over the entire length of said fragment of i.

Most preferably, said at least one MG-specific probe, which is suitable for the detection of MG in real-time multiplex amplification, preferably is:
iii. a fragment of at least 15 nucleotides of said MG reference template sequence, or of the sequence that is fully complementary to said MG reference template sequence over the entire length of said reference template sequence, or
iv. a conservative variant thereof, which derives from a fragment of iii. by deletion and/or substitution and/or addition of at least one nucleotide, but which has retained the capacity of being a MG-specific probe, wherein said fragment does not anneal to said CT or NG reference template sequences, e.g., a conservative variant of a fragment of iii., the sequence of which has at least 90% identity with a fragment of iii. over the entire length of said fragment of iii.

Most preferably, said at least one NG-specific probe, which is suitable for the detection of NG in real-time multiplex amplification, preferably is:
v. a fragment of at least 15 nucleotides of said NG reference template sequence, or of the sequence that is fully complementary to said NG reference template sequence over the entire length of said reference template sequence,or
vi. a conservative variant thereof, which derives from a fragment of v. by deletion and/or substitution and/or addition of at least one nucleotide, but which has retained the capacity of being a NG-specific probe, wherein said fragment does not anneal to said CT or MG reference template sequences, e.g., a conservative variant of a fragment of v., the sequence of which has at least 90% identity with a fragment of v. over the entire length of said fragment of v. (global alignment, also referred to as "needle" alignment).

Said percentage of identity preferably is of at least 91 %, more preferably of at least 92%, even more preferably of at least 93%, still more preferably of at least 94%, most preferably of at least 95%, e.g., 95%, at least 96%, at least 97%, at least 98%, or at least 99%.

A probe of the invention comprises at least 15 nucleotides. For example, it consists of 15-60 nucleotides. A probe of the invention preferably consists of 15-50, more preferably of 15-40, even more preferably of 15-30, still more preferably of 16-30, even still more preferably of 18-30, most preferably of 19-30, still most preferably of 21-29, even still most preferably of 22-27 nucleotides, e.g., 22, 23, 24, 25, 26 or 27 nucleotides.

Said at least one CT-specific probe preferably is of SEQ ID NO: 8 or 10 (CT probes SCT 175b and 175c), or is the sequence that is fully complementary to this SEQ ID sequence, over the entire length of this SEQ ID sequence.

Said at least one MG-specific probe preferably is selected from the group consisting of SEQ ID NO: 16; 22; 28; 34 and 40 (MG probes SF-MG 258c, MGBR 140c, MGBR 186j, MGBR 178q, MGBR 204u), or is the sequence that is fully complementary to this SEQ ID sequence, over the entire length of this SEQ ID sequence.

Said at least one NG-specific probe is of SEQ ID NO: 45 (NG probe pilEc), or is the sequence that is fully complementary to this SEQ ID sequence, over the entire length of this SEQ ID sequence.

A probe of the invention can be linked to at least one detection label, and/or at least one nucleotide arm that is unrelated to CT, MG and NG and that is intended to carry a quencher or a reporter (e.g., a fluorophore).

Various formats (types) of probes, including Taqman ™ probes (hydrolysis probes), molecular beacons TM (beacon probes or molecular beacon probes), and Scorpion™ probes are known in the art.

It may e.g., be linked to at least one beacon arm, or to at least one ScorpionTM arm, preferably at least one of such arms in 5' and/or 3', most preferably two of such arms, in 5' and in 3', respectively.

One of preferred formats is the beacon format. The structure of molecular beacons is as follows. A short nucleotide sequence (so-called beacon arm) which is unrelated to the target sequence is thus covalently linked to both ends of the probe. A short unrelated arm is thus linked in 5' of the probe, and is labelled with a fluorescent moiety (i.e. fluorescent dye or fluorescent marker). Another but still unrelated arm is linked to the 3' end of probe and is labelled with a fluorescence quenching moiety. Thus, molecular beacons have a fluorophore and a quencher at opposite ends. The 5' short arm is totally complementary to the one in 3' so that they can anneal together, and thus can assume a hairpin structure when unhybridized to the target in solution. In this hairpin conformation, the quencher and the fluorescent dye are close enough to each other to allow efficient quenching of the fluorophore. However, when the probe encounters a target molecule, annealing is favoured with respect to the hairpin conformation when values of beacon arm Tm and probe Tm are suitably chosen (theoretically: probe Tm > beacon arm Tm > primer Tm, wherein Tm is the melting temperature of interest). The fluorophore and quencher move away from each other and the fluorophore can then fluoresce when illuminated by suitable light excitation. As PCR proceeds, amplification product accumulates, and the amount of fluorescence at any given cycle depends on the amount of amplification product present at that time. (See e.g., Sanjay Tyagi and Fred Russell Kramer, Nature Biotechnology 1996, volume 14, pages 303-308; Nature Biotechnology 1998, volume 16, pages 49-53).

(Remark: It is also possible to link the fluorophore at the 3' end, while attaching the quencher at the 5' end.)

Schematically, said probe can have the following formulae (molecular beacon format):
5' Fluorophore-(arm1)-probe-(arm2)-Quencher 3'
5' Quencher-(arm1)-probe-(arm2)-Fluorophore 3'
wherein arm1 and arm2 can be any short nucleotide sequences, e.g. in the range of 3-10 nucleotides, preferably 5, 6, 7 nucleotides, allowing for the hair pin structure formation under suitable stringency conditions, i.e. arm1 and arm2 are totally complementary to anneal under the desired stringency conditions (standard PCR stringency conditions include, for example, an annealing temperature of 55 to 65°C and an Mg concentration of 4 to 8 mM). However, arm1 and arm2 are unrelated to the target sequence of the probe, i.e. the hairpin conformation resulting from the annealing between arm1 and arm2 is essentially the only possible secondary structure for the probe when unhybridized. The skilled person would know how to choose such arms for a given probe.

Illustrative beacon arms are given in example 1 below.

By fluorophore, it is herein understood any fluorescent marker/dye known in the art. Examples of such suitable fluorescent markers include Fam, Hex, Tet, Joe, Rox, Tamra, Max, Edans, Cy dyes such as Cy5, Fluorescein, Coumarin, Eosine, Rhodamine, Bodipy, Alexa, Cascade Blue, Yakima Yellow, Lucifer Yellow and Texas Red (all of them are Trade-Marks), the family of ATTO dyes.

By quencher, we herein understand any quencher known in the art. Examples of such quenchers include Dabcyl, Dark Quencher, Eclipse Dark Quencher, ElleQuencher, Tamra, BHQ and QSY (all of them are Trade-Marks).

The skilled person would know which combinations of dye/quencher are suitable when designing a probe.

In a preferred embodiment according to the invention, spectral properties of said probes can be chosen as to not interfere with each other. In particular, when probes are used in multiplex, each single probe can have its own fluorophore being spectrally significantly different from each other, i.e., the absorption/emission spectra are essentially nonoverlapping. This advantageously allows for low-noise multiplex detection for all single probes, making sure that individual signals do not interfere with each other in detection. Examples of dyes which can be used together in multiplex include Fam with Tamra, Fam with Tamra with Texas Red.

The choice of appropriate dyes to be used together may also be dependent of the filter contained in the amplification apparatus.

Said at least one CT-specific probe most preferably is of SEQ ID NO: 9 or 11 (CT probes SCT 175b and 175c with beacon arms), or the sequence that is fully complementary to this SEQ ID sequence, over the entire length of this SEQ ID sequence.

Said at least one MG-specific probe most preferably is selected from the group consisting of SEQ ID NO: 17; 23; 29; 35 and 41 (MG probes SF-MG 258c, MGBR 140c, MGBR 186j, MGBR 178q, MGBR 204u with beacon arms), or is the sequence that is fully complementary to this SEQ ID sequence, over the entire length of this SEQ ID sequence. Said at least one NG-specific probe most preferably is of SEQ ID NO: 46 (NG probe pilEc with beacon arms), or is the sequence that is fully complementary to this SEQ ID sequence, over the entire length of this SEQ ID sequence.

Said at least one CT- or MG- or NG- specific probe may also be an oligonucleotide, which is a conservative variant of said probe SEQ ID, as above-described, i.e., which derives from said SEQ ID sequence by deletion and/or substitution and/or addition of at least one nucleotide, but which has retained the capacity of being a CT- or MG- or NG-specific probe, e.g., a conservative variant of said SEQ ID sequence, the sequence of which has at least 90% identity with said SEQ ID sequence, over the entire length of said SEQ ID sequence (global alignment, also referred to as "needle" alignment).

One of the special technical features shared by said CT, MG and NG reference template sequences is that they are reference template sequences, which are suitable for construct and produce CT-, MG- and NG-targeted primers, as well as CT-, MG- and NG-specific probes, which can be used together in multiplex in the same tube to specifically detect at least one CT and/or at least one MG and/or at least one NG, advantageously at least one CT and at least one MG and at least one NG, in real-time time in said same tube.

Preferably, said at least two CT-targeted primers are one oligonucleotide of SEQ ID NO: 7 and one oligonucleotide of SEQ ID NO: 12, and said at least one CT-specific probe is selected from the group consisting of SEQ ID NO: 8; 9; 10; 11.

Preferably, said at least two MG-targeted primers are one oligonucleotide of SEQ ID NO: 15 and one oligonucleotide of SEQ ID NO: 18, and said at least one MG-specific probe is of SEQ ID NO: 16 or 17, or is the sequence that is fully complementary to this SEQ ID sequence, over the entire length of this SEQ ID sequence.

Preferably, said at least two MG-targeted primers are one oligonucleotide of SEQ ID NO: 21 and one oligonucleotide of SEQ ID NO: 24, and said at least one MG-specific probe is of SEQ ID NO: 22 or 23, or is the sequence that is fully complementary to this SEQ ID sequence, over the entire length of this SEQ ID sequence.

Preferably, said at least two MG-targeted primers are one oligonucleotide of SEQ ID NO: 27 and one oligonucleotide of SEQ ID NO: 30, and said at least one MG-specific probe is of SEQ ID NO: 28 or 29, or is the sequence that is fully complementary to this SEQ ID sequence, over the entire length of this SEQ ID sequence.

Preferably, said at least two MG-targeted primers are one oligonucleotide of SEQ ID NO: 33 and one oligonucleotide of SEQ ID NO: 36, and said at least one MG-specific probe is of SEQ ID NO: 34 or 35, or is the sequence that is fully complementary to this SEQ ID sequence, over the entire length of this SEQ ID sequence.

Preferably, said at least two MG-targeted primers are one oligonucleotide of SEQ ID NO: 39 and one oligonucleotide of SEQ ID NO: 36, and said at least one MG-specific probe is of SEQ ID NO: 40 or 41, or is the sequence that is fully complementary to this SEQ ID sequence, over the entire length of this SEQ ID sequence.

Preferably, said at least two NG-targeted primers are one oligonucleotide of SEQ ID NO: 44 and one oligonucleotide of SEQ ID NO: 47, and said at least one NG-specific probe is of SEQ ID NO: 45 or 46, or is the sequence that is fully complementary to this SEQ ID sequence, over the entire length of this SEQ ID sequence.

Advantageously, in accordance with the invention, said amplification primers can comprise at least two CT-targeted primers, and at least two MG-targeted primers, and at least two NG-targeted primers.

Preferably:
- said at least two CT-targeted primers are one oligonucleotide of SEQ ID NO: 7 and one oligonucleotide of SEQ ID NO: 12, and
- said at least two MG-targeted primers are one oligonucleotide of SEQ ID NO: 15 and one oligonucleotide of SEQ ID NO: 18; or one oligonucleotide of SEQ ID NO: 21 and one oligonucleotide of SEQ ID NO: 24; or one oligonucleotide of SEQ ID NO: 27 and one oligonucleotide of SEQ ID NO: 30; or one oligonucleotide of SEQ ID NO: 33 and one oligonucleotide of SEQ ID NO: 36; or one oligonucleotide of SEQ ID NO: 39 and one oligonucleotide of SEQ ID NO: 36, and
- said at least two NG-targeted primers are one oligonucleotide of SEQ ID NO: 44 and one oligonucleotide of SEQ ID NO: 47.

Advantageously, in accordance with the invention, said at least one probe can comprise at least one CT-specific probe and at least one MG-specific probe and at least one NG-specific probe.

Preferably:
- said at least two CT-targeted primers are one oligonucleotide of SEQ ID NO: 7 and one oligonucleotide of SEQ ID NO: 12, and said at least one CT-specific probe is of SEQ ID NO: 8, 9, 10 or 11, or is the sequence that is fully complementary to this SEQ ID sequence, over the entire length of this SEQ ID sequence, and
- said at least two NG-targeted primers are one oligonucleotide of SEQ ID NO: 44 and one oligonucleotide of SEQ ID NO: 47, and said at least one NG-specific probe is of SEQ ID NO: 45 or 46, or is the sequence that is fully complementary to this SEQ ID sequence, over the entire length of this SEQ ID sequence; and
- said at least two MG-targeted primers and said at least one MG-specific probe are as follows:
   - said at least two MG-targeted primers are one oligonucleotide of SEQ ID NO: 15 and one oligonucleotide of SEQ ID NO: 18, and said at least one MG-specific probe is of SEQ ID NO: 16 or 17, or is the sequence that is fully complementary to this SEQ ID sequence, over the entire length of this SEQ ID sequence; or
   - said at least two MG-targeted primers are one oligonucleotide of SEQ ID NO: 21 and one oligonucleotide of SEQ ID NO: 24, and said at least one MG-specific probe is of SEQ ID NO: 22 or 23, or is the sequence that is fully complementary to this SEQ ID sequence, over the entire length of this SEQ ID sequence; or
   - said at least two MG-targeted primers are one oligonucleotide of SEQ ID NO: 27 and one oligonucleotide of SEQ ID NO: 30, and said at least one MG-specific probe is of SEQ ID NO: 28 or 29, or is the sequence that is fully complementary to this SEQ ID sequence, over the entire length of this SEQ ID sequence; or
   - said at least two MG-targeted primers are one oligonucleotide of SEQ ID NO: 33 and one oligonucleotide of SEQ ID NO: 36, and said at least one MG-specific probe is of SEQ ID NO: 34 or 35, or is the sequence that is fully complementary to this SEQ ID sequence, over the entire length of this SEQ ID sequence; or
   - said at least two MG-targeted primers are one oligonucleotide of SEQ ID NO: 39 and one oligonucleotide of SEQ ID NO: 36, and said at least one MG-specific probe is of SEQ ID NO: 40 or 41, or is the sequence that is fully complementary to this SEQ ID sequence, over the entire length of this SEQ ID sequence.

In accordance with an advantageous embodiment of the invention, the detection of CT and/or MG and/or NG can be made in real-time multiplex amplification.

Of course, the primers and probes of the invention are also suitable for other protocols, including simplex protocols, multiplex protocols, end-point protocols, qualitative protocols, quantitative protocols, combinations thereof, and the like.

Said amplification can be any nucleic acid amplification, which is found appropriate to the skilled person, for example a PCR (Polymerase Chain Reaction), or an isothermal amplification technique, e.g., TMA (transcription mediated amplification), NASBA (nucleic acid sequence based amplification), 3SR (self sustained sequence replication) or strand displacement amplification.

Said amplification preferably is PCR.

In a preferred embodiment, the primers according to the invention are used in a final concentration range 20 - 2000 nM. Typically, said primers can be used at a final concentration range of 20-1300 nM, preferably of 20-1250 nM, more preferably of 25-1250 nM, e.g., of about 25, 125, 250, 500, 850, 1250 nM.

Probe concentration in a PCR reaction can be optimized, typically by varying the final concentration from 50 nM to 1000 nM. In a preferred embodiment, each probe according to the invention is used at a final concentration range of 75-300nM, preferably 75-250 nM, more preferably 100-250 nM, even more preferably 150-200nM, e.g., of about 150 nM or about 200 nM.

Appropriate amplification conditions are known to those skilled in the art. They include temperature conditions, in particular thermal cycling conditions, e.g., temperature, duration, number, heating rate of the cycles. In a preferred embodiment, said temperature conditions include conditions suitable for a PCR. In another preferred embodiment, said conditions include conditions suitable for a Q-PCR.

In accordance with the invention, an internal control (IC), which is unrelated to CT and/or MG and/or NG can also be implemented, such as the IC of SEQ ID NO: 48.

An appropriate primer pair for amplification of said IC comprises the primer pair of SEQ ID NO: 49 and 52. An appropriate IC probe comprises the probe of SEQ ID NO: 50 (SEQ ID NO: 51 in beacon format).

These IC, IC primers and IC probes are also encompassed individually as such by the application.

A 16S rRNA primer pair may also be implemented, such as, e.g., the primer pair of SEQ ID NO: 53 and 54. This system is used in order to detect the presence of bacterial DNA in sample.

The application also describes an amplicon obtainable by implementation of the process of the invention on a CT- and/or MG- and/or NG-containing sample.

The invention relates to any amplification composition obtainable by implementation of the process of the invention on a CT- and/or MG- and/or NG-containing sample, and further comprising:
- said CT-targeted primers, MG-targeted primers and NG-targeted primers as defined in the process of the invention; or
- a primer system of the invention, which comprises three pairs of oligonucleotides, wherein:
   o for each one of said three oligonucleotide pairs:
      ■ the oligonucleotides are of 14-30 nucleotides each, and
      ■ the sequence of the sequence of one oligonucleotide of said pair being at least 80% identical to the sequence of the same length that is the exact 5' terminal end of a reference template sequence, and the sequence of the other oligonucleotide of said pair being at least 80% identical to the sequence of the same length that is the exact 5' terminal end of the sequence that is fully complementary to said reference template sequence over the entire length of said reference template sequence,
   o and wherein said reference template sequence is:
      ■ for one of said three pairs: SEQ ID NO: 6;
      ■ for another one of said three pairs: SEQ ID NO: 14 or SEQ ID NO: 20 or SEQ ID NO: 26 or SEQ ID NO: 32 or SEQ ID NO: 38;
      ■ for still another one of said three pairs: SEQ ID NO: 43.

The application also describes the primers and probes as such, i.e., as individual oligonucleotide products.

According to the application and to the invention, all the provided oligonucleotides can be either kept separately, or partially mixed, or totally mixed.

Said oligonucleotides can be provided under dry form, or solubilized in a suitable solvent, as judged by the skilled person. Suitable solvents include TE, PCR-grade water, and the like.

The application further describes every product that is herein described, and more particularly to every reference template polynucleotide, to every primer and to every probe, as an individual product.

The application also describes every possible combination that can be made of at least two products of the invention, preferably of at least three products of the invention, such as, e.g., of at least two primers of the invention and at least one probe of the invention.

The application thus describes a polynucleotide suitable for use as a reference template sequence in the design of primers and probes that can be used in the same tube for the detection of CT and MG and NG in real-time multiplex amplification, wherein said polynucleotide is selected from:
- for the design of CT primers and probes: a fragment consisting of positions 5571-5760 (SEQ ID NO: 5) of the CT sequence of SEQ ID NO: 1 (CT pCHL1 plasmid; J03321), or of a conservative sub-fragment thereof, which has retained the property of being a suitable reference template sequence, to construct and produce CT-targeted primers, which allow for a real-time multiplex detection of CT, or a sequence that is fully complementary to said fragment or sub-fragment over the entire length of said fragment or sub-fragment,
- for the design of MG primers and probes: a fragment consisting of:
   - positions 1-270 (SEQ ID NO: 13) of the MG sequence of SEQ ID NO: 2 (5' region of MG adhesin gene; X91074), or of a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment,
   - positions 1140-1290 (SEQ ID NO: 19) of the MG sequence of SEQ ID NO: 3 (MG adhesion gene; M31431), or of a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment,
   - positions 1060-1250 (SEQ ID NO: 25) of the MG sequence of SEQ ID NO: 3, or of a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment,
   - positions 1520-1710 (SEQ ID NO: 31) of the MG sequence of SEQ ID NO: 3, or of a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment,
   - positions 1500-1710 (SEQ ID NO: 37) of the MG sequence of SEQ ID NO: 3, or of a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment,
- for the design of NG primers and probes: a fragment consisting of positions 101-380 (SEQ ID NO: 42) of the NG sequence of SEQ ID NO: 4 (NG *pil*E gene; AF042097), or of a conservative sub-fragment thereof, which has retained the property of being a suitable reference template sequence, to construct and produce NG-targeted primers, which allow for a real-time multiplex detection of NG, or a sequence that is fully complementary to said fragment or sub-fragment over the entire length of said fragment or sub-fragment.

Preferably, said reference template polynucleotide is:
- for the design of CT primers and probes: the fragment consisting of positions 5580-5754 (SEQ ID NO: 6) of the CT sequence of SEQ ID NO:1, or a sequence that is fully complementary to said fragment over the entire length of said fragment,
- for the design of MG primers and probes:
   - the fragment consisting of positions 2-259 (SEQ ID NO: 14) of the MG sequence of SEQ ID NO:2, or a sequence that is fully complementary to said fragment or sub-fragment over the entire length of said fragment, or
   - the fragment consisting of positions 1144-1283 (SEQ ID NO:20) of the MG sequence of SEQ ID NO:3, or a sequence that is fully complementary to said fragment or sub-fragment over the entire length of said fragment, or
   - the fragment consisting of positions 1064-1249 (SEQ ID NO:26) of the MG sequence of SEQ ID NO:3, or a sequence that is fully complementary to said fragment or sub-fragment over the entire length of said fragment, or
   - the fragment consisting of positions 1527-1704 (SEQ ID NO:32) of the MG sequence of SEQ ID N0:3, or a sequence that is fully complementary to said fragment or sub-fragment over the entire length of said fragment, or
   - the fragment consisting of positions 1501-1704 (SEQ ID NO:38) of the MG sequence of SEQ ID N0:3, or a sequence that is fully complementary to said fragment or sub-fragment over the entire length of said fragment,
   - for the design of NG primers and probes: the fragment consisting of positions 114-365 (SEQ ID NO: 43) of the NG sequence of SEQ ID NO: 4, or a sequence that is fully complementary to said fragment or sub-fragment over the entire length of said fragment.

The invention relates to a method of production of primers and probes that can be used in the same tube for the detection of CT and MG and NG in real-time multiplex amplification, which comprises producing at least one primer pair and at least one probe, wherein one oligonucleotide of said primer pair is of 14-30 nucleotides, the sequence of which is at least 80% identical to the sequence of the same length that is the exact 5' terminal end of a reference template sequence, and the other oligonucleotide of said primer pair is of 14-30 nucleotides, the sequence of which is at least 80% identical to the sequence of the same length that is the exact 5' terminal end of the sequence that is fully complementary to said reference template sequence over the entire length of said reference template sequence,
wherein said probe is:
i. a fragment of at least 15 nucleotides of said reference template sequence, or a fragment of at least 15 nucleotides of the sequence that is fully complementary to said reference template sequence over the entire length of said reference template sequence, or
ii. a conservative variant of a fragment as defined in i., the sequence of said conservative variant being at least 90% identical to the sequence of said fragment of i. over the entire length of said fragment of i.,
   said probe being optionally linked to at least one detection label and/or at least one beacon arm,
   wherein said reference template sequence is a reference template sequence of the application.

The invention also relates to a primer, which is especially adapted to the detection of CT and/or MG and/or NG in real-time multiplex amplification, which is:
an oligonucleotide of 14-30 nucleotides, the sequence of which is at least 80% identical to the sequence of the same length that is the exact 5' terminal end of a reference template sequence or of the sequence that is fully complementary to said reference template sequence over the entire length of said reference template sequence,
wherein said reference template sequence is:
   - SEQ ID NO: 6; or
   - SEQ ID NO: 14 or SEQ ID NO: 20 or SEQ ID NO: 26 or SEQ ID NO: 32 or SEQ ID NO: 38; or
   - SEQ ID NO: 43.

The invention also relates to a primer, which is especially adapted to the detection of CT and/or MG and/or NG in real-time multiplex amplification, which is:
- a CT-targeted primer, selected from the group consisting of SEQ ID NO: 7; 12, or
- a MG-targeted primer, selected from the group consisting of SEQ ID NO: 15; 18; 21; 24; 27; 30; 33; 36; 39, or
- a NG-targeted primer, selected from the group consisting of SEQ ID NO: 44; 47,
- a conservative variant of such primers, as herein defined.

The invention also relates to a primer system, which is especially adapted to the detection of CT and/or MG and/or NG in real-time multiplex amplification, which comprises:
at least one pair of oligonucleotides of 14-30 nucleotides each,
the sequence of one oligonucleotide of said pair being at least 80% identical to the sequence of the same length that is the exact 5' terminal end of a reference template sequence, and
the sequence of the other oligonucleotide of said pair being at least 80% identical to the sequence of the same length that is the exact 5' terminal end of the sequence that is fully complementary to said reference template sequence over the entire length of said reference template sequence,
wherein said reference template sequence is:
   - SEQ ID NO: 6; or
   - SEQ ID NO: 14 or SEQ ID NO: 20 or SEQ ID NO: 26 or SEQ ID NO: 32 or SEQ ID NO: 38; or
   - SEQ ID NO: 43.

The invention also relates to a primer system, which is especially adapted to the detection of CT and/or MG and/or NG in real-time multiplex amplification, which comprises at least two CT-targeted primers and/or at least two MG-targeted primers and/or at least two NG-targeted primers, such as:
- at least one CT-targeted primer pair of SEQ ID NO: 7 and SEQ ID NO: 12, and/or
- at least one MG-targeted primer pair selected from the following pairs: SEQ ID NO: 15 and 18; SEQ ID NO: 21 and 24; SEQ ID NO: 27 and 30; SEQ ID NO: 33 and 36; SEQ ID NO: 39 and 36, and/or
- at least one NG-targeted primer pair of SEQ ID NO: 44 and 47.

The invention also relates to a primer system, which comprises three of said pairs of oligonucleotides, and wherein said reference template sequence is:
- for one of said three pairs: SEQ ID NO: 6;
- for another one of said three pairs: SEQ ID NO: 14 or SEQ ID NO: 20 or SEQ ID NO: 26 or SEQ ID NO: 32 or SEQ ID NO: 38;
- for still another one of said three pairs: SEQ ID NO: 43.

The invention also relates to a probe, which is especially adapted to the detection of CT and/or MG and/or NG in real-time multiplex amplification, which is:
- a CT-specific probe of SEQ ID NO: 8; or 10, or which consists of a sequence that is fully complementary to this SEQ ID sequence, over the entire length of this SEQ ID sequence, or
- a MG-specific probe of SEQ ID NO: 16; 22; 28; 34 or 40, or which consists of a sequence that is fully complementary to this SEQ ID sequence, over the entire length of this SEQ ID sequence, or
- a NG-specific probe of SEQ ID NO: 45, or which consists of a sequence that is fully complementary to this SEQ ID sequence, over the entire length of this SEQ ID sequence, or
- a conservative variant of such probes, as herein defined,
   said probe being optionally linked to at least one detection label and/or at least one beacon arm.

The invention also relates to a beacon probe, which is especially adapted to the detection of CT and/or MG and/or NG in real-time multiplex amplification, which is:
- a CT-specific probe of SEQ ID NO: 9 or 11, or which consists of a sequence that is fully complementary to this SEQ ID sequence, over the entire length of this SEQ ID sequence, or
- a MG-specific probe of SEQ ID NO: 17; 23; 29; 35 or 41, or which consists of a sequence that is fully complementary to this SEQ ID sequence, over the entire length of this SEQ sequence, or
- a NG-specific probe of SEQ ID NO: 45 or 46, or which consists of a sequence that is fully complementary to this SEQ ID sequence, over the entire length of this SEQ ID sequence, or
- a conservative variant of such probes.

The application also describes a primer and probe system, which is especially adapted to the detection of CT and/or MG and/or NG in real-time multiplex amplification, which comprises at least one primer of the application and at least one probe of the application, preferably at least one primer system of the application, and at least one probe system of the application.

The invention relates to a primer and probe system, which is especially adapted to the detection of CT and/or MG and/or NG in real-time multiplex amplification, which comprises at least one primer of the invention and at least one probe of the invention, preferably at least one primer system of the invention, and at least one probe of the invention.

The application also describes an amplicon, obtainable by amplification of at least one nucleic acid from CT and/or MG and/or NG, by means of at least one primer system of the invention.

The application also describes an amplification composition, comprising at least one amplicon according to the invention.

The invention relates to any amplification composition, obtainable by amplification of at least one nucleic acid from CT and/or MG and/or NG, by means of at least one primer system of the invention, and further comprising:
- said CT-targeted primers, MG-targeted primers and NG-targeted primers as defined in the process of the invention; or
- a primer system of the invention, which comprises three pairs of oligonucleotides, wherein:
   o for each one of said three oligonucleotide pairs:
      ■ the oligonucleotides are of 14-30 nucleotides each, and
      ■ the sequence of the sequence of one oligonucleotide of said pair being at least 80% identical to the sequence of the same length that is the exact 5' terminal end of a reference template sequence, and the sequence of the other oligonucleotide of said pair being at least 80% identical to the sequence of the same length that is the exact 5' terminal end of the sequence that is fully complementary to said reference template sequence over the entire length of said reference template sequence,
   o and wherein said reference template sequence is:
      ■ for one of said three pairs: SEQ ID NO: 6;
      ■ for another one of said three pairs: SEQ ID NO: 14 or SEQ ID NO: 20 or SEQ ID NO: 26 or SEQ ID NO: 32 or SEQ ID NO: 38;
      ■ for still another one of said three pairs: SEQ ID NO: 43.

The invention also relates to a kit for the diagnosis of an infection by CT and/or MG and/or NG, which comprises:
- at least one primer system of the invention, and/or
- at least one probe of the invention,
- optionally, instructions for the use thereof and/or nucleotides.

In the kit according to the invention, the oligonucleotides (primers, probes) can be either kept separately, or partially mixed, or totally mixed.

Said oligonucleotides can be provided under dry form, or solubilized in a suitable solvent, as judged by the skilled person. Suitable solvents include TE, PCR-grade water, and the like.

In a preferred embodiment, the kit according to the invention can also contain further reagents suitable for a PCR step.

Such reagents are known to those skilled in the art, and include water, like nuclease-free water, RNase free water, DNAse-free water, PCR-grade water; salts, like magnesium, potassium; buffers such as Tris; enzymes, including polymerases, such as Taq, Vent, Pfu (all of them Trade-Marks), activable polymerase, and the like; nucleotides like deoxynucleotides, dideoxunucleotides, dNTPs, dATP, dTTP, dCTP, dGTP, dUTP; other reagents, like DTT and/or RNase inhibitors; and polynucleotides like polyT, polydT, and other oligonucleotides, e.g., primers.

In another preferred embodiment, the kit according to the invention comprises PCR controls. Such controls are known in the art, and include qualitative controls, positive controls, negative controls, internal controls, quantitative controls, internal quantitative controls, as well as calibration ranges. The internal control for said PCR step can be a template which is unrelated to the target template in the PCR step. Such controls also may comprise control primers and/or control probes. For example, in the case of HPV detection, it is possible to use as an internal control, a polynucleotide chosen within a gene whose presence is excluded in a sample originating from a human body (for example, from a plant gene), and whose size and GC content is equivalent to those from the target sequence.

Illustrative internal controls comprise the IC of SEQ ID NO: 48. Appropriate IC primers thus comprise the primer of SEQ ID NO: 49 and the primer of SEQ ID NO: 50, which together form a primer pair capable of amplifying said IC of SEQ ID NO: 48. Appropriate IC probes comprise the probe of SEQ ID NO: 50 (or of SEQ ID NO: 51, in beacon format).

In a preferred embodiment, the kit according to the invention contains means for extracting and/or purifying nucleic acid from a biological sample, e.g., from urine. Such means are well known to those skilled in the art.

In a preferred embodiment, the kit according to the invention contains instructions for the use thereof. Said instructions can advantageously be a leaflet, a card, or the like. Said instructions can also be present under two forms: a detailed one, gathering exhaustive information about the kit and the use thereof, possibly also including literature data; and a quick-guide form or a memo, e.g., in the shape of a card, gathering the essential information needed for the use thereof.

The invention also relates to all the medical, biological, pharmaceutical applications of the detection process of the invention, and/or of the primers and/or probes of the invention. The invention thus relates to a process for the diagnosis or prognosis of a CT and/or MG and/or NG infection, which comprises detecting CT and/or MG and/or NG with at least one primer of the invention.

The application also describes a process for monitoring the efficiency of an anti-CT and/or anti-MG and/or anti-NG treatment or drug, or an anti-CT and/or anti-MG and/or anti-NG candidate treatment or drug, which comprises determining by the detection method of the invention whether said treatment, drug, candidate treatment or candidate drug induces the non-reoccurrence, non-persistence, disappearance, or a decrease in the presence of at least one CT and/or MG and/or NG, whereby a positive determination indicates that said treatment, drug, candidate treatment or candidate drug is efficient.

The application also describes a method to produce an anti-CT and/or anti-MG and/or anti-NG drug, which comprises:
providing at least one anti-CT and/or anti-MG and/or anti-NG candidate drug,
administering said at least one candidate anti-CT and/or anti-MG and/or anti-NG drug to a cell culture or to a non-human animal, wherein said cell culture or animal is or comprises at least one CT and/or MG and/or NG, and
determining by the detection method of the invention whether said candidate anti-CT and/or anti-MG and/or anti-NG drug induces the regression or disappearance of said at least one CT and/or MG and/or NG,
whereby a positive determination indicates that said candidate drug is an efficient CT and/or MG and/or NG drug.

In the present application, start and end values of any described range are to be understood as comprised within said range, e.g., an expression such as "position X to Y of a sequence" describes a sequence extending from nucleotide in position X to nucleotide in position Y, wherein both nucleotide in position X and nucleotide in position Y are part of said sequence.

The term "comprising", which is synonymous with "including" or "containing", is openended, and does not exclude additional, unrecited element(s), ingredient(s) or method step(s), whereas the term "consisting of" is a closed term, which excludes any additional element, step, or ingredient which is not explicitly recited.

The term "essentially consisting of" is a partially open term, which does not exclude additional, unrecited element(s), step(s), or ingredient(s), as long as these additional element(s), step(s) or ingredient(s) do not materially affect the basic and novel properties of the invention.

The term "comprising" (or "comprise(s)") hence includes the term "consisting of" ("consist(s) of'), as well as the term "essentially consisting of" ("essentially consist(s) of'). Accordingly, the term "comprising" (or "comprise(s)") is, in the present application, meant as more particularly encompassing the term "consisting of" ("consist(s) of"), and the term "essentially consisting of" ("essentially consist(s) of').

In the present application, the term "at least x" relating to a set or group of n elements (wherein x is different from zero, and n is a number that is higher than x), explicitly encompasses each value, which is comprises between x and n. For example, the term "at least one" relating to a group or set of six elements explicitly encompasses one, two, three, four, five and six of said elements, as well as at least two, at least three, at least four, at least five of said elements.

Each of the relevant disclosures of all references cited herein is specifically incorporated by reference. The following examples are offered by way of illustration, and not by way of limitation.

### EXAMPLE 1: design of the primers and probes

### 1.1. Selection of primers and probe for C. trachomatis (CT)

As a source of appropriate CT targets, the inventors selected the cryptic plasmid, which is present in all *C. trachomatis* serovars. This plasmid is contained at 7-10 copies per genome.

Four different sequences of this plasmid are available from Genbank:
- plasmid pCHL1, of serotype D strain GO/86 (accession J03321; 7502 bp),
- plasmid pCTT1 (accession M 19487; 7496 bp), of serotype B,
- plasmid pLGV440 (accession X06707; 7501 bp) of serovar L1,
- plasmid CTPLAS75 (accession X07547.1; 7499 bp) of serovar L2.

There is less than 1% variation between these four sequences (Comanducci, et al., 1990, Plasmid, 23: 149-154).

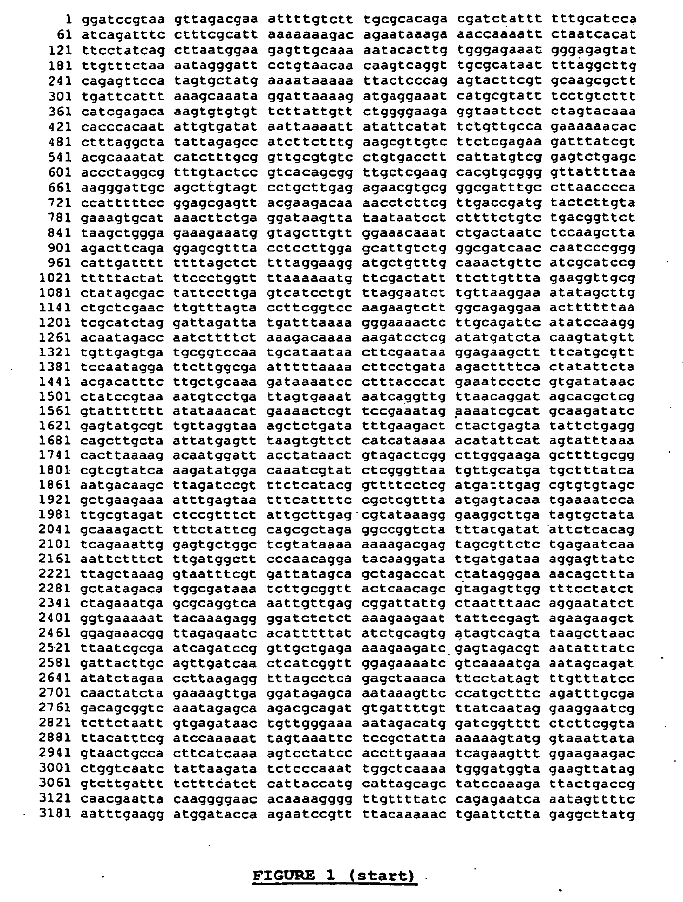

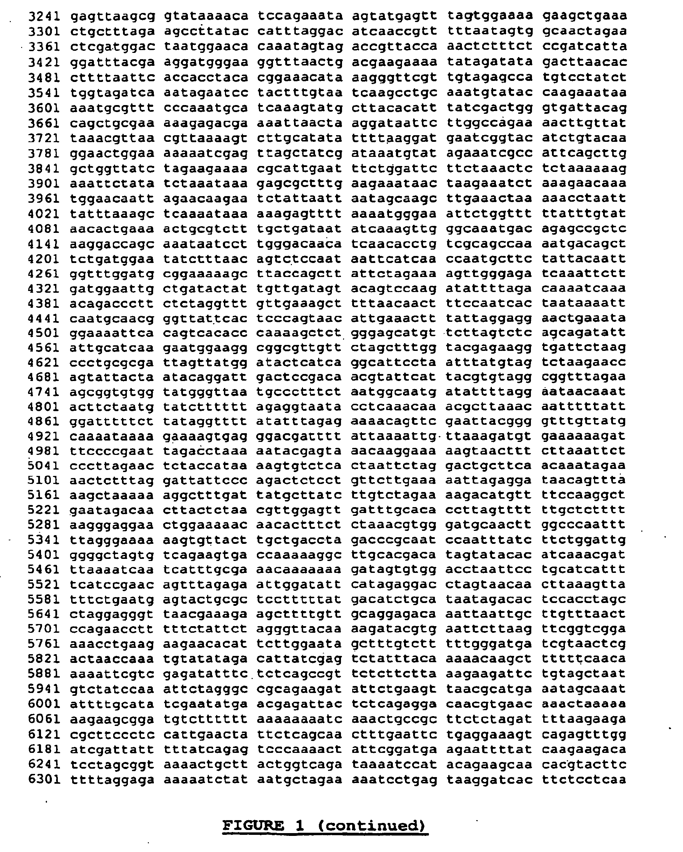

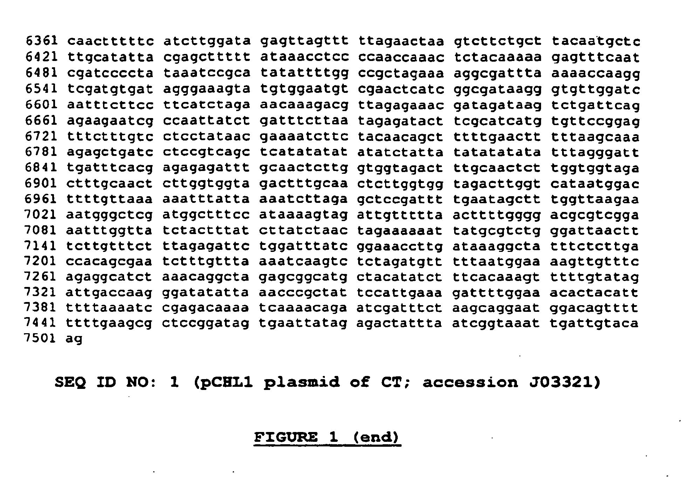

The sequence of plasmid pCHL1, which is available under accession number J03321, is shown on the enclosed Figure 1 (SEQ ID NO: 1; 7502nt).

A selected CT target sequence is located in ORF5 of pCHL1. A sub-sequence of SEQ ID NO: 5 has been selected by the inventors within the sequence of pCHL1 ORF5.

### CT sub-sequence selected within pCHL1 of SEQ ID NO: 1:

5571 cttaaagtta
5581tttctgaatg agtactgcgc tcctttttat ga(catctgca taatagacac tcca[ccta)gc
5641 ctaggagggt taacgaaaga a]gcttttgtt gcaggagaca aattaattgc ttgtttaact

5701 ccagaacctt tttctattct agggttacaa aagatacgtg aattcttaag ttcggtcgga
SEQ ID NO: 5

Within this CT sub-sequence of SEQ ID NO: 5, a CT target sequence has been selected by the inventors (SEQ ID NO: 6). The forward and reverse primers are referred to as U-PC 5580 and L-PC 5754, respectively. Two FAM-labelled fluorescent probes (SCT175b, SCT175c) proved to be successful in detecting the amplicon. The sequences of these CT primers and probes are shown in the above CT sub-sequence of SEQ ID NO: 5 (from 5' to 3'):
- the sequence of a CT forward primer (U-PC 5580) (underlined and bold characters),
- the respective sequences of two CT probes (probe SCT 175b in underlined and bold characters between parenthesis; probe SCT175c in underlined and bold characters between brackets), and
- the target sequence of a CT reverse primer (L-PC 5754) (in underlined and bold characters).

The selected CT target sequence therefore is:

### CT target sequence (175nt): positions 5580 to 5754 of SEQ ID NO:1

5580 a
5581 tttctgaatg agtactgcgc tcctttttat gacatctgca taatagacac tccacctagc
5641 ctaggagggt taacgaaaga agcttttgtt gcaggagaca aattaattgc ttgtttaact
5701 ccagaacctt tttctattct agggttacaa aagatacgtg aattcttaag ttcg
SEQ ID NO: 6

CT forward primer U-PC 5580 (20nt): positions 5580 to 5599 of SEQ ID NO: 1
ATT TCT GAA TGA GTA CTG CG **SEQ ID NO: 7**
CT probe SCT 175b (26 nt): positions 5613 to 5638 of SEQ ID NO: 1
CAT CTG CAT AAT AGA CAC TCC ACC TA **SEQ ID NO: 8**
beacon® arms: CGC GC in 5'; GC GCG in 3'
probe in beacon® format:
CGC GCC ATC TGC ATA ATA GAC ACT CCA CCT AGC GCG **(SEQ ID NO: 9)**
dye/quencher: FAM in 5'; Dabcyl in 3'
CT probe SCT 175c (27nt): positions 5635 to 5661 of SEQ ID NO: I
CCT AGC CTA GGA GGG TTA ACG AAA GAA **SEQ ID NO: 10**
beacon® arms: ACG CGC in 5'; GCG CGT in 3'
probe in beacon® format:
ACG CGC CCT AGC CTA GGA GGG TTA ACG AAA GAA GCG CGT (**SEQ ID NO: 11**)
dye/quencher: FAM in 5'; Dabcyl in 3'
CT reverse primer L-PC 5754 (22nt): complementary to positions 5733 to 5754 of SEQ ID NO: 1
CGA ACT TAA GAA TTC ACG TAT C **SEQ ID NO: 12**

### 1.2. Selection of primers and probe for Mycoplasma genitalium (MG)

The MG target sequence is selected within the gene coding for MgPa adhesin protein (major surface protein). This gene is referred to as adhesin gene, or Pa gene, or MgPa gene.

It is present at one copy per bacterium genome, and has been completely sequenced for reference strain G-37 (accession M31431).

A 5' region of this gene has also been sequenced for four other MG strains; these sequences are available from GenBank:
- accession X91074, strain M2341;
- accession X91073, strain M2321;
- accession X91071 strain 2288;
- accession X91072, strain 2300.

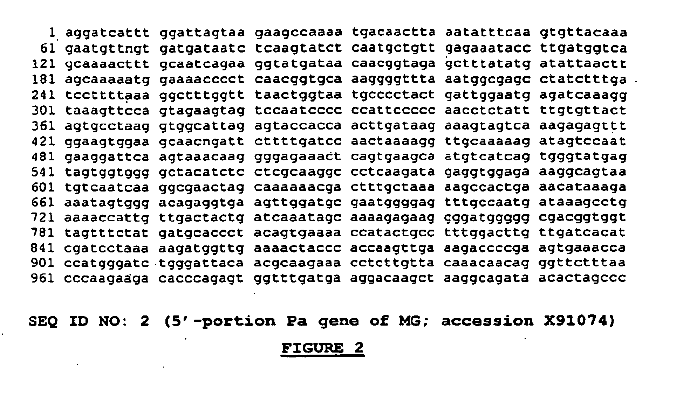

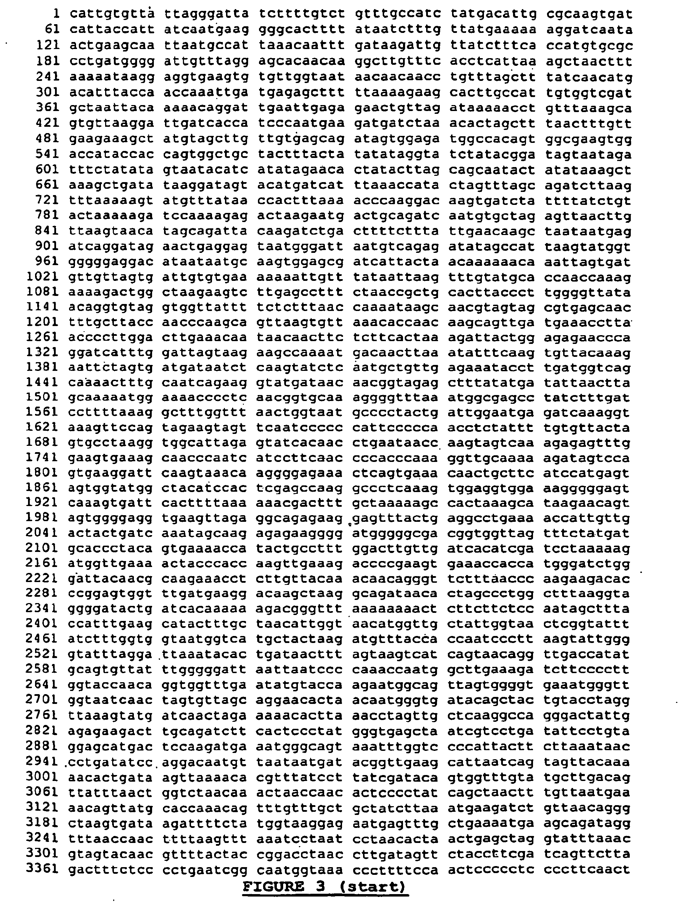

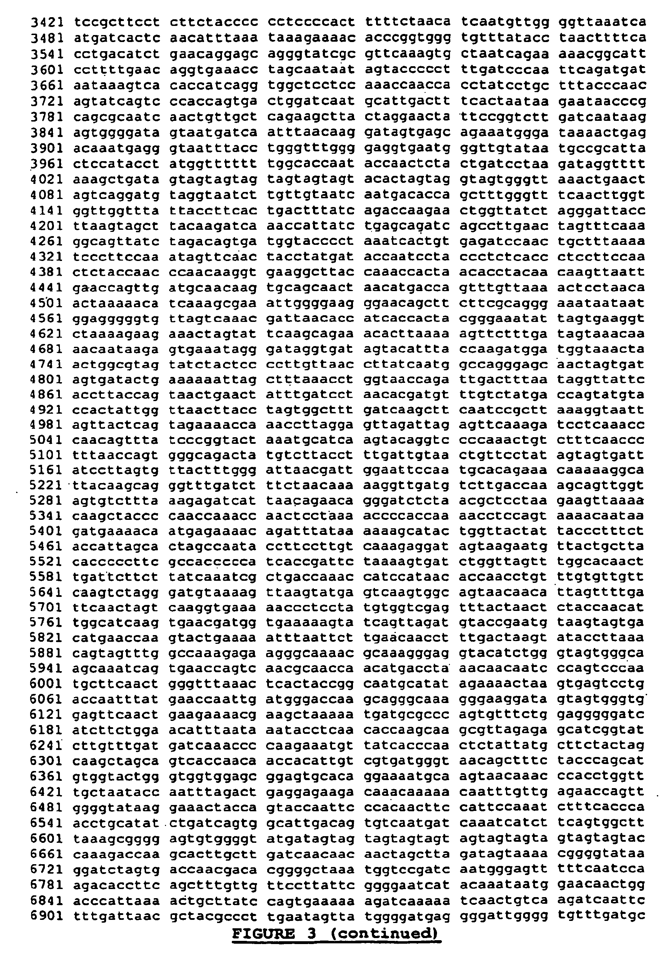

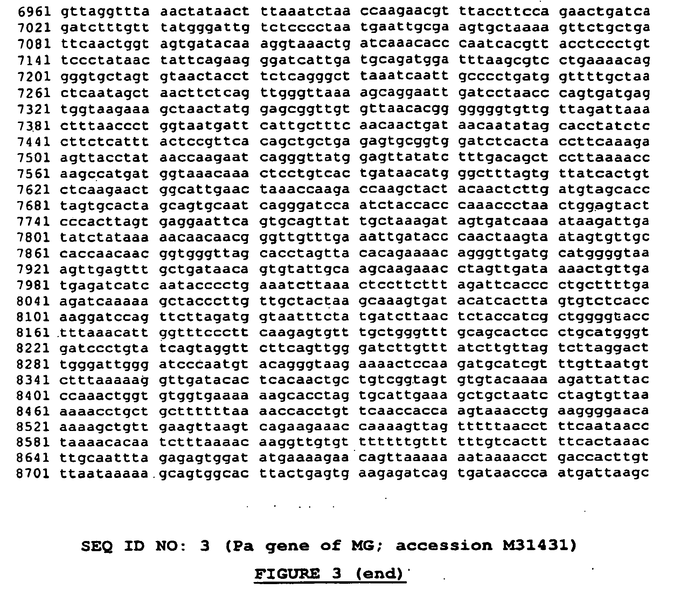

The sequence of the 5' region of the adhesin gene of strain M2341, which is available under accession number X91074, is shown on the enclosed Figure 2 (SEQ ID NO: 2). The sequence of the adhesin gene of strain G-37, which is available under accession M31431, is shown on the enclosed Figure 3 (SEQ ID NO: 3).

### 1.2.1. Design on the MG adhesin gene 5'-portion having accession number X91074 (SEQ ID NO: 2):

A selected MG target sequence is located within the following Pa gene sub-sequence: MG sub-sequence selected within MgPa sequence of SEQ ID NO: 2:

1 aggatcattt ggattagtaa gaagccaaaa tgacaactta aatatttcaa gtgttacaaa
61 gaatgttngt gatgataatc tcaagtatct caatgctgtt gagaaatacc ttgatggtca
121 gcaaaacttt gcaatcagaa ggtatgataa caacggtaga gctttatatg atattaactt
181 agcaaaaatg gaaaacccct caacggtgca aaggggttta aatggcgagc ctatctttga
241 tccttttaaa ggctttggtt taactggtaa SEQ ID NO: 13

A MG target sequence (SEQ ID NO: 14) has been selected within this MG sub-sequence of SEQ ID NO: 13. The sequence of a MG forward primer (U-MG 1320), the sequence of a MG probe (SF-MG 258c), and the target sequence of a MG reverse primer (L-MG 1578) are shown in underlined and bold characters within the above MG sub-sequence of SEQ ID NO: 13 (from 5' to 3', respectively).

The selected MG target sequence therefore is:

### MG target sequence (258nt): positions 2 to 259 of SEQ ID NO: 2:

ggatcattt ggattagtaa gaagccaaaa tgacaactta aatatttcaa gtgttacaaa
gaatgttngt gatgataatc tcaagtatct caatgctgtt gagaaatacc ttgatggtca
gcaaaacttt gcaatcagaa ggtatgataa caacggtaga gctttatatg atattaactt
agcaaaaatg gaaaacccct caacggtgca aaggggttta aatggcgagc ctatctttga
tcctttaaa ggctttggt SEQ ID NO: 14

MG forward primer (U-MG 1320; 24nt): positions 2 to 25 of SEQ ID NO: 2
GGA TCA TTT GGA TTA GTA AGA AGC **SEQ ID NO: 15**
MG probe (SF-MG 258c; 27nt): positions 136 to 162 of SEQ ID NO: 2
CAG AAG GTA TGA TAA CAA CGG TAG AGC **SEQ ID NO: 16**
beacon® arms: TGC GCA in 5'; TGC GCA in 3'
probe in beacon® format:
TGC GCA CAG AAG GTA TGA TAA CAA CGG TAG AGC TGC GCA **(SEQ ID NO: 17)**
dye/quencher: Tamra in 5'; Dabcyl in 3'
MG reverse primer (L-MG 1578; 23nt): complementary to positions 237 to 259 of SEQ, ID NO: 2
ACC AAA GCC TTT AAA AGG ATC AA **SEQ ID NO: 18**

### 1.2.2. Design on the MG adhesin gene having accession number M31431 (SEO ID NO: 3):

Four other MG targets have been selected by the inventors. These four other MG targets are also located within the MG adhesin gene, more particularly within the 5' region of this gene. They are defined with respect to the adhesin gene sequence, which is available under accession number M31431 (SEQ ID NO: 3, shown in Figure 3).

These four other MG target sequences are located within the following MG subsequences:
- positions 1140-1290 of SEQ ID NO: 3 (sub-sequence of **SEQ ID NO: 19**);
- positions 1060-1250 of SEQ ID NO: 3 (sub-sequence of **SEQ ID NO: 25**);
- positions 1520-1710 of SEQ ID NO: 3 (sub-sequence of **SEQ ID NO: 31**);
- positions 1500-1710 of SEQ ID NO: 3 (sub-sequence of **SEQ ID NO: 37**).
- positions 1140-1290 of SEQ ID NO: 3 (sub-sequence of **SEQ ID NO: 19):** 
1140 a acaggtgtag gtggttattt tctctttaac caaaataagc aacgtagtag cgtgagcaac
1201 tttgcttacc aacccaagca gttaagtgtt aaacaccaac aagcagttga tgaaacctta
1261 accccttgga cttgaaacaa taacaacttc SEQ ID NO: 19
- positions 1060-1250 of SEQ ID NO: 3 (sub-sequence of **SEQ ID NO: 25**): 
1060 g tttgtatgca ccaaccaaag
1081 aaaagactgg ctaagaagtc ttgagccttt ctaaccgctg cacttaccct tggggttata
1141 acaggtgtag gtggttattt tctctttaac caaaataagc aacgtagtag cgtgagcaac
1201 tttgcttacc aacccaagca gttaagtgtt aaacaccaac aagcagttga
SEQ ID NO: 25
- positions 1520-1710 of SEQ ID NO: 3 (sub-sequence of **SEQ ID NO: 31):** 
1520 c aacggtgcaa aggggtttaa atggcgagcc tatctttgat
1561 ccttttaaag gctttggttt aactggtaat gcccctactg attggaatga gatcaaaggt
1621 aaagttccag tagaagtagt tcaatccccc cattccccca acctctattt tgtgttacta
 1681 gtgcctaagg tggcattaga gtatcacaac SEQ ID NO: 31
- positions 1500-1710 of SEQ ID NO: 3 (sub-sequence of SEQ ID NO: 37):

1500 a gcaaaaatgg aaaacccctc aacggtgcaa aggggtttaa atggcgagcc tatctttgat
1561 ccttttaaag gctttggttt aactggtaat gcccctactg attggaatga gatcaaaggt
1621 aaagttccag tagaagtagt tcaatccccc cattccccca acctctattt tgtgttacta
1681 gtgcctaagg tggcattaga gtatcacaac SEQ ID NO: 37

### 1.2.2.1. Primer pair (U-MG 1144 / L-MG 1283), amplicon of 140 bp:

MG target sequence (140 bp): positions 1144 to 1283 of SEQ ID NO: 3

1144 ggtgtag gtggttattt tctctttaac caaaataagc aacgtagtag cgtgagcaac
1201 tttgcttacc aacccaagca gttaagtgtt aaacaccaac aagcagttga tgaaacctta
1261 acccccttgga cttgaaacaa taa SEQ ID NO: 20

In bold and underlined characters, are shown (from 5' to 3') the sequences of the MG forward primer (U-MG1144), of the target for the MG probe (MGBR 140c), and of the target for the MG reverse primer (L-MG1283).
MG forward (U-MG 1144, 21 nt) positions 1144 to 1164 of SEQ ID NO: 3
GGT GTA GGT GGT TAT TTT CTC **SEQ ID NO: 21**
MG probe (MGBR 140c Tamra/Dabcyl, 25 nt) positions 1208 to 1232 of SEO ID NO: 3
ACC AAC CCA AGC AGT TAA GTG TTA A **SEQ ID NO: 22**
beacon® arms: **CGCGTT** in 5'; **A ACG CG** in 3'
probe in beacon® format:
**CGCGTT** AC CAA CCC AAG CAG TTA AGT GTT AA **A ACG CG (SEQ ID NO: 23)**
dye/quencher: Tamra in 5'; Dabcyl in 3'
MG reverse primer (L-MG 1283. 22 nt): complementary to positions 1262 to 1283 of SEQ ID NO: 3
TTA TTG TTT CAA GTC CAA GGG G **SEQ ID NO: 24**

### 1.1.2.2. Primer pair (U MG 1087 / L MG1249), amplicon of 186 bp:

MG target sequence (186 bp): positions 1064 to 1249 of SEQ ID NO: 3

1064 gt atgca ccaaccaaag
1081 aaaagactgg ctaagaagtc ttgagccttt ctaaccgctg cacttaccct tggggttata
1141 acaggtgtag gtggttattt tctctttaac caaaataagc aacgtagtag cgtgagcaac
1201 tttgcttacc aacccaagca gttaagtgtt aaacaccaac aagcagttg
SEQ ID NO: 26

In bold and underlined characters, are shown (from 5' to 3') the sequences of the MG forward primer (U-MG1087), of the target for the MG probe (MGBR 186j Tamra/Dabcyl), and of the target for the MG reverse primer (L-MG1249).
MG forward (U-MG 1087, 24 nt), positions 1064 to 1087 of SEQ ID NO: 3 GTATGCACCAACCAAAGAAAAGAC **SEQ ID NO: 27**
MG probe (MGBR 186j Tamra/Dabcyl, 28 nt) positions 1142 to 1168 of SEQ ID NO:3 CAG GTG TAG GTG GTT ATT TTC TCT TTA **SEQ ID NO: 28**
beacon® arms: CGC GTT in 5'; AAC GCG in 3'
probe in beacon® format:
CGC GTT CAG GTG TAG GTG GTT ATT TTC TCT TTA AAC GCG **SEQ ID NO: 29**
dye/quencher: Tamra in 5'; Dabcyl in 3'
MG reverse primer (L-MG 1249, 24 nt): complementary to positions 1226 to 1249 of SEQ ID NO:3
CAA CTG CTT GTT GGT GTT TAA CAC **SEQ ID NO: 30**

### 1.2.2.3. Primer pair (U-MG 1527 / L-MG 1704), amplicon of 177 bp

### MG target sequence (178 bp): positions 1527 to 1704 of SEQ ID NO: 3

1527 gcaa aggggtttaa atggcgagcc tatctttgat
1561 ccttttaaag gctttggttt aactggtaat gcccctactg attggaatga gatcaaaggt
1621 aaagttccag tagaagtagt tcaatccccc cattccccca acctctattt tgtgttacta
1681 gtgcctaagg tggcattaga gtat

SEQ ID NO: 32

In bold and underlined characters, are shown (from 5' to 3') the sequences of the MG forward primer (U-MG1527), of the target for the MG probe (MGBR 178q Tamra/Dabcyl), and of the target for the MG reverse primer (L-MG1704).
MG forward (U-MG 1527, 24 nt), positions 1527 to 1550 of SEQ ID NO: 3
GCA AAG GGG TTT AAA TGG CGA GCC **SEQ ID NO: 33**
MG probe (MGBR 178q Tamra/Dabcyl, 26 nt) positions 1607 to 1630 of SEQ ID NO:3
ATG AGA TCA AAG GTA AAG TTC CAG TA **SEQ ID NO: 34**
beacon® arms: AGC GTC in 5';GAC GCT in 3'
probe in beacon® format:
AGC GTC ATG AGA TCA AAG GTA AAG TTC CAG TA GAC GCT **(SEQ ID**
**NO:35)**
dye/quencher: Tamra in 5'; Dabcyl in 3'
MG reverse primer (L-MG 1704, 24 nt): complementary to positions 1681 to 1704 of SEQ ID NO:3
ATA CTC CAA TAC CAC CTT AGG CAC **SEQ ID NO: 36**

### 1.2.2.4. Primer pair (U MG 1501 / LMG 1704), amplicon of 203 bp

### MG target sequence (203 bp):positions 1501 to 1704 of SEQ ID NO: 3

1501 gcaaaaatgg aaaacccctc aacggtgcaa aggggtttaa atggcgagcc tatctttgat
1561 ccttttaaag gctttggttt aactggtaat gcccctactg attggaatga gatcaaaggt
1621 aaagttccag tagaagtagt tcaatccccc cattccccca acctctattt tgtgttacta
1681 gtgcctaagg tggcattaga gtat SEQ ID NO: 38

In bold and underlined characters, are shown (from 5' to 3') the sequences of the MG forward primer (U-MG 1501), of the target for the MG probe (MGBR 178q Tamra/Dabcyl), and of the target for the MG reverse primer (L-MG 1704).
MG forward (U-MG 1501, 25 nt), positions 1501 to 1525 of SEQ ID NO: 3
GCA AAA ATG GAA AAC CCC TCA ACG G **SEQ ID NO: 39**
MG probe (MGBR 204u Tamra/Dabcyl, 27 nt), positions 1600 to 1626 of SEQ ID NO: 3
GAT TGG AAT GAG ATC AAA GGT AAA GTT **SEQ ID NO: 40**
beacon® arms: CGC CCT in 5'; AGG GCG in 3'
probe in beacon® format:
CGC CCT GAT TGG AAT GAG ATC AAA GGT AAA GTT AGG GCG **(SEQ ID NO: 41)**
dye/quencher: Tamra in 5'; Dabcyl in 3'
MG reverse primer (L-MG 1704, 24 nt): complementary to positions 1681 to 1704 of SEQ ID NO: 3
ATA CTC CAA TAC CAC CTT AGG CAC **SEQ ID NO: 36**

### 1.3. Selection of primers and probe for Neisseria gonorrhoeae (NG)

The selection of an appropriate nucleotide target is difficult to achieve for NG. The genome of NG is indeed highly homologous to the one of *M. meningitidis* (at about 98%), and is homologous to the genome of commensal species such as *N. cinerea, N. lactamica, N. sicca, N. subflava, N. mucosa.*

We selected the *pil*E gene to detect NG. The *pil*E gene codes for type IV pili. It is present within every pathogenic NG, and is absent from non-pathogenic NG (NG strains often loose this gene when they are grown *in vitro*).

The *pilE* gene is present at a variable copy number, and is often subject to nucleotide variations. The *pil*E gene is also present in *N. lactamica* and *N. cinerea,* and may also be present in some other bacterial geni, such as some *Pseudomonas, Bacteroides, and Bacillus.*

A primer pair (U-pilE 159 / L-pilE 406) has been selected. The obtained amplicon is of 252 bp. A fluorescent (ATTO 647N/Dabcyl) probe has been selected to hybridize to the amplicon.

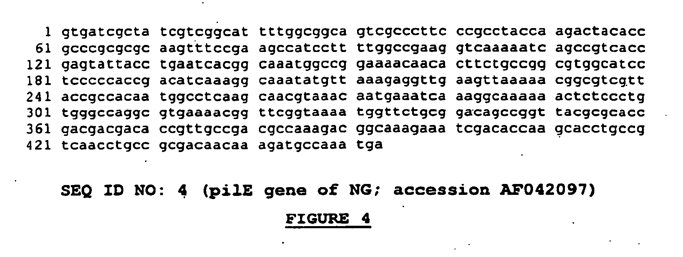

The sequence of the *pil*E gene, which is accessible under accession number AF042097, is shown on the enclosed Figure 4 (SEQ ID NO: 4).

### NG sub-sequence selected within pilE of SEQ ID NO: 4 (positions 101-380):

101                                                    gtcaaaatc agccgtcacc
121 gagtattacc tgaatcacgg caaatggccg gaaacaaca cttctgccgg cgtggcatcc
181 tcccccaccg acatcaaagg caaatatgtt aaagaggttg aagttaaaaa cggcgtcgtt

241 accgccacaa tggcctcaag caacgtaaac aatgaaatca aaggcaaaaa actctccctg
301 tgggccaggc gtgaaaacgg ttcggtaaaa tggttctgcg gacagccggt tacgcgcacc
361 gacgacgaca ccgttgccga SEQ ID NO: 42

Underlined are shown (from 5' to 3') the sequences of the NG forward primer (U-pilE 159), of the target for the NG probe (pilEc), and of the target for the NG reverse primer (L-pilE 406).

### NG target sequence (252nt): positions 114 to 365 of SEQ ID NO: 4

cgtcacc
gagtattacc tgaatcacgg caaatggccg gaaaacaaca cttctgccgg cgtggcatcc
tcccccaccg acatcaaagg caaatatgtt aaagaggttg aagttaaaaa cggcgtcgtt
accgccacaa tggcctcaag caacgtaaac aatgaaatca aaggcaaaaa actctccctg
tgggccaggc gtgaaaacgg ttcggtaaaa tggttctgcg gacagccggt tacgcgcacc
gacga SEQ ID NO: 43

NG forward primer (U-pilE 159; 19nt): positions 114 to 132 of SEQ ID NO: 4
CGT CAC CGA GTA TTA CCT G **SEQ ID NO: 44**
NG probe (pilEc; 22nt): complementary to positions 285 to 306 de SEQ ID NO: 4
GGC CCA CAG GGA GAG TTT TTT G **SEQ ID NO: 45**
beacon® arms: ACT GCG in 5'; CGC AGT in 3'
Probe in beacon® format:
ACT GCG GGC CCA CAG GGA GAG TTT TTT G CGC AGT **(SEQ ID NO: 46)**
dye/quencher: Atto 647 N in 5'; Dabcyl in 3'
NG reverse primer (L-pilE 406; 17nt): complementary to positions 349 to 365 of SEQ ID NO: 4
TCG TCG GTG CGC GTA AC **SEQ ID NO: 47**

### 1.4. Internal Control (IC):

The Internal Control (IC) consists in a single-stranded random sequence.

The IC comprises a sequence which is unrelated to CT, MG and NG, and which preferably is also unrelated to human nucleic acids. The IC further comprises a sequence located in 5' of this unrelated sequence and a sequence located 3' of this unrelated sequence, wherein one of said 5'- and 3'-located sequences has the sequence of a primer of a primer pair, and the other of said 5'- and 3'-located sequences is the complementary sequence of the other primer of the same primer pair, such that said primer pair can hybridize to said IC in such locations and following such an orientation that this primer pair can function as an amplification forward and reverse primer pair on said IC. For example, said 5'-located sequence is identical to the forward primer of a primer pair, and said 3'-located sequence is complementary to the reverse primer of said primer pair.

This primer pair may be a primer pair which is used for the detection of CT, MG, or NG, or it can be a different primer pair, which has then to be specifically added in the PCR mix when implementing a multiplex PCR.

In the present example, the IC is of 92 bases, and comprises 5'- and 3'-located sequences which hybridize to a primer pair (primer pair IS 368 and IS 569), which is different from the CT, MG and NG primer pairs.

### Internal Control sequence (92nt):

ATTGGATCCCAGCACGCTAATTACCCAGTATCAGATGACGTGGCAGCCATG
AGAGTGGGACAGTCGTCCTTCAAGGAGCACATCAGCGGATC
SEQ ID NO: 48

Underlined are shown (from 5' to 3') the sequences of the IC forward primer (IS 368), of the target of the IC probe, and of the target of the reverse primer (IS 569).

### IC forward primer (IS 368; 17nt)

CAGCACGCTAATTACCC **SEQ ID NO: 49**

### IC probe (SIMB ATTO 590; 23nt)

CCC ACT CTC ATG GCT GCC ACG TC **SEQ ID NO: 50**
beacon arms: CAGGCG in 5'; CGCCTG in 3'
probe in beacon format:
CAGGCG CCC ACT CTC ATG GCT GCC ACG TC CGCCTG **(SEQ ID NO: 51)**
dye/quencher: ATTO 590 in 5'; Dabcyl in 3'

### IC reverse primer (IS 569; 17nt)

GCTGATGTGCTCCTTGA **SEQ ID NO: 52**
**1.5. 16S rRNA**

A fragment of the 16S rRNA which is present in all micro-organisms is also amplified. The following 16S rRNA primers have been used:
S1-F: AGT TTG ATC ATG GCT CAG **SEQ ID NO: 53**
S1-R: GTA TTA CCG CGG CTG CT **SEQ ID NO: 54**

### 1.6. Sequences and SEO ID NO:

**Table 1: SEQ ID NO sequences**

| **SEQ ID NO:** | | |
|---|---|---|
| **CT, MG and NG reference sequences** | | |
| 1 | pCHL1 plasmid of **CT** | Accession number J03321 |
| 2 | 5' region of adhesin gene of **MG** | Accession number X91074 |
| 3 | Adhesin gene of **MG** | Accession number M31431 |
| 4 | *pil*E gene of **NG** | Accession number AF042097 |

| **CT real-time amplification systems of the invention (CT systems n°1, n°2)** | | |
|---|---|---|
| 5 | Selected **CT** sub-sequence | 5571-5760 from SEQ ID NO: 1 |
| 6 | **CT target (175 nt)** | **5580-5754 from SEQ ID NO:1** |
| **7** | CT forward primer U-PC 5580 | 5580-5599 from SEQ ID NO:1 |
| 8 | CT probe SCT 175b | 5613-5638 from SEQ ID NO:1 |
| 9 | CT probe SCT 175b in beacon format | |
| **12** | CT reverse primer L-PC 5754 | 5733-5754 from SEQ ID NO: 1 |
| 5 | Selected **CT** sub-sequence | 5571-5760 from SEQ ID NO: 1 |
| 6 | **CT target (175nt)** | **5580-5754 from SEQ ID NO:1** |
| **7** | CT forward primer U-PC 5580 | 5580-5599 from SEQ ID NO:1 |
| 10 | CT probe SCT 175c | 5635-5661 from SEQ ID NO:1 |
| **11** | CT probe SCT 175c in beacon format | |
| **12** | CT reverse primer L-PC 5754 | 5733-5754 from SEQ ID NO:1 |

| **MG real-time amplification systems of the invention (MG system n°1, n°2, n°3, n°4, n°5)** | | |
|---|---|---|
| 13 | Selected **MG** sub-sequence | 1-270 from SEQ ID NO:2 |
| 14 | **MG target (258 nt)** | **2-259 from SEQ ID NO:2** |
| **15** | MG forward primer U-MG 1320 | 2-25 from SEQ ID NO:2 |
| 16 | MG probe SF-MG 258c | 136-162 from SEQ ID NO:2 |
| **17** | MG probe SF-MG 258c in beacon format | |
| **18** | MG reverse primer L-MG 1578 | 237-259 from SEQ ID NO:2 |
| 19 | Selected MG sub-sequence | 1140-1290 from SEQ ID NO:3 |
| 20 | **MG target (140 nt)** | **1144-1283 from SEQ ID NO: 3** |
| **21** | MG forward primer U-MG 1144 | 1144-1164 from SEQ ID NO: 3 |
| 22 | MG probe MGBR 140c | 1208-1232 from SEQ ID NO: 3 |
| **23** | MG probe MGBR 140c in beacon format | |
| **24** | MG reverse primer L-MG 1283 | 1262-1283 from SEQ ID NO: 3 |
| 25 | Selected MG sub-sequence | 1060-1250 from SEQ ID NO:3 |
| 26 | **MG target (186 nt)** | **1064-1249 from SEQ ID NO:3** |
| **27** | MG forward primer U-MG 1087 | 1064-1087 from SEQ ID NO:3 |
| 28 | MG probe MGBR 186j | 1142-1168 from SEQ ID NO:3 |
| **29** | MG probe MGBR 186j in beacon format | |
| **30** | MG reverse primer L-MG 1249 | 1226-1249 from SEQ ID NO:3 |
| 31 | Selected MG sub-sequence | 1520-1710 from SEQ ID NO: 3 |
| 32 | **MG target (178 nt)** | **1527-1704 from SEQ ID NO:3** |
| **33** | MG forward primer U-MG 1527 | 1527-1550 from SEQ ID NO:3 |
| 34 | MG probe MGBR 178q | 1607-1630 from SEQ ID NO:3 |
| **35** | MG probe MGBR 178q in beacon format | |
| **36** | MG reverse primer L-MG 1704 | 1681-1704 from SEQ ID NO:3 |
| 37 | Selected MG sub-sequence | 1500-1710 from SEQ ID NO: 3 |
| 38 | **MG target (204 nt)** | **1501-1704 from SEQ ID NO:3** |
| **39** | MG forward primer U-MG 1501 | 1501-1525 from SEQ ID NO:3 |
| 40 | MG probe MGBR 204u | 1600-1626 from SEQ ID NO:3 |
| **41** | MG probe in beacon format | |
| **36** | MG reverse primer L-MG 1704 | 1681-1704 from SEQ ID NO:3 |

| **NG real-time amplification system of the invention** | | |
|---|---|---|
| 42 | Selected NG sub-sequence | 101-380 from SEQ ID NO: 4 |
| 43 | **NG target (252 nt)** | **114-365 from SEQ ID NO: 4** |
| **44** | NG forward primer U-pilE 159 | 114-132 from SEQ ID NO: 4 |
| 45 | NG probe pilEc | 285-306 from SEQ ID NO: 4 |
| **46** | NG probe in beacon format | |
| **47** | NG reverse primer L-pilE 406 | 349-365 from SEQ ID NO: 4 |

| **Internal Control (IC)** | | |
|---|---|---|
| **48** | IC sequence (92nt) | Unrelated to CT, MG and NG |
| **49** | IC forward primer IS 368 | |
| 50 | IC probe | |
| **51** | IC probe in beacon format | |
| **52** | IC reverse primer IS 569 | |

| **16S rRNA** | | |
|---|---|---|
| **53** | 16S rRNA forward primer S1-F | Fragment of 16S rRNA |
| **54** | 16S rRNA reverse primer S1-R | Fragment of 16S rRNA |
| 55 | MG primer MgPa-1 | |
| 56 | MG primer MgPa-3 | |
| 57 | MG Southern blot probe MgPa-2 | |

### 2. EXAMPLE 2: specificity and sensitivity of CT, MG and NG real-time amplification systems of the invention:

- specificity in real-time simplex PCR (CT or MG or NG real-time amplification system on a panel of bacterial strains);
- sensitivity of these systems in real-time multiplex PCR (CT+MG+NG+IC systems together in the same mix, on a mix of a pre-determined quantity of CT, MG and NG DNA).

### 2.1. Materials and Methods:

### 2.1.1. Description of the extraction method:

Lysis is performed in the presence of detergents and of Chelex™ X-100 beads (i.e., a resin which binds divalent ions, and which also is a chaotropic agent).

### Composition of the lysis buffer:

8% of Chelex™ X-100 resin (Bio-Rad, ref.: 142-1253); 0.5 % NP-40 (Sigma, ref.: Igepal I-3021); 0.5 % Tween 20 (VWR, ref.: 28829296) in Tris 10 mM buffer (Sigma, ref.: T-6791); EDTA 1 mM (Sigma, ref.: E-1644) pH 8.3.

### Method:

- collect 1 mL of sample (sample of urine, or of transport medium), and centrifuge it for 10 minutes at 8,000rpm; discard the whole supernatant,
- add 400 µL lysis buffer in the centrifugation pellet,
- add 10 µL of liquid internal control,
- vortex 30",
- incubation 10 min at 95°C,
- centrifugation for 5 min at 8,000 rpm,
- PCR on 10 µL of supernatant.

The internal control (IC) is added at the extraction step.

The IC solution is of 9.6 10⁴ copies of IC per 10 µL, to obtain 2.4 10³ copies per PCR test after extraction.

The negative control is achieved by collecting 400 µL of lysis buffer into which the IC is added.

### 2.1.2. PCR conditions:

* Reactants:
   Hot Start Taq Polymerase Qiagen (5U/µL, ref 203205), containing the PCR buffer dNTP: Promega ref U 151 (4X 25 mM)
   MgCl₂: Sigma, ref M-2670
   PVP10: Sigma, ref PVP-10
   Glycerol: VWR, ref 24388295
* Thermocyclor: iQ1 (Bio-Rad)

### 2.1.3. Amplification of a fragment of the gene coding for 16S rRNA

The products amplified by the 16S rRNA primers (S1-F, and S1-R) are visualised by a Bet staining after electrophoretic migration on a 7.5% acrylamide gel.

### 2.1.4. Real-time simplex PCR for Chlamydia trachomatis

* composition of the mix
   In a 1X PCR mix, add: 0.2 µM of the SCT 175b probe (SEQ ID NO: 9), 0.5 µM of each CT primers (U-PC 5580 -SEQ ID NO: 7-, et L-PC 5754 -SEQ ID NO: 12-), 5% Glycerol, 0.3 % PVP 10, 2U of Taq Polymerase, 1 mM dNTP and 6 mM final of MgCl₂. 10 µL of DNA are added to this mix.
* thermocycling:
   First cycle: 15" at 95°C
   Second cycle: 30" at 95°C
   Third cycle: 45" at 56°C
   Fourth cycle: 30" at 72°C
   Repeat 50 times from cycle 2 to cycle 4.

### 2.1.5. Real-time simplex PCR for Mycoplasma genitalium

* composition of the mix
   In a 1X PCR mix, add: 0.2 µM of the MG probe (SF-MG 258c -SEQ ID NO: 17-), 0.5 µM of each MG primers (U-MG 1320 -SEQ ID NO: 15-, and L-MG 1578 -SEQ ID NO: 18-), 5% Glycerol, 0.3 % PVP 10, 2U of Taq Polymerase, 1 mM dNTP and 6 mM final of MgCl₂.
   10 µL of DNA are added to this mix.
* thermocycling:
   First cycle: 15" at 95°C
   Second cycle: 30" at 95°C
   Third cycle: 45" at 57°C
   Fourth cycle: 30" at 72°C
   Repeat 50 times from cycle 2 to cycle 4

### 2.1.6. Real-time simplex PCR for Neisseria gonorrhoeae

* composition of the mix
   In a 1X PCR mix, add : 0.2 µM of the NG probe (pilEc -SEQ ID NO: 46-), 0.5 µM of each NG primers (U pilE 159 -SEQ ID NO: 44-, et L-pilE 406 -SEQ ID NO: 47-), 5% Glycerol, 0.3 % PVP 10, 2U of Taq Polymerase, 1 mM dNTP and 4 mM final of MgCl₂. 10 µL of DNA are added to this mix.
* thermocycling:
   First cycle: 15" at 95°C
   Second cycle: 30" at 95°C
   Third cycle: 45" at 57°C
   Fourth cycle: 30" at 72°C
   Repeat 50 times from cycle 2 to cycle 4

### 2.1.7. Real-time multiplex PCR

* composition of the mix
   In a 1X PCR mix, add the following primers:
   CT forward primer U-PC 5580 (SEQ ID NO: 7) at 0.125 µM,
   CT reverse primer L-PC 5754 (SEQ ID NO: 12) at 0.5 µM,
   MG forward primer U-MG 1320 (SEQ ID NO: 15) at 0.025 µM,
   MG reverse primer L-MG 1578 (SEQ ID NO: 18) at 1.25 µM,
   NG forward primer U-pilE 159 (SEQ ID NO: 44) at 0.85 µM,
   NG reverse primer L-pilE 406 (SEQ ID NO: 47) at 0.25 µM,
   IC forward primer IS 368 (SEQ ID NO: 49) at 0.5 µM, and
   IC reverse primer IS 569 (SEQ ID NO: 52) at 0.5 µM

The probes are used at the following concentrations:
CT probe SCT 175b (SEQ ID NO: 9) at 0.15 µM (fluorophore FAM),
MG probe SF-MG 258c (SEQ ID NO: 17) at 0.2 µM (fluorophore TAMRA), and
NG probe pilEc (SEQ ID NO: 46) at 0.2 µM (fluorophore ATTO 647N).
IC probe SIMB (SEQ ID NO: 51) at 0.15 µM (fluorophore ATTO-590),

The following reactants are further added:
5% Glycerol, 0.3 % PVP 10, 2U of Taq Polymerase, 1 mM dNTP and 5 mM final of MgCl₂.
10 µL of DNA are added to this mix.
* thermocycling:
   First cycle: 15" at 95°C.
   Second cycle: 30" at 95°C
   Third cycle: 50" at 58°C
   Fourth cycle: 30" at 72°C
   Repeat 50 times from cycle 2 to cycle 4
* starting material:
   CT DNA: *Chlamydia trachomatis* LGV II strain 434, available from ABi, lot 141-115, 1.63 10¹⁰ elementary body / ml, 100 µL at 50 ng/ µL.

ABi is Advanced Biotechnologies Inc., RiversPark II, 9108 Guilford Road, Columbia, MD 21046-2701, U.S.A.

MG DNA: strain G-37 available from ATCC, deposit number 33530 (source culture = ATCC 33530D), lot 2305272, concentration 200 ng / µL.

ATCC is: American Type Culture Collection, 10801 University Boulevard Manassas, Virginia 20110-2209, U.S.A.

NG DNA: strain 107031 from the CNCM (Collection de l'Institut Pasteur, BP 52, 25 rue du Docteur Roux, 75724 Paris cedex 15, France), reference strain for antimicrobial disk susceptibility test (count has been made on Petri dish).

Quantity of IC: 1,000 copies per PCR

### 2.2. Results :

The CT and MG primers and probes have a perfect specificity: they do not cross-react with any nucleic acid other than CT or MG nucleic acids (respectively); they notably do not cross-react with other bacterial or with human nucleic acids.

The NG primers and probes are specific for NG, except that they cross-react with *N*. *meningitidis.*

### 2.2.1. Specificity test for the CT system in real-time simplex PCR:

The amplicon is of 175 bp.

The CT primers and probes of the invention detect all CT serovars. They notably detect the following serovars: A, B, Ba, C, D, E, F, H, I, J, K, L1, L2a and L3.

The CT primers and probes of the invention have also been tested on 24 different bacterial strains which may be found in the urogenital sphere, and were shown to be non cross-reactive. For these 24 other strains, presence of DNA was confirmed by end-point PCR using the 16S rRNA primers (S 1 R and S 1 F primers), followed by deposition on gel.

Specificity results are shown in table 1 below (column "simplex CT").

### 2.2.2. Specificity test for the MG system in real-time simplex PCR:

The amplicon is of 258 bp.

The MG primers and probes of the invention detect the nine MG strains that have been tested (strains G-37, 2282, 2288, 2300, 2321, 2341, M30, UTMB1 and TW-10-51).

The MG primers and probes of the invention did not cross-react with any of the 29 non-MG bacterial DNA tested.

Results are reported in the above table 2, column "simplex MG".

**Table 2: specificity of the CT et MG systems**

| **Strain** | **Simplex CT** | **Simplex MG** | **16S rRNA** |
|---|---|---|---|
| human DNA | **negative** | **negative** | // |
| *Chlamydia trachomatis,* serovar A, clinical strain, CHU Bordeaux | **positive** | NT | **positive** |
| *Chlamydia trachomatis,* serovar B, clinical strain, CHU Bordeaux | **positive** | NT | **positive** |
| *Chlamydia trachomatis,* serovar Ba, clinical strain, CHU Bordeaux | **positive** | NT | **positive** |
| *Chlamydia trachomatis,* serovar C, clinical strain, CHU Bordeaux | **positive** | NT | **positive** |
| *Chlamydia trachomatis,* serovar D, strain UW-3/Cx, ATCC VR 885 | **positive** | NT | **positive** |
| *Chlamydia trachomatis,* serovar F, clinical strain, CHU Bordeaux | **positive** | NT | **positive** |
| *Chlamydia trachomatis,* serovar H, clinical strain, CHU Bordeaux | **positive** | NT | **positive** |
| *Chlamydia trachomatis,* serovar I, clinical strain, CHU Bordeaux | **positive** | NT | **positive** |
| *Chlamydia trachomatis,* serovar J, clinical strain, CHU Bordeaux | **positive** | NT | **positive** |
| *Chlamydia trachomatis,* serovar K, clinical strain, CHU Bordeaux | **positive** | NT | **positive** |
| *Chlamydia trachomatis,* serovar L1, clinical strain, CHU Bordeaux | **positive** | NT | **positive** |
| *Chlamydia trachomatis,* serovar L2a, clinical strain, CHU Bordeaux | **positive** | NT | **positive** |
| *Chlamydia trachomatis,* serovar L3, clinical strain, CHU Bordeaux | **positive** | NT | **positive** |
| *Escherichia coli* ATCC 25922 | **negative** | **negative** | **positive** |
| *Enterobacter cloacae* ATCC 13047 | **negative** | **negative** | **positive** |
| *Staphylococcus saprophyticus,* clinical strain | negative | negative | **positive** |
| *Enterococcus faecium,* clinical strain | negative | negative | **positive** |
| *Enterococcus faecalis,* clinical strain | negative | negative | **positive** |
| *Proteus mirabilis* ATCC 29906 | negative | negative | **positive** |
| *Lactobacillus acidophilus, clinical* strain | negative | negative | **positive** |
| *Bacillus subtilis,* clinical strain | negative | NT | **positive** |
| *Gardnerella vaginalis,* clinical strain | negative | negative | **positive** |
| *Neisseria gonorrhoeae,* 5 clinical strains | negative | negative | **positive** |
| *Neisseria cinerea* DSMZ 4630 | NT | negative | **positive** |
| *Neisseria lactamica* DSMZ 4691 | NT | negative | **positive** |
| *Neisseria sicca,* clinical strain | NT | negative | **positive** |
| *Neisseria meningitidi,* clinical strain | NT | negative | **positive** |
| *Staphylococcus aureus* ATCC 25923 | negative | negative | **positive** |
| *Staphylococcus simulans* strain Institut Pasteur | NT | negative | **positive** |
| *Pseudomonas aeruginosa,* ATCC 27853 | negative | negative | **positive** |
| *Klebsiella pneumoniae,* CIP 104298 | negative | NT | **positive** |
| *Strepcococcus agalactiae,* ATCC 12403 | negative | negative | **positive** |
| *Streptococcus bovis* CIP 105065 | negative | **negative** | **positive** |
| *Acinetobacter baumanii,* ATCC 49139 | negative | **negative** | **positive** |
| *Staphylococcus epidermidis* ATCC 49139 | negative | **negative** | **positive** |
| *Candida albicans* clinical strain | negative | negative | **positive** |
| *Mycoplasma pneumoniae* clinical strain | negative | negative | **positive** |
| *Neisseria mucosa* clinical strain | negative | negative | **positive** |
| *Rhodococcus equi* clinical strain | negative | negative | **positive** |
| *Moraxella catarrhalis,* clinical strain | negative | negative | **positive** |
| *Mycoplasma genitalium,* strain 0-37, ATCC 33530 | negative | **Positive** | **positive** |
| *Mycoplasma genitalium,* strain 2282, clinical strain, Statens Serum | NT | **positive** | **positive** |
| Institut, Danemark | | | |
| *Mycoplasma genitalium,* strain 2288, clinical strain, Statens Serum | NT | **positive** | **positive** |
| Institut, Danemark | | | |
| *Mycoplasma genitalium,* strain 2300, clinical strain, Statens Serum | NT | **positive** | **positive** |
| Institut, Danemark | | | |
| *Mycoplasma genitalium,* strain 2321, clinical strain, Statens Serum | NT | **positive** | **positive** |
| Institut, Danemark | | | |
| *Mycoplasma genitalium,* strain 2341, clinical strain, Statens Serum | NT | **positive** | **positive** |
| Institut, Danemark | | | |
| *Mycoplasma genitalium,* strain M30, clinical strain, CHU Bordeaux | NT | **positive** | **positive** |
| *Mycoplasma genitalium,* strain UTMB1, clinical strain, CHU | NT | **positive** | **positive** |
| Bordeaux | | | |
| *Mycoplasma genitalium,* strain TW-10-51, clinical strain, CHU Bordeaux | NT | **positive** | **positive** |
| *Mycoplasma orale* ATCC 23714 | negative | negative | **positive** |
| *Mycoplasma fermentans* strain PG18, clinical strain, CHU Bordeaux | NT | negative | **positive** |
| *Mycoplasma penetrans* strain GTU64, clinical strain, CHU Bordeaux | NT | negative | **positive** |

| | | | |
|---|---|---|---|
| NT: not tested CHU Bordeaux = Hospital of Bordeaux, France | | | |

### 2.2.3. Specificity test for the NG system in real-time simplex PCR:

The amplicon is of 252 bp.

The NG primers and probes of the invention have been assayed in real-time PCR on 55 different NG strains. All results were positive.

The NG primers and probes of the invention have also been tested on several *Neisseria* species other than NG. Results are shown in table 3 below. No amplification was obtained with the following species: *N. sicca* (3 strains), *N. polysaccharia* (1 strain), *N. subflava* (4 strains), *N. mucosa* (3 strains), *N. cinerea* (1 strain), and *N. lactamica* (2 strains).

Among the 16 *N. meningitidis* strains that have been tested, 10 gave a positive response (NM cross-reaction).

**Table 3: specificity of the NG system in simplex PCR with different Neisseria species**

| **Strain** | **Simplex NAG** | **16S rRNA** |
|---|---|---|
| **Human DNA** | negative | positive |
| *N. cinerea (1850)* DSMZ 4630 | negative | positive |
| *N. lactamica* (1851) DSMZ 4691 | negative | positive |
| *N. lactamica* (1874) | negative | positive |
| *N.* ***meningitidis* 10 clinical strains** | **positive** | **positive** |
| *N. meningitidis* 6 clinical strains | negative | positive |
| *N. mucosa* (1853, 1870, 1871) | negative | positive |
| *N. polysaccharia* (1852) CIP 100.113T | negative | positive |
| *N. sicca* (1854, 1872, 1873) | negative | positive |
| *N. subflava* (1855, 1876, 1877) | negative | positive |
| *N. subflava* (1875) CIP 73.13 | negative | positive |
| ***N. gonorrhoeae,* 55 clinical strains** | **positive** | **positive** |

The specificity of the NG primers of the invention has been assayed in end-point PCR, followed by deposition on gel, with 13 bacterial strains that do not belong to the *Neisseria* genus. No amplification has been detected. Results are reported in table 4 below.

**Table 4: specificity relative to non-Neisseria strains**

| **Strain** | **End-point PCR for NG** | **16S rRNA** |
|---|---|---|
| Human DNA | negative | // |
| *Escherichia coli* ATCC 25922 | negative | **positive** |
| *Enterobacter cloacae* ATCC 13047 | negative | **positive** |
| *Staphylococcus saprophyticus,* clinical strain | negative | **positive** |
| *Enterococcus faecium,* clinical strain | negative | **positive** |
| *Enterococcus faecalis,* clinical strain | negative | **positive** |
| *Proteus mirabilis* ATCC 29906 | negative | **positive** |
| *Bacillus subtilis,* clinical strain | negative | **positive** |
| *Garnerella vaginalis,* clinical strain | negative | **positive** |
| *Pseudomonas aeruginosa,* ATCC 27853 | negative | **positive** |
| *Strepcococcus agalactiae,* ATCC 12403 | negative | **positive** |
| *Acinetobacter baumanii,* ATCC 49139 | negative | **positive** |
| *Staphylococcus epidermidis* ATCC 49139 | negative | **positive** |
| *Moraxella catarrhalis,* clinical strain | negative | **positive** |

### 2.2.4. Results in multiplex PCR:

### PCR sensitivity

The CT, MG, NG and IC primers and probes of the invention have been assayed in quadruplex on a mix of a pre-determined quantity of CT, MG and NG DNA (the experiment was made in duplicate).

Results are as follows:

**Table 5: CT probe (FAM)**

| **Copy number** | quadruplex mix | |
|---|---|---|
| | **Ct** | **RFU** |
| **1000** | 31.75 +/-0.10 | 400 |
| **100** | 35.20 +/- 0.63 | 300 |
| **10** | 41.30 +/- 2.62 | 100 |
| **1** | ND | |

| | | |
|---|---|---|
| ND: not detected ⇒ sensitivity is of 10 copy per PCR | | |

**Table 6: MG probe (TAMRA)**

| **Copy numbers** | **quadruplex mix** | |
|---|---|---|
| | **Ct** | **RFU** |
| **1000** | 36.93 +/- 2.07 | 140 |
| **100** | 40.63 +/- 0.85 | 100 |
| **10** | ND | |
| **1** | ND | |

| | | |
|---|---|---|
| ND: not detected ⇒ sensitivity is of 100 copies per PCR. | | |

**Table 7: NG probe (ATTO 647N)**

| **Copy number** | **Quadruplex mix** | |
|---|---|---|
| | **Ct** | **RFU** |
| **1000** | 27.30+/- 0.28 | 350 |
| **100** | 31.33 +/-0.43 | 325 |
| **10** | 34.4 +/- 0.37 | 250 |
| **1** | 37.95+/-0.51 | 200 |

| | | |
|---|---|---|
| ⇒ Sensitivity is of 1 copy per PCR | | |

**Table 8: IC probe (ATTO- 590)**

| **Copy number** | **Quadruplex mix** | |
|---|---|---|
| | **Ct** | **RFU** |
| **1000** | 34.35 +/- 0.24 | 225 |
| **100** | 34.15 +/- 0.37 | |
| **10** | 34.53 +/-0.17 | |
| **1** | 34.65 +/-0.40 | |

| | | |
|---|---|---|
| ⇒ There is no Ct variation for the IC, whatever quantity of DNA is being used. | | |

### 3. EXAMPLE 3: specific and sensitivity of CT, MG and NG real-time amplification systems of the invention:

- specificity in real-time multiplex PCR (CT+MG+NG+IC real-time amplification systems of the invention, together in multiplex in the same mix, tested on a panel of bacterial strains);
- sensitivity in real-time multiplex PCR (CT+MG+NG+IC systems together in multiplex in the same mix, tested on a mix of a pre-determined quantity of CT, MG and NG DNA).

### 3.1. Material and Methods:

### Real Time Multiplex PCR

* composition of the first mix
   In a1X PCR mix, add the following primers:
   CT forward primer U-PC 5580 (SEQ ID NO: 7) at 0.125 µM,
   CT reverse primer L-PC 5754 (SEQ ID NO: 12) at 0.5 µM,
   MG forward primer U-MG 1144 (SEQ ID NO: 21) at 0.5 µM,
   MG reverse primer L-MG 1283 (SEQ ID NO: 24) at 0.5 µM,
   NG forward primer U-pilE 159 (SEQ ID NO: 44) at 0.85 µM,
   NG reverse primer L-pilE 406 (SEQ ID NO: 47) at 0.25 µM,
   IC forward primer IS 368 (SEQ ID NO: 49) at 0.5 µM, and
   IC reverse primer IS 569 (SEQ ID NO: 52) at 0.5 µM.
The probes are used at the following concentrations:
   CT probe SCT 175b (SEQ ID NO: 9) at 0.15 µM (fluorophore FAM),
   MG probe MGBR 140c (SEQ ID NO: 23) at 0.2 µM (fluorophore TAMRA
   NG probe pilEc (SEQ ID NO: 46) at 0.2 mM (fluorophore ATTO 647N) and
   IC probe SIMB (SEQ ID NO: 51) at 0.15 µM (fluorophore ATTO-590),
* composition of the second mix
   In a 1X PCR mix, add the following primers:
   CT forward primer U-PC 5580 (SEQ ID NO: 7) at 0.125 µM,
   CT reverse primer L-PC 5754 (SEQ ID NO: 12) at 0.5 µM,
   MG forward primer U-MG 1087 (SEQ ID NO: 27) at 0.5 µM,
   MG reverse primer L-MG 1249 (SEQ ID NO: 30) at 0.5 µM,
   NG forward primer U-pilE 159 (SEQ ID NO: 44) at 0.85 µM,
   NG reverse primer L-pilE 406 (SEQ ID NO: 47) at 0.25 µM,
   IC forward primer IS 368 (SEQ ID NO: 49) at 0.5 µM, and
   IC reverse primer IS 569 (SEQ ID NO: 52) at 0.5 µM.
The probes are used at the following concentrations:
   CT probe SCT 175b (SEQ ID NO: 9) at 0.15 µM (fluorophore FAM),
   MG probe MGBR 186j (SEQ ID NO: 29) at 0.2 µM (fluorophore TAMRA)
   NG probe pilEc (SEQ ID NO: 46) at 0.2 µM (fluorophore ATTO 647N) and
   IC probe SIMB (SEQ ID NO: 51) at 0.15 µM (fluorophore ATTO-590),
* composition of the third mix
   In a 1X PCR mix, add the following primers:
   CT forward primer U-PC 5580 (SEQ ID NO: 7) at 0.125 µM,
   CT reverse primer L-PC 5754 (SEQ ID NO: 12) at 0.5 µM,
   MG forward primer U-MG 1527 (SEQ ID NO: 33) at 0.5 µM,
   MG reverse primer L-MG 1704 (SEQ ID NO: 36) at 0.5 µM,
   NG forward primer U-pilE 159 (SEQ ID NO: 44) at 0.85 µM,
   NG reverse primer L-pilE 406 (SEQ ID NO: 47) at 0.25 µM,
   IC forward primer IS 368 (SEQ ID NO: 49) at 0.5 µM, and
   IC reverse primer IS 569 (SEQ ID NO: 52) at 0.5 µM.
The probes are used at the following concentrations:
   CT probe SCT 175b (SEQ ID NO: 9) at 0.15 µM (fluorophore FAM),
   MG probe MGBR 178q (SEQ ID NO: 35) at 0.2 µM (fluorophore TAMRA NG probe pilEc (SEQ ID NO: 46) at 0.2 µM (fluorophore ATTO 647N) and
   IC probe SIMB (SEQ ID NO: 51) at 0.15 µM (fluorophore ATTO-590),
* composition of the fourth mix
   In a 1X PCR mix, add the following primers:
   CT forward primer U-PC 5580 (SEQ ID NO: 7) at 0.125 µM,
   CT reverse primer L-PC 5754 (SEQ ID NO: 12) at 0.5 µM,
   MG forward primer U-MG 1501 (SEQ ID NO: 39) at 0.5 µM,
   MG reverse primer L-MG 1704 (SEQ ID NO: 36) at 0.5 µM,
   NG forward primer U-pilE 159 (SEQ ID NO: 44) at 0.85 µM,
   NG reverse primer L-pilE 406 (SEQ ID NO: 47) at 0.25 µM,
   IC forward primer IS 368 (SEQ ID NO: 49) at 0.5 µM, and
   IC reverse primer IS 569 (SEQ ID NO: 52) at 0.5 µM.
The probes are used at the following concentrations:
   CT probe SCT 175b (SEQ ID NO: 9) at 0.15 µM (fluorophore FAM),
   MG probe MGBR 204u (SEQ ID NO: 41) at 0.2 µM (fluorophore TAMRA)
   NG probe pilEc (SEQ ID NO: 46) at 0.2 µM (fluorophore ATTO 647N) and
   IC probe SIMB (SEQ ID NO: 51) at 0.15 µM (fluorophore ATTO-590),
The following reactants are further added:
   5% Glycerol, 0.3 % PVP 10, 2U of Taq Polymerase, 1 mM dNTP and 5 mM final of MgCl_{2.}
   10 µL of DNA are added to this mix.
* thermocycling:
   First cycle: 15" at 95°C
   Second cycle: 30" at 95°C
   Third cycle: 50" at 58°C
   Fourth cycle: 30" at 72°C
   Repeat 50 times from cycle 2 to cycle 4
   16S rRNA PCR
   cf. chapter 1.6.1

### 3.2. Results

### 3.2.1. Specificity test for the MG systems in real-time multiplex PCR

The MG primers and probes of the invention detect the nine MG strains that have been tested (strains G-37, 2282, 2288, 2300, 2321, 2341, M30, UTMB1 and TW-10-51).

The MG primers and probes of the invention have also been tested on 27 different bacterial strains which may be found in the urogenital sphere, and were shown to be non cross-reactive. For these 27 different bacterial strains, presence of DNA was confirmed by end-point PCR using the 16S rRNA primers (SIR and S1F primers), followed by deposition on gel.

Results are reported in the tables below.

**Table 9: specificity of the MG system in real-time multiplex PCR**

| | **First Multiplex** | **Second multiplex** | **Third multiplex** | **Fourth multiplex** | **16S rRNA** |
|---|---|---|---|---|---|
| Amplicon size | 140 nt | 186 nt | 178 nt | 204 nt | |
| Strain | | | | | |
| human DNA | negative | negative | negative | negative | // |
| *Chlamydia trachomatis,* serovar D, strain | negative | negative | negative | negative | positive |
| UW-3/Cx, ATCC VR 885 | | | | | |
| *Chlamydia trachomatis,* serovar L1, clinical | negative | negative | negative | negative | positive |
| strain, CHU Bordeaux | | | | | |
| *Escherichia coli* ATCC 25922 | negative | negative | negative | negative | positive |
| *Enterobacter cloacae* ATCC 13047 | negative | negative | negative | negative | positive |
| *Staphylococcus saprophyticus,* clinical | negative | negative | negative | negative | positive |
| strain | | | | | |
| *Enterococcus faecium,* clinical strain | negative | negative | negative | negative | positive |
| *Enterococcus faecalis,* clinical strain | negative | negative | negative | negative | positive |
| *Proteus mirabilis* ATCC 29906 | negative | negative | negative | negative | positive |
| *Lactobacillus acidophilus,* clinical strain | negative | negative | negative | negative | positive |
| *Bacillus subtilis,* clinical strain | negative | negative | negative | negative | **positive** |
| *Gardnerella vaginalis,* clinical strain | negative | negative | negative | negative | **positive** |
| *Neisseria gonorrhoeae, 1* clinical strain | negative | negative | negative | negative | **positive** |
| *Staphylococcus aureus* ATCC 25923 | negative | negative | negative | negative | **positive** |
| *Pseudomonas aeruginosa,* ATCC 27853 | negative | negative | negative | negative | **positive** |
| *Klebsiella pneumoniae,* CIP 104298 | negative | negative | negative | negative | **positive** |
| *Streptococcus agalactiae,* ATCC 12403 | negative | negative | negative | negative | **positive** |
| *Streptococcus bovis* CIP 105065 | negative | negative | negative | negative | **positive** |
| *Acinetobacter baumanii,* ATCC 49139 | negative | negative | negative | negative | **positive** |
| *Staphylococcus epidermidis* ATCC 49139 | negative | negative | negative | negative | **positive** |
| *Candida albicans* clinical strain | negative | negative | negative | negative | **positive** |
| *Mycoplasma pneumoniae* clinical strain | negative | negative | negative | negative | **positive** |
| *Neisseria mucosa* clinical strain | negative | negative | negative | negative | **positive** |
| *Rhodococcus equi* clinical strain | negative | negative | negative | negative | **positive** |
| *Moraxella catarrhalis,* clinical strain | negative | negative | negative | negative | **positive** |
| *Mycoplasma genitalium,* strain G-37, ATCC | **positive** | **positive** | **positive** | **positive** | **positive** |
| 33530 | | | | | |
| *Mycoplasma genitalium,* strain 2282, | **positive** | **positive** | **positive** | **positive** | **positive** |
| clinical strain, Statens Serum Institut, | | | | | |
| Danemark | | | | | |
| *Mycoplasma genitalium,* strain 2288, | **positive** | **positive** | **positive** | **positive** | **positive** |
| clinical strain, Statens Serum Institut, | | | | | |
| Danemark | | | | | |
| *Mycoplasma genitalium,* strain 2300, | **positive** | **positive** | **positive** | **positive** | **positive** |
| clinical strain, Statens Serum Institut, | | | | | |
| Danemark | | | | | |
| *Mycoplasma genitalium,* strain 2321, | **positive** | **positive** | **positive** | **positive** | **positive** |
| clinical strain, Statens Serum Institut, | | | | | |
| Danemark | | | | | |
| *Mycoplasma genitalium,* strain 2341, | **positive** | **positive** | **positive** | **positive** | **positive** |
| clinical strain, Statens Serum Institut, | | | | | |
| Danemark | | | | | |
| *Mycoplasma genitalium,* strain M30, clinical | **positive** | **positive** | **positive** | **positive** | **positive** |
| strain, CHU Bordeaux | | | | | |
| *Mycoplasma genitalium,* strain UTMH1, | **positive** | **positive** | **positive** | **positive** | **positive** |
| clinical strain, CHU Bordeaux | | | | | |
| *Mycoplasma genitalium,* strain TW-10-51, | **Positive** | **positive** | **Positive** | **positive** | **positive** |
| clinical strain, CHU Bordeaux | | | | | |
| *Mycoplasma orale* ATCC 23714 | negative | negative | negative | negative | **positive** |
| *Mycoplasma fermentans* strain PG18, clinical strain, CHU Bordeaux, | negative | negative | negative | negative | **positive** |
| *Mycoplasma penetrans* strain GTU64, | negative | negative | negative | negative | **positive** |
| clinical strain, CHU Bordeaux | | | | | |

| | | | | | |
|---|---|---|---|---|---|
| NT: not tested CHU Bordeaux = Hospital of Bordeaux, France | | | | | |

**Table 10: specificity of the CT system in real-time multiplex PCR**

| | **First Multiplex** | **Second multiplex** | **Third multiplex** | **Fourth multiplex** | **16S rRNA** |
|---|---|---|---|---|---|
| Amplicon size | 175 nt | 175 nt | 175 nt | 175 nt | |
| **Strain** | | | | | |
| human DNA | negative | negative | negative | negative | // |
| *Chlamydia trachomatis,* serovar D, strain | **positive** | **positive** | **positive** | **positive** | **positive** |
| UW-3/Cx, ATCC VR 885 | | | | | |
| *Chlamydia trachomatis,* serovar L1, clinical | **positive** | **positive** | **positive** | **positive** | **positive** |
| strain, CHU Bordeaux | | | | | |
| *Escherichia coli* ATCC 25922 | negative | negative | negative | negative | **positive** |
| *Enterobacter cloacae* ATCC 13047 | negative | negative | negative | negative | **positive** |
| *Staphylococcus saprophyticus,* clinical | negative | negative | negative | negative | **positive** |
| strain | | | | | |
| *Enterococcus faecium,* clinical strain | negative | negative | negative | negative | **positive** |
| *Enterococcus faecalis,* clinical strain | negative | negative | negative | negative | **positive** |
| *Proteus mirabilis* ATCC 29906 | negative | negative | negative | negative | **positive** |
| *Lactobacillus acidophilus,* clinical strain | negative | negative | negative | negative | **positive** |
| *Bacillus subti*/*is,* clinical strain | negative | negative | negative | negative | **positive** |
| *Gardnerella vaginalis,* clinical strain | negative | negative | negative | negative | **positive** |
| *Neisseria gonorrhoeae,* 1 clinical strain | negative | negative | negative | negative | **positive** |
| *Staphylococcus aureus* ATCC 25923 | negative | negative | negative | negative | **positive** |
| *Pseudomonas aeruginosa,* ATCC 27853 | negative | negative | negative | negative | **positive** |
| *Klebsiella pneumoniae,* CIP 104298 | negative | negative | negative | negative | **positive** |
| *Strepcococcus agalactiae,* ATCC 12403 | negative | negative | negative | negative | **positive** |
| *Streptococcus bovis* CIP 105065 | negative | negative | negative | negative | **positive** |
| *Acinetobacter baumanii,* ATCC 49139 | negative | negative | negative | negative | **positive** |
| *Staphylococcus epidermidis* ATCC 49139 | negative | negative | negative | negative | **positive** |
| *Candida albicans* clinical strain | negative | negative | negative | negative | **positive** |
| *Mycoplasma pneumoniae* clinical strain | negative | negative | negative | negative | **positive** |
| *Neisseria mucosa* clinical strain | negative | negative | negative | negative | **positive** |
| *Rhodococcus equi* clinical strain | negative | negative | negative | negative | **positive** |
| *Moraxella catarrhalis,* clinical strain | negative | negative | negative | negative | **positive** |
| *Mycoplasma genitalium,* strain G-37, ATCC | negative | negative | negative | negative | **positive** |
| 33530 | | | | | |
| *Mycoplasma genitalium,* strain 2282, | negative | negative | negative | negative | **positive** |
| clinical stain, Statens Serum Institut, | | | | | |
| Danemark | | | | | |
| *Mycoplasma genitalium,* strain 2288, | negative | negative | negative | negative | **positive** |
| clinical strain, Statens Serum Institut, | | | | | |
| Danemark | | | | | |
| *Mycoplasma genitalium,* strain 2300, | negative | negative | negative | negative | **positive** |
| clinical strain, Statens Serum Institut, | | | | | |
| Danemark | | | | | |
| *Mycoplasma genitalium,* strain 2321, | negative | negative | negative | negative | **positive** |
| clinical strain, Statens Serum Institut, | | | | | |
| Danemark | | | | | |
| *Mycoplasma genitalium,* strain 2341, | negative | negative | negative | negative | **positive** |
| clinical strain, Statens Serum Institut, | | | | | |
| Danemark | | | | | |
| *Mycoplasma genitalium,* strain M30, clinical | negative | negative | negative | negative | **positive** |
| strain, CHU Bordeaux | | | | | |
| *Mycoplasma genitalium,* strain UTMB1, | negative | negative | negative | negative | **positive** |
| clinical strain, CHU Bordeaux | | | | | |
| *Mycoplasma genitalium,* strain TW-10-51, | negative | negative | negative | negative | **positive** |
| clinical strain, CHU Bordeaux | | | | | |
| *Mycoplasma orale* ATCC 23714 | negative | negative | negative | negative | **positive** |
| *Mycoplasma fermentans* strain PG18, clinical strain, CHU Bordeaux | negative | negative | negative | negative | **positive** |
| *Mycoplasma penetrans* strain GTU64, | negative | negative | negative | negative | **positive** |
| clinical strain, CHU Bordeaux | | | | | |

| | | | | | |
|---|---|---|---|---|---|
| NT: not tested CHU Bordeaux = Hospital of Bordeaux, France | | | | | |

**Table 11: specificity of the NG system in real-time multiplex PCR**

| | **First Multiplex** | **Second multiplex** | **Third multiplex** | **Fourth multiplex** | **16S rRNA** |
|---|---|---|---|---|---|
| Amplicon size | 252nt | 252nt | 252nt | 252nt | |
| **Strain** | | | | | |
| human DNA | negative | negative | negative | negative | // |
| *Chlamydia trachomatis,* serovar D, strain | negative | negative | negative | negative | **positive** |
| UW-3/Cx, ATCC VR 885 | | | | | |
| *Chlamydia trachomatis,* serovar L1, clinical | negative | negative | negative | negative | **positive** |
| strain, CHU Bordeaux | | | | | |
| *Escherichia coli* ATCC 25922 | negative | negative | negative | negative | **positive** |
| *Enterobacter cloacae* ATCC 13047 | negative | negative | negative | negative | **positive** |
| *Staphylococcus* s*aprophyticus,* clinical | negative | negative | negative | negative | **positive** |
| strain | | | | | |
| *Enterococcus faecium,* clinical strain | negative | negative | negative | negative | **positive** |
| *Enterococcus faecalis,* clinical strain | negative | negative | negative | negative | **positive** |
| *Proteus mirabilis* ATCC 29906 | negative | negative | negative | negative | **positive** |
| *Lactobacillus acidophilus,* clinical strain | negative | negative | negative | negative | **positive** |
| *Bacillus subtilis,* clinical strain | negative | negative | negative | negative | **positive** |
| *Gardnerella vaginalis,* clinical strain | negative | negative | negative | negative | **positive** |
| *Neisseria gonorrhoeae,* 1 clinical strain | positive | positive | Positive | positive | **positive** |
| *Staphylococcus aureus* ATCC 25923 | negative | negative | negative | negative | **positive** |
| *Pseudomonas aeruginosa,* ATCC 27853 | negative | negative | negative | negative | **positive** |
| *Klebsiella pneumoniae,* CIP 104298 | negative | negative | negative | negative | **positive** |
| *Strepcococcus agalactiae,* ATCC 12403 | negative | negative | negative | negative | **positive** |
| *Streptococcus bovis* CIP 105065 | negative | negative | negative | negative | **positive** |
| *Acinetobacter baumanii,* ATCC 49139 | negative | negative | negative | negative | **positive** |
| *Staphylococcus epidermidis* ATCC 49139 | negative | negative | negative | negative | **positive** |
| *Candida albicans* clinical strain | negative | negative | negative | negative | **positive** |
| *Mycoplasma pneumoniae* clinical strain | negative | negative | negative | negative | **positive** |
| *Neisseria mucosa* clinical strain | negative | negative | negative | negative | **positive** |
| *Rhodococcus equi* clinical strain | negative | negative | negative | negative | **positive** |
| *Moraxella catarrhalis,* clinical strain | negative | negative | negative | negative | **positive** |
| *Mycoplasma genitalium,* strain G-37, ATCC | negative | negative | negative | negative | **positive** |
| 33530 | | | | | |
| *Mycoplasma genitalium,* strain 2282, | negative | negative | negative | negative | **positive** |
| clinical strain, Statens Serum Institut, | | | | | |
| Danemark | | | | | |
| *Mycoplasma genitalium,* strain 2288, | negative | negative | negative | negative | positive |
| clinical strain, Statens Serum Institut, | | | | | |
| Danemark | | | | | |
| *Mycoplasma genitalium,* strain 2300, | negative | negative | negative | negative | positive |
| clinical strain, Statens Serum Institut, | | | | | |
| Danemark | | | | | |
| *Mycoplasma genitalium,* strain 2321, | negative | negative | negative | negative | positive |
| clinical strain, Statens Serum Institut, | | | | | |
| Danemark | | | | | |
| *Mycoplasma genitalium,* strain 2341, | negative | negative | negative | negative | positive |
| clinical strain, Statens Serum Institut, | | | | | |
| Danemark | | | | | |
| *Mycoplasma genitalium,* strain M30, clinical | negative | negative | negative | negative | positive |
| strain, CHU Bordeaux | | | | | |
| *Mycoplasma genitalium,* strain UTMB1, | negative | negative | negative | negative | positive |
| clinical strain, CHU Bordeaux | | | | | |
| *Mycoplasma genitalium,* strain TW-10-51, | negative | negative | negative | negative | positive |
| clinical strain, CHU Bordeaux | | | | | |
| *Mycoplasma orale* ATCC 23714 | negative | negative | negative | negative | positive |
| *Mycoplasma fermentans* strain PG18, | negative | negative | negative | negative | positive |
| clinical strain, CHU Bordeaux | | | | | |
| *Mycoplasma penetrans* strain GTU64, | negative | negative | negative | negative | positive |
| clinical strain, CHU Bordeaux | | | | | |

| | | | | | |
|---|---|---|---|---|---|
| NT: not tested CHU Bordeaux = Hospital of Bordeaux, France | | | | | |

### 3.2.2. Results in quadruplex mix PCR: sensitivity

The CT, MG, NG and IC primers and probes of the invention have been assayed in quadruplex on a mix of a pre-determined quantity of CT, MG and NG DNA (the experiment was made in quadricate). Four multiplex are tested, each multiplex containing one MG simplex described on top.

Results are as follows:

**Table 12: MG probe (TAMRA)**

| **Copy** | **First quadruplex** | **Second quadruplex** | **Third quadruplex** | **Fourth quadruplex** |
|---|---|---|---|---|
| **number** | **mix** | **mix** | **mix** | **mix** |
| | **Probe MGBR140c** | **Probe MGBR 186j** | **Probe MGBR 178q** | **Probe MGBR 204u** |
| | **Ct** | **Ct** | **Ct** | **Ct** |
| **1000** | 33.48+/-2.03 | 34.58+/-0.75 | 37.68+/-1.11 | 37.78+/-0.64 |
| **100** | 39.75+/-2.92 | 40.05+/-2.01 | 42.23+/-3.81 | 44.20+/-2.39 |
| **10** | 41.73+/-0.46 | ND | ND | ND |
| **1** | ND | ND | ND | ND |

| | | | | |
|---|---|---|---|---|
| ND: not detected ⇒ sensitivity is of 10 copies per PCR for the first quadruplex mix (Probe MGBR140c and 100 copies per PCR for other multiplex | | | | |

**Table 13: CT probe (FAM)**

| **Copy** | **First quadruplex** | **Second quadruplex** | **Third quadruplex** | **Fourth quadruplex** |
|---|---|---|---|---|
| **number** | **mix** | **mix** | **mix** | **mix** |
| | **Probe MGBR140c** | **Probe MGBR 186j** | **Probe MGBR 178q** | **Probe MGBR 204u** |
| | **Ct** | **Ct** | **Ct** | **Ct** |
| **1000** | 30.13+/-0.54 | 31.63+/-0.34 | 30.85+/-0.52 | 31.33+/-0.29 |
| **100** | 33.95+/-0.62 | 34.55+/-0.87 | 33.63+/-0.74 | 34.88+/-0.71 |
| **10** | 37.53+/-1.66 | 41.40+/-3.58 | 42.47+/-0.51 | 39.55+/-3.67 |
| **1** | ND | ND | ND | ND |

| | | | | |
|---|---|---|---|---|
| ND: not detected ⇒ sensitivity is of 10 copy per PCR for all multiplex tested | | | | |

**Table 14: NG probe (ATTO 647N)**

| **copy** | **First quadruplex** | **Second quadruplex** | **Third quadruplex** | **Fourth quadruplex** |
|---|---|---|---|---|
| **number** | **mix** | **mix** | **mix** | **mix** |
| | **Probe MGBR140c** | **Probe MGBR 186j** | **Probe MGBR 178q** | **Probe MGBR 204u** |
| | **Ct** | **Ct** | **Ct** | **Ct** |
| **1000** | 26.80+/-0.33 | 27.13+/-0.40 | 27.20+/-0.29 | 27.28+/-0.22 |
| **100** | 29.85+/-0.10 | 30.15+/-0.75 | 30.20+/-0.64 | 30.35+/-0.61 |
| **10** | 32.63+/-1.64 | 33.20+/-0.43 | 32.58+/-0.22 | 34+/-0.29 |
| **1** | 36.33+/-0.61 | 36.15+/-0.13 | 36.33+/-1.34 | 36.53+/-0.39 |

| | | | | |
|---|---|---|---|---|
| ND: not detected ⇒ Sensitivity is of 1 copy per PCR for all multiplex tested | | | | |

**Table 15: IC probe (ATTO- 590) ⇒ There is no Ct variation for the IC, whatever quantity of DNA is being used**

| **Copy** | **First quadruplex** | **Second quadruplex** | **Third quadruplex** | **Fourth quadruplex** |
|---|---|---|---|---|
| **number** | **mix** | **mix** | **mix** | **mix** |
| | **Probe MGBR140c** | **Probe MGBR 186j** | **Probe MGBR 178q** | **Probe MGBR 204u** |
| | **Ct** | **Ct** | **Ct** | **Ct** |
| **1000** | 34.38+/-0.17 | 35.03+/-0.30 | 35.25+/-0.64 | 34.80+/-0.22 |
| **100** | 34+/-0.18 | 34.90+/-0.41 | 34.75+/-0.13 | 34.63+/-0.15 |
| **10** | 34.08+/-0.31 | 34.38+/-0.28 | 34.33+/-0.22 | 34.80+/-0.20 |
| **1** | 34.55+/-0.13 | 34.88+/-0.26 | 34.65+/-0.31 | 35.40+/-0.28 |

### 4. EXAMPLE 4: samples collected from patients (first void urine tests)

CT+MG+NG+IC real-time amplification systems of the invention, together in multiplex in the same mix;
compared to Roche Amplicor CT test, to in house MG PCR test, and to NG culture test.

### 4.1. Material and Methods:

### 4.1.1. Samples:

Samples are collected from human patients (Saint Louis Hospital, France). The first void urines are stored at- 20°C until use.
19 samples (1 to 19) are tested in multiplex. A negative control (sample without DNA), a positive *C. trachomatis* control (only *C. trachomatis* DNA), a positive *M. genitalium* control (only *M. genitalium* DNA) and a *N. gonorrhoeae* (only *N. gonorrhoeae* DNA) positive control are tested in the same run.

### 4.1.2. Description of the extraction method:

*cf.* Example 2

### 4.1.3. Prior art CT or MG or NG detection methods:

CT detection: COBAS Amplicor^{®} CT test available from Roche Diagnostics (Amplicor CT/NG amplification kit ref: ART: 07 59 41 4, and Cobas Amplicor CT detection kit ref.: Art 07 5749 7.)

MG detection: in-house MG PCR test: A primer set, MgPa-1 (5' - AGT TGA TGA AAC CTT AAC CCC TTG G - 3'; SEQ ID NO: 55) and MgPa-3 (5'- CCG TTG AGG GGT TTT CCA TTT TTG C -3'; SEQ ID NO: 56) was used to amplify the 281 base pair fragment of the major adhesion gene (Jensen JS, Uldum SA, J Søndergård-Andersen, J Vuust, and K Lind, "Polymerase chain reaction for detection of Mycoplasma genitalium in clinical samples" J. Clin. Microbiol., 1991; 29: 46-50). The specificity of the 281 base pair amplified fragment was verified by hybridization with the 25 mer MgPa 2 probe (5'- GAC CAT CAA GGT ATT TCT CAA CAG C 3'; SEQ ID NO: 57), labelled with fluorescein - 11 dUTP with use of the ECL oligonucleotide 3- tail labelling system (Amersham International, Amersham UK). The specimens from which the 281 base pair DNA fragment, visible after Southern blot hybridization with the internal probe, was obtained were regarded as positive (Casin I, Vexiau-Robert D, De La Salmoniere P, Eche A, Grandry B, Janier M. "High prevalence of Mycoplasma genitalium in the lower genitourinary tract of women attending a sexually transmitted disease clinic in Paris, France", Sex Transm Dis., June 2002; 29: 353-359).

NG detection: standard NG culture test, as described in *"*Performance Standards for Antimicrobial Susceptibility Testing; sixteenth Information Supplement", January 2006, p130 Table 2F, edited by Clinical and Laboratory Standards Institute (CLSI) antimicrobial susceptibility testing standards M2-A9 and M7-A7.

### 4.1.4. Real-time multiplex PCR of the invention:

*cf.* Example 2.

### Real-time multiplex PCR

* composition of the mix
   In a 1X PCR mix, add the following primers:
   CT forward primer U-PC 5580 (SEQ ID NO: 6) at 0.125 µM,
   CT reverse primer L-PC 5754 (SEQ ID NO: 12) at 0.5 µM,
   MG forward primer U-MG 1320 (SEQ ID NO: 15) at 0.025 µM,
   MG reverse primer L-MG 1578 (SEQ ID NO: 18) at 1.25 µM,
   NG forward primer U-pilE 159 (SEQ ID NO: 44) at 0.85 µM,
   NG reverse primer L-pilE 406 (SEQ ID NO: 47) at 0.25 µM,
   IC forward primer IS 368 (SEQ ID NO: 49) at 0.5 µM, and
   IC reverse primer IS 569 (SEQ ID NO: 52) at 0.5 µM.
The probes are used at the following concentrations:
   CT probe SCT 175b (SEQ ID NO: 9) at 0.15 µM (fluorophore FAM),
   MG probe SFMG 258c (SEQ ID NO: 17) at 0.2 µM (fluorophore TAMRA), and
   NG probe pilEc (SEQ ID NO: 46) at 0.2 µM (fluorophore ATTO 647N).
   IC probe SIMB (SEQ ID NO: 51) at 0.15 µM (fluorophore ATTO-590),
The following reactants are further added:
   5% Glycerol, 0.3 % PVP 10, 2U of Taq Polymerase, 1 mM dNTP and 5 mM final of MgCl₂.
   10 µL of DNA are added to this mix.
* thermocycling:
   First cycle: 15" at 95°C
   Second cycle: 30" at 95°C
   Third cycle: 50" at 58°C
   Fourth cycle: 30" at 72°C
   Repeat 50 times from cycle 2 to cycle 4

### 4.2. Results: Table 16: (ND: not detected, SD: Standard deviation, IC: internal control)

| | **First PCR** **result** | **Second** **PCR result** | **Culture** **result** | **Real Time PCR, Present invention** | | | | | | | |
|---|---|---|---|---|---|---|---|---|---|---|---|
| | | | | ***C. trachomatis*** | | ***M. genitalium*** | | ***N. gonorrhoeae*** | | **IC** | |
| **Sample** | ***C.*** | ***M.*** | ***N.*** | **Mean** | **SD** | **Mean** | **SD** | **Mean** | **SD** | **Mean** | **SD** |
| | ***trachomatis*** | ***genitalium*** | ***gonorrhoeae*** | | | | | | | | |
| 1 | + | - | - | 33.0 | 00.0 | ND | ND | ND | ND | 34.15 | 0.35 |
| 2 | + | - | - | 35.95 | 0.07 | ND | ND | ND | ND | 34.70 | 0.14 |
| 3 | + | - | - | 27.95 | 0.21 | ND | ND | ND | ND | 34.70 | 0 |
| 4 | + | - | - | 23.95 | 0.21 | ND | ND | ND | ND | 34.10 | 0 |
| 5 | + | - | - | 31.70 | 0.14 | ND | ND | ND | ND | 34.25 | 0.21 |
| 6 | + | - | - | 33.75 | 0.35 | ND | ND | ND | ND | 34.75 | 0.07 |
| 7 | + | - | - | 29.05 | 0.07. | ND | ND | ND | ND | 34.65 | 0.07 |
| 8 | + | - | - | 29.95 | 0.07 | ND | ND | ND | ND | 34.05 | 0.19 |
| 9 | + | - | - | 24.50 | 0.28 | ND | ND | ND | ND | 34.95 | 0.07 |
| 10 | + | - | - | 31.25 | 0.49 | ND | ND | ND | ND | 34.80 | 0.57 |
| 11 | - | - | + | ND | ND | ND | ND | 37.05 | 1.34 | 35.15 | 0.92 |
| 12 | + | - | - | 31 | 0 | ND | ND | ND | ND | 34.10 | 0.28 |
| 13 | - | - | + | ND | ND | ND | ND | 45.15 | 3.04 | 34.60 | 0.14 |
| 14 | - | - | + | ND | ND | ND | ND | 29.50 | 0.28 | 35.45 | 0.92 |
| 15 | + | - | - | 31.80 | 0.42 | ND | ND | ND | ND | 34.55 | 0.35 |
| 16 | + | - | - | 32.75 | 0.07 | ND | ND | ND | ND | 35.30 | 0.57 |
| 17 | - | - | + | ND | ND | ND | ND | 33.45 | 0.21 | 34.75 | 0.07 |
| 18 | - | - | + | ND | ND | ND | ND | 39.30 | 0.85 | 34.80 | 0.42 |
| 19 | + | - | - | 31.20 | 0.42 | ND | ND | ND | ND | 34.45 | 0.92 |
| Negative | NT | NT | NT | ND | ND | ND | ND | ND | ND | 34.25 | 0.07 |
| control | | | | | | | | | | | |
| Positive CT | NT | NT | NT | 28.85 | 0.21 | ND | ND | ND | ND | 35.40 | 0.14 |
| control | | | | | | | | | | | |
| Positive M. | NT | NT | NT | ND | ND | 31.80 | 0.14 | ND | ND | 34.65 | 0.21 |
| genitalium | | | | | | | | | | | |
| control | | | | | | | | | | | |
| Positive *N.* | NT | NT | NT | ND | ND | ND | ND | 25.30 | 0.14 | 35.60 | 0.57 |
| *gonorrhoeae* | | | | | | | | | | | |
| control | | | | | | | | | | | |

The IC is perfectly detected in the real-time multiplex system of the invention, which confirms that there is no Taq polymerase inhibitor.

The real-time multiplex amplification of the invention has a good reproducibility, the standard deviations being very low, except for two points on *N. gonorrhoeae.*

The detection results obtained with the real-time multiplex system of the invention are at least as accurate as the ones obtained with the prior art ones.

## Claims

1. **A process** for the detection of *Chlamydia trachomatis* (CT) and/or *Mycoplasma genitalium* (MG) and/or *Neisseria gonorrhoeae* (NG), in a sample, which is suitable for said detection in real-time multiplex amplification,
wherein said detection comprises the determination of whether at least one amplicon has been, or is, produced from said sample, or from nucleic acid material thereof, by amplification by means of amplification primers,
whereby a positive determination indicates that at least one CT and/or at least one MG and/or at least one NG is(are) present in said sample,
wherein said amplification primers comprise:
- at least two primers, which are intended for targeting CT, and which are suitable for the detection of CT in real-time multiplex amplification,
wherein said at least two CT-targeted primers are oligonucleotides, which consist of 14-30 nucleotides, the sequences of which are suitable for use as forward and reverse primers, respectively, in the amplification of at least one CT reference template sequence, wherein
said at least one CT reference template sequence is a fragment consisting of positions 5571-5760 (SEQ ID NO: 5) of the CT sequence of SEQ ID NO: 1, or of a conservative sub-fragment thereof, which has retained the property of being a suitable reference template sequence, to construct and produce CT-targeted primers, which allow for a real-time multiplex detection of CT,
and
- at least two primers, which are intended for targeting MG, and which are suitable for the detection of MG in real-time multiplex amplification,
wherein said at least two MG-targeted primers are oligonucleotides, which consist of 14-30 nucleotides, the sequences of which are suitable for use as forward and reverse primers, respectively, in the amplification of at least one MG reference template sequence, wherein
said at least one MG reference template sequence is a fragment consisting of:
- positions 1-270 (SEQ ID NO: 13) of the MG sequence of SEQ ID NO: 2, or of a conservative sub-fragment thereof, which has retained the property of being a suitable reference template sequence, to construct and produce MG-targeted primers, which allow for a real-time multiplex detection of MG,
- positions 1140-1290 (SEQ ID NO: 19) of the MG sequence of SEQ ID NO: 3, or of a conservative sub-fragment thereof, which has retained the property of being a suitable reference template sequence, to construct and produce MG-targeted primers, which allow for a real-time multiplex detection of MG,
- positions 1060-1250 (SEQ ID NO: 25) of the MG sequence of SEQ ID NO: 3, or of a conservative sub-fragment thereof, which has retained the property of being a suitable reference template sequence, to construct and produce MG-targeted primers, which allow for a real-time multiplex detection of MG,
- positions 1520-1710 (SEQ ID NO: 31) of the MG sequence of SEQ ID NO: 3, or of a conservative sub-fragment thereof, which has retained the property of being a suitable reference template sequence, to construct and produce MG-targeted primers, which allow for a real-time multiplex detection of MG,
- positions 1500-1710 (SEQ ID NO: 37) of the MG sequence of SEQ ID NO: 3, or of a conservative sub-fragment thereof, which has retained the property of being a suitable reference template sequence, to construct and produce MG-targeted primers, which allow for a real-time multiplex detection of MG,
and
- at least two primers, which are intended for targeting NG, and which are suitable for the detection of NG in real-time multiplex amplification,
wherein said at least two NG-targeted primers are oligonucleotides, which consist of 14-30 nucleotides, the sequences of which are suitable for use as forward and reverse primers, respectively, in the amplification of at least one NG reference template sequence, wherein said at least one NG reference template sequence is a fragment consisting of positions 101-380 (SEQ ID NO: 42) of the NG sequence of SEQ ID NO: 4, or of a conservative sub-fragment thereof, which has retained the property of being a suitable reference template sequence, to construct and produce NG-targeted primers, which allow for a real-time multiplex detection of NG,
wherein said determination of whether at least one amplicon has been or is produced, is carried out by means of at least one probe, which is intended to anneal to said at least one amplicon, and wherein said at least one probe comprises:
- at least one CT-specific probe, which is suitable for the detection of CT in real-time multiplex amplification, wherein said at least one CT-specific probe is:
i. a fragment of at least 15 nucleotides of said CT reference template sequence, or a fragment of at least 15 nucleotides of the sequence that is fully complementary to said CT reference template sequence over the entire length of said reference template sequence, or
ii. a conservative variant of such a fragment, the sequence of which has at least 90% identity with a fragment of i. over the entire length of said fragment of i.,
and
- at least one MG-specific probe, which is suitable for the detection of MG in real-time multiplex amplification, wherein said at least one MG-specific probe is:
iii.a fragment of at least 15 nucleotides of said MG reference template sequence, or a fragment of at least 15 nucleotides of the sequence that is fully complementary to said MG reference template sequence over the entire length of said reference template sequence, or
iv. a conservative variant of such a fragment, the sequence of which has at least 90% identity with a fragment of iii. over the entire length of said fragment of iii.,
and
- at least one NG-specific probe, which is suitable for the detection of NG in real-time multiplex amplification, wherein said at least one NG-specific probe is:
v. a fragment of at least 15 nucleotides of said NG reference template sequence, or a fragment of at least 15 nucleotides of the sequence that is fully complementary to said NG reference template sequence over the entire length of said reference template sequence, or
vi. a conservative variant of such a fragment, the sequence of which has at least 90% identity with a fragment of v. over the entire length of said fragment of v.,
said CT, MG and NG reference template sequences sharing the special technical feature of being reference template sequences, which are suitable for the construction and production of CT-, MG- and NG-targeted primers, as well as of CT-, MG- and NG-specific probes, which can be used together in multiplex in the same tube to specifically detect CT and/or MG and/or NG, advantageously CT and MG and NG, in real-time in said same tube.

2. The detection process of claim 1, wherein said at least one CT reference template sequence is the fragment consisting of positions 5580-5754 (SEQ ID NO: 6) of the CT sequence of SEQ ID NO: 1.

3. The detection process of claim 1 or 2, wherein said at least one MG reference template sequence is:
- the fragment consisting of positions 2-259 (SEQ ID NO: 14) of the MG sequence of SEQ ID NO:2, or
- the fragment consisting of positions 1144-1283 (SEQ ID NO:20) of the MG sequence of SEQ ID NO:3, or
- the fragment consisting of positions 1064-1249 (SEQ ID NO:26) of the MG sequence of SEQ ID NO:3, or
- the fragment consisting of positions 1527-1704 (SEQ ID NO:32) of the MG sequence of SEQ ID NO:3, or
- the fragment consisting of positions 1501-1704 (SEQ ID NO:38) of the MG sequence of SEQ ID NO:3,
said MG reference template sequences sharing the specific technical feature of being suitable references to construct and produce MG-targeted primers, as well as MG-specific probes, which can be used together in multiplex in the same tube to specifically detect CT and/or MG and/or NG, advantageously CT and MG and NG, in real-time time in said same tube.

4. The detection process of any one of the preceding claims, wherein said at least one NG reference template sequence is the fragment consisting of positions 114-365 (SEQ ID NO: 43) of the NG sequence of SEQ ID NO: 4.

5. The detection process of any one of the preceding claims, wherein said at least two CT-targeted primers are one oligonucleotide of SEQ ID NO: 7, and one oligonucleotide of SEQ ID NO: 12.

6. The detection process of any one of the preceding claims, wherein said at least two MG-targeted primers are one oligonucleotide of SEQ ID NO: 15 and one oligonucleotide of SEQ ID NO: 18, or are at least one oligonucleotide selected from the group consisting of SEQ ID NO: 21; 27; 33; 39, and at least one oligonucleotide selected from the group consisting of SEQ ID NO: 24; 30; 36.

7. The detection process of claim 6, wherein said at least two MG-targeted primers are one oligonucleotide of SEQ ID NO: 15 and one oligonucleotide of SEQ ID NO: 18.

8. The detection process of claim 6, wherein said at least two MG-targeted primers are at least one oligonucleotide selected from the group consisting of SEQ ID NO: 21; 27 and at least one oligonucleotide selected from the group consisting of SEQ ID NO: 24; 30; 36.

9. The detection process of claim 8, wherein said at least two MG-targeted primers are one oligonucleotide of SEQ ID NO: 21 and at least one oligonucleotide selected from the group consisting of SEQ ID NO: 24; 36.

10. The detection process of claim 9, wherein said at least two MG-targeted primers are one oligonucleotide of SEQ ID NO: 21 and one oligonucleotide of SEQ ID NO: 24.

11. The detection process of claim 8, wherein said at least two MG-targeted primers are one oligonucleotide of SEQ ID NO: 27 and at least one oligonucleotide selected from the group consisting of SEQ ID NO: 24; 30; 36.

12. The detection process of claim 11, wherein said at least two MG-targeted primers are one oligonucleotide of SEQ ID NO: 27 and one oligonucleotide of SEQ ID NO: 30.

13. The detection process of claim 6, wherein said at least two MG-targeted primers are one oligonucleotide of SEQ ID NO: 36, and at least one oligonucleotide selected from the group consisting of SEQ ID NO: 21; 27; 33 and 39.

14. The detection process of claim 13, wherein said at least two MG-targeted primers are one oligonucleotide of SEQ ID NO: 33 and one oligonucleotide of SEQ ID NO: 36.

15. The detection process of claim 13, wherein said at least two MG-targeted primers are one oligonucleotide of SEQ ID NO: 39 and one oligonucleotide of SEQ ID NO: 36.

16. The detection process of any one of the preceding claims, wherein said at least two NG-targeted primers are one oligonucleotide of SEQ ID NO: 44 and one oligonucleotide of SEQ ID NO: 47.

17. The detection process of any one of claims 1-16, wherein said at least one CT-specific probe is of SEQ ID NO: 8 or 10, or is the sequence that is fully complementary to this SEQ ID sequence, over the entire length of this SEQ ID sequence, said probe being optionally linked to at least one detection label and/or at least one nucleotide arm that is unrelated to CT, MG and NG and that is intended to carry a quencher or a reporter.

18. The detection process of claim 17, wherein said at least one probe is linked to at least one beacon arm.

19. The detection process of claim 18, wherein said at least one CT-specific probe is of SEQ ID NO: 9 or 11, or the sequence that is fully complementary to this SEQ ID sequence, over the entire length of this SEQ ID sequence.

20. The detection process of any one of claims 17-19, wherein said at least two CT-targeted primers are one oligonucleotide of SEQ ID NO: 7 and one oligonucleotide of SEQ ID NO: 12, and said at least one CT-specific probe is selected from the group consisting of SEQ ID NO: 8; 9; 10; 11.

21. The detection process of any one of claims 1-20, wherein said at least one MG-specific probe is selected from the group consisting of SEQ ID NO: 16; 22; 28; 34 and 40, or is the sequence that is fully complementary to this SEQ ID sequence, over the entire length of this SEQ ID sequence, said probe being optionally linked to at least one detection label and/or at least one nucleotide arm that is unrelated to CT, MG and NG and that is intended to carry a quencher or a reporter.

22. The detection process of claim 21, wherein said at least one probe has at least one beacon arm.

23. The detection process of claim 22, wherein said at least one MG-specific probe is selected from the group consisting of SEQ ID NO: 17; 23; 29; 35 and 41, or is the sequence that is fully complementary to this SEQ ID sequence, over the entire length of this SEQ ID sequence.

24. The detection process of any one of claims 21-23, wherein said at least two MG-targeted primers are one oligonucleotide of SEQ ID NO: 15 and one oligonucleotide of SEQ ID NO: 18, and wherein said at least one MG-specific probe is of SEQ ID NO: 16 or 17, or is the sequence that is fully complementary to this SEQ ID sequence, over the entire length of this SEQ ID sequence.

25. The detection process of any one of claims 21-23, wherein said at least two MG-targeted primers are one oligonucleotide of SEQ ID NO: 21 and one oligonucleotide of SEQ ID NO: 24, and wherein said at least one MG-specific probe is of SEQ ID NO: 22 or 23, or is the sequence that is fully complementary to this SEQ ID sequence, over the entire length of this SEQ ID sequence.

26. The detection process of any one of claims 21-23, wherein said at least two MG-targeted primers are one oligonucleotide of SEQ ID NO: 27 and one oligonucleotide of SEQ ID NO: 30, and wherein said at least one MG-specific probe is of SEQ ID NO: 28 or 29, or is the sequence that is fully complementary to this SEQ ID sequence, over the entire length of this SEQ ID sequence.

27. The detection process of any one of claims 21-23, wherein said at least two MG-targeted primers are one oligonucleotide of SEQ ID NO: 33 and one oligonucleotide of SEQ ID NO: 36, and wherein said at least one MG-specific probe is of SEQ ID NO: 34 or 35, or is the sequence that is fully complementary to this SEQ ID sequence, over the entire length of this SEQ ID sequence.

28. The detection process of any one of claims 21-23, wherein said at least two MG-targeted primers are one oligonucleotide of SEQ ID NO: 39 and one oligonucleotide of SEQ ID NO: 36, and wherein said at least one MG-specific probe is of SEQ ID NO: 40 or 41, or is the sequence that is fully complementary to this SEQ ID sequence, over the entire length of this SEQ ID sequence.

29. The detection process of any one of claims 1-28, wherein said at least one NG-specific probe is of SEQ ID NO: 45, or is the sequence that is fully complementary to this SEQ ID sequence, over the entire length of this SEQ ID sequence, said probe being optionally linked to at least one detection label and/or at least one nucleotide arm that is unrelated to CT, MG and NG and that is intended to carry a quencher or a reporter.

30. The detection process of claim 29, wherein said at least one probe has at least one beacon arm.

31. The detection process of claim 30, wherein said at least one NG-specific probe is of SEQ ID NO: 46, or is the sequence that is fully complementary to this SEQ ID sequence, over the entire length of this SEQ ID sequence.

32. The detection process of any one of claims 29-31, wherein said at least two NG-targeted primers are one oligonucleotide of SEQ ID NO: 44 and one oligonucleotide of SEQ ID NO: 47, and wherein said at least one NG-specific probe is of SEQ ID NO: 45 or 46, or is the sequence that is fully complementary to this SEQ ID sequence, over the entire length of this SEQ ID sequence.

33. The detection process of any one of claims 1-32, wherein:
- said at least two CT-targeted primers are one oligonucleotide of SEQ ID NO: 7 and one oligonucleotide of SEQ ID NO: 12, and
- said at least two MG-targeted primers are one oligonucleotide of SEQ ID NO: 15 and one oligonucleotide of SEQ ID NO: 18; or one oligonucleotide of SEQ ID NO: 21 and one oligonucleotide of SEQ ID NO: 24; or one oligonucleotide of SEQ ID NO: 27 and one oligonucleotide of SEQ ID NO: 30; or one oligonucleotide of SEQ ID NO: 33 and one oligonucleotide of SEQ ID NO: 36; or one oligonucleotide of SEQ ID NO: 39 and one oligonucleotide of SEQ ID NO: 36, and
- said at least two NG-targeted primers are one oligonucleotide of SEQ ID NO: 44 and one oligonucleotide of SEQ ID NO: 47.

34. The detection process of any one of claims 1-33, wherein:
- said at least two CT-targeted primers are one oligonucleotide of SEQ ID NO: 7 and one oligonucleotide of SEQ ID NO: 12, and said at least one CT-specific probe is of SEQ ID NO: 8, 9, 10 or 11, or is the sequence that is fully complementary to this SEQ ID sequence, over the entire length of this SEQ ID sequence, and
- said at least two NG-targeted primers are one oligonucleotide of SEQ ID NO: 44 and one oligonucleotide of SEQ ID NO: 47, and said at least one NG-specific probe is of SEQ ID NO: 45 or 46, or is the sequence that is fully complementary to this SEQ ID sequence, over the entire length of this SEQ ID sequence; and
- said at least two MG-targeted primers and said at least one MG-specific probe are as follows:
- said at least two MG-targeted primers are one oligonucleotide of SEQ ID NO: 15 and one oligonucleotide of SEQ ID NO: 18, and said at least one MG-specific probe is of SEQ ID NO: 16 or 17, or is the sequence that is fully complementary to this SEQ ID sequence, over the entire length of this SEQ ID sequence; or
- said at least two MG-targeted primers are one oligonucleotide of SEQ ID NO: 21 and one oligonucleotide of SEQ ID NO: 24, and said at least one MG-specific probe is of SEQ ID NO: 22 or 23, or is the sequence that is fully complementary to this SEQ ID sequence, over the entire length of this SEQ ID sequence; or
- said at least two MG-targeted primers are one oligonucleotide of SEQ ID NO: 27 and one oligonucleotide of SEQ ID NO: 30, and said at least one MG-specific probe is of SEQ ID NO: 28 or 29, or is the sequence that is fully complementary to this SEQ ID sequence, over the entire length of this SEQ ID sequence; or
- said at least two MG-targeted primers are one oligonucleotide of SEQ ID NO: 33 and one oligonucleotide of SEQ ID NO: 36, and said at least one MG-specific probe is of SEQ ID NO: 34 or 35, or is the sequence that is fully complementary to this SEQ ID sequence, over the entire length of this SEQ ID sequence; or
- said at least two MG-targeted primers are one oligonucleotide of SEQ ID NO: 39 and one oligonucleotide of SEQ ID NO: 36, and said at least one MG-specific probe is of SEQ ID NO: 40 or 41, or is the sequence that is fully complementary to this SEQ ID sequence, over the entire length of this SEQ ID sequence.

35. The detection process of any of the preceding claims, wherein said at least two CT-targeted primers and/or said at least two MG-targeted primers and/or said at least two NG-targeted primers consist of 17-25 nucleotides.

36. The detection process of any of the preceding claims, wherein said at least one CT-specific probe and/or said at least one MG-specific probe and/or said at least one NG-specific probe consist(s) of 22-27 nucleotides.

37. The detection process of any of the preceding claims, which further comprises the amplification of an Internal Control (IC), the sequence of which is unrelated to CT, MG, NG, such as the IC of SEQ ID NO: 48.

38. The detection process of any one the preceding claims, which is a real-time multiplex amplification.

39. The detection process of claim 38, wherein said amplification is a PCR.

40. **Method of production of primers and probes** that can be used in the same tube for the detection of CT and MG and NG in real-time multiplex amplification, which comprises producing at least one primer pair and at least one probe,
wherein one oligonucleotide of said primer pair is of 14-30 nucleotides, the sequence of which is at least 80% identical to the sequence of the same length that is the exact 5' terminal end of a reference template sequence, and the other oligonucleotide of said primer pair is of 14-30 nucleotides, the sequence of which is at least 80% identical to the sequence of the same length that is the exact 5' terminal end of the sequence that is fully complementary to said reference template sequence over the entire length of said reference template sequence,
wherein said probe is:
i. a fragment of at least 15 nucleotides of said reference template sequence, or a fragment of at least 15 nucleotides of the sequence that is fully complementary to said reference template sequence over the entire length of said reference template sequence or
ii. a conservative variant of a fragment as defined in i., the sequence of said conservative variant being at least 90% identical to the sequence of said fragment of i. over the entire length of said fragment of i.,
said probe being optionally linked to at least one detection label and/or at least one beacon wherein said reference template sequence is:
- for the design of CT primers and probes: a fragment consisting of positions 5571-5760 (SEQ ID NO: 5) of the CT sequence of SEQ ID NO: 1, or a conservative sub-fragment thereof, which has retained the property of being a suitable reference template sequence, to construct and produce CT-targeted primers, which allow for a real-time multiplex detection of CT, or a sequence that is fully complementary to said fragment or sub-fragment over the entire length of said fragment or sub-fragment, wherein said conservative sub-fragment consists of positions 5580-5754 (SEQ ID NO: 6) of the CT sequence of SEQ ID NO: 1,
- for the design of MG primers and probes: a fragment consisting of:
- positions 1-270 (SEQ ID NO: 13) of the MG sequence of SEQ ID NO: 2, or a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment, wherein said conservative sub-fragment consists of positions 2-259 (SEQ ID NO: 14) of the MG sequence of SEQ ID NO: 2,
- positions 1140-1290 (SEQ ID NO: 19) of the MG sequence of SEQ ID NO: 3, or a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment, wherein said conservative sub-fragment consists of positions 1144-1283 (SEQ ID NO: 20) of the MG sequence of SEQ ID NO: 3,
- positions 1060-1250 (SEQ ID NO: 25) of the MG sequence of SEQ NO: 3, or a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment, wherein said conservative sub-fragment consists of positions 1064-1249 (SEQ ID NO: 26) of the MG sequence of SEQ ID NO: 3,
- positions 1520-1710 (SEQ ID NO: 31) of the MG sequence of SEQ ID NO: 3, or a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment, wherein said conservative sub-fragment consists of positions 1527-1704 (SEQ ID NO: 32) of the MG sequence of SEQ ID NO: 3,
- positions 1500-1710 (SEQ ID NO: 37) of the MG sequence of SEQ ID NO: 3, or a conservative sub-fragment thereof, which has retained the property of being a suitable reference template sequence, to construct and produce MG-targeted primers, which allow for a real-time multiplex detection of MG, or a sequence that is fully complementary to said fragment or sub-fragment over the entire length of said fragment or sub-fragment, wherein said conservative sub-fragment consists of positions 1501-1704 (SEQ ID NO: 38) of the MG sequence of SEQ ID NO: 3,
- for the design of NG primers and probes: a fragment consisting of positions 101-380 (SEQ ID NO: 42) of the NG sequence of SEQ ID NO: 4, or a conservative sub-fragment thereof, which has retained the property of being a suitable reference template sequence, to construct and produce NG-targeted primers, which allow for a real-time multiplex detection of NG, or a sequence that is fully complementary to said fragment or sub-fragment over the entire length of said fragment or sub-fragment, wherein said conservative sub-fragment consists of positions 114-365 (SEQ ID NO: 43) of the NG sequence of SEQ ID NO: 4.

41. **A primer, which is especially adapted to** the detection of CT and/or MG and/or NG in real-time multiplex amplification, which is:
an oligonucleotide of 14-30 nucleotides, the sequence of which is at least 80% identical to the sequence of the same length that is the exact 5' terminal end of a reference template sequence or of the sequence that is fully complementary to said reference template sequence over the entire length of said reference template sequence,
wherein said reference template sequence is: - SEQ ID NO: 6; or
- SEQ ID NO: 14 or SEQ NO: 20 or SEQ ID NO: 26 or SEQ ID NO: 32 or SEQ ID NO: 38; or
- SEQ ID NO: 43.

42. The primer of claim 41, which is:
- a CT-targeted primer, selected from the group consisting of SEQ ID NO:7; 12, or
- a MG-targeted primer, selected from the group consisting of SEQ ID NO: 15; 18; 21; 24; 27;30;33;36;39,or
- a NG-targeted primer, selected from the group consisting of SEQ ID NO: 44; 47.

43. **A primer system, which is especially adapted to** the detection of CT and/or MG and/or NG in real-time multiplex amplification, which comprises:
at least one pair of oligonucleotides of 14-30 nucleotides each,
the sequence of one oligonucleotide of said pair being at least 80% identical to the sequence of the same length that is the exact 5' terminal end of a reference template sequence, and
the sequence of the other oligonucleotide of said pair being at least 80% identical to the sequence of the same length that is the exact 5' terminal end of the sequence that is fully complementary to said reference template sequence over the entire length of said reference template sequence,
wherein said reference template sequence is:
- SEQ NO: 6; or
- SEQ NO: 14 or SEQ ID NO: 20 or SEQ ID NO: 26 or SEQ ID NO: 32 or SEQ NO: 38; or
- SEQ NO: 43.

44. The primer system of claim 43, which comprises:
- at least one CT-targeted primer pair of SEQ ID NO: 7 and SEQ ID NO: 12, and/or
- at least one MG-targeted primer pair selected from the following pairs: SEQ ID NO: 15 and 18; SEQ ID NO:21 and 24; SEQ ID NO: 27 and 30; SEQ ID NO:33 and 36; SEQ ID NO:39 and 36, and/or
- at least one NG-targeted primer pair of SEQ ID NO: 44 and 47.

45. The primer system of claim 43 or 44, which comprises three of said pairs of oligonucleotides, and wherein said reference template sequence is:
- for one of said three pairs: SEQ NO: 6;
- for another one of said three pairs: SEQ ID NO: 14 or SEQ ID NO: 20 or SEQ ID NO: 26 or SEQ ID NO: 32 or SEQ ID NO: 38;
- for still another one of said three pairs: SEQ ID NO: 43.

46. **A probe, which is especially adapted to** the detection of CT and/or MG and/or NG in real-time multiplex amplification, which is:
- a CT-specific probe of SEQ ID NO: 8; or 10, or which consists of a sequence that is fully complementary to this SEQ ID sequence, over the entire length of this SEQ ID sequence,
- a MG-specific probe of SEQ ID NO: 16; 22; 28; 34 or 40, or which consists of a sequence that is fully complementary to this SEQ ID sequence, over the entire length of this SEQ ID sequence,
- a NG-specific probe of SEQ ID NO: 45, or which consists of a sequence that is fully complementary to this SEQ ID sequence, over the entire length of this SEQ ID sequence, said probe being optionally linked to at least one detection label and/or at least one beacon arm.

47. The probe of claim 46, which is:
- a CT-specific probe of SEQ ID NO: 9 or 11, or which consists of a sequence that is fully complementary to this SEQ ID sequence, over the entire length of this SEQ ID sequence,
- a MG-specific probe of SEQ ID NO: 17; 23; 29; 35 or 41, or which consists of a sequence that is fully complementary to this SEQ ID sequence, over the entire length of this SEQ ID sequence,
- a NG-specific probe of SEQ ID NO: 45 or 46, or which consists of a sequence that is fully complementary to this SEQ ID sequence, over the entire length of this SEQ ID sequence.

48. A primer and probe system, which is especially adapted to the detection of CT and/or MG and/or NG in real-time multiplex amplification, which comprises at least one primer system of any one of claims 43-45, and at least one probe of any one of claims 46-47.

49. An amplification composition, comprising at least one amplicon obtainable by implementation of the process of any one of claims 1-39 on a CT- and/or MG - and/or NG-containing sample, or by amplification of at least one nucleic acid from a CT- and/or MG- and/or NG- containing sample by means of at least one primer system of any one of claims 43-45, and further comprising:
- the CT-targeted primers as defined in claim 1 and the MG-targeted primers as defined in claim 1 and the NG-targeted primers as defined in claim 1; or
- the primer system of claim 45.

50. The amplification composition of claim 49, which comprises three amplicons, wherein said three amplicons are obtainable:
- by implementation of the process of any one of claims 1-39 on a CT- and MG- and NG-containing sample, or
- by amplification of at least one nucleic acid from CT, and of at least one nucleic acid from MG, and at least one nucleic acid from NG, by means of the primer system of claim 45.

51. A kit for the diagnosis of an infection by CT and/or MG and/or NG, which comprises:
- at least one primer system of any one of claims 43-45 and/or
- at least one probe of any one of claims 46-47,
- optionally, instructions for the use thereof and/or nucleotides.

52. The kit of claim 50, which further comprises the Internal Control (IC) oligonucleotide of SEQ ID NO: 48, and/or the IC primer pair of SEQ ID NO: 49 and NO: 52, and/or the IC probe of SEQ ID NO: 50, and/or the IC probe of SEQ ID NO: 51.

## Patentansprüche

1. Ein Verfahren zum Nachweis von *Chlamydia trachomatis* (CT) und/oder *Mycoplasma genitalium* (MG) und/oder *Neisseria gonorrhoeae* (NG), in einer Probe, welche für besagten Nachweis in Echtzeitmehrfachamplifikation geeignet ist,
wobei besagter Nachweis die Bestimmung umfasst, ob mindestens ein Amplifikat von besagter Probe, oder von Nukleinsäurematerial davon, durch Amplifikation mittels Amplifikationsprimern produziert wurde oder produziert wird,
wobei eine positive Bestimmung darauf hindeutet, dass mindestens ein CT und/oder mindestens ein MG und/oder mindestens ein NG in besagter Probe anwesend ist (sind),
wobei besagte Amplifikationsprimer umfassen:
- mindestens zwei Primer, welche für die Erkennung von CT bestimmt sind, und welche für den Nachweis von CT in Echtzeitmehrfachamplifikation geeignet sind,
wobei besagte mindestens zwei auf CT gerichtete Primer Oligonukleotide sind, welche aus 14-30 Nukleotiden bestehen, die Sequenzen davon sind für die Verwendung als Vorwärts- bzw. Rückwärtsprimer für die Amplifikation von mindestens einer CT-Referenztemplatesequenz geeignet, wobei besagte mindestens eine CT-Referenztemplatesequenz ein Fragment bestehend aus den Positionen 5571-5760 (SEQ ID NO: 5) von der CT-Sequenz in SEQ ID NO: 1, oder von einem konservierten Subfragment davon, welches die Eigenschaft behalten hat, eine geeignete Referenztemplatesequenz zu sein, um auf CT gerichtete Primer zu konstruieren und herzustellen, welche einen Echtzeitmehrfachnachweis von CT erlauben, und
- mindestens zwei Primer, welche für den Nachweis von MG gedacht sind, und welche für den Nachweis von MG in Echtzeitmehrfachamplifikation geeignet sind,
wobei besagte mindestens zwei auf MG gerichtete Primer Oligonukleotide sind, welche aus 14-30 Nukleotiden bestehen, die Sequenzen davon sind für die Verwendung als Vorwärts- bzw. Rückwärtsprimer für die Amplifikation von mindestens einer MG-Referenztemplatesequenz geeignet, wobei besagte mindestens eine MG-Referenztemplatesequenz ein Fragment ist bestehend aus:
- Positionen 1-270 (SEQ ID NO: 13) der MG-Sequenz in SEQ ID NO: 2, oder von einem konservierten Subfragment davon, welches die Eigenschaft behalten hat, eine geeignete Referenztemplatesequenz zu sein, um auf MG gerichtete Primer zu konstruieren und herzustellen, welche den Echtzeitmehrfachnachweis von MG erlauben,
- Positionen 1140-1290 (SEQ ID NO: 19) der MG-Sequenz in SEQ ID NO: 3, oder von einem konservierten Subfragment davon, welches die Eigenschaft behalten hat, eine geeignete Referenztemplatesequenz zu sein, um auf MG gerichtete Primer zu konstruieren und herzustellen, welche den Echtzeitmehrfachnachweis von MG erlauben,
- Positionen 1060-1250 (SEQ ID NO: 25) der MG-Sequenz in SEQ ID NO: 3, oder von einem konservierten Subfragment davon, welches die Eigenschaft behalten hat, eine geeignete Referenztemplatesequenz zu sein, um auf MG gerichtete Primer zu konstruieren und herzustellen, welche den Echtzeitmehrfachnachweis von MG erlauben,
- Positionen 1520-1710 (SEQ ID NO: 31) der MG-Sequenz in SEQ ID NO: 3, oder von einem konservierten Subfragment davon, welches die Eigenschaft behalten hat, eine geeignete Referenztemplatesequenz zu sein, um auf MG gerichtete Primer zu konstruieren und herzustellen, welche den Echtzeitmehrfachnachweis von MG erlauben,
- Positionen 1500-1710 (SEQ ID NO: 37) der MG-Sequenz in SEQ ID NO: 3, oder von einem konservierten Subfragment davon, welches die Eigenschaft behalten hat, eine geeignete Referenztemplatesequenz zu sein, um auf MG gerichtete Primer zu konstruieren und herzustellen, welche den Echtzeitmehrfachnachweis von MG erlauben,
und
- mindestens zwei Primer, welche für den Nachweis von MG gedacht sind, und welche für den Nachweis von NG in Echtzeitmehrfachamplifikation geeignet sind,
wobei besagte mindestens zwei auf NG gerichtete Primer Oligonukleotide sind, welche aus 14-30 Nukleotiden bestehen, die Sequenzen davon sind für die Verwendung als Vorwärts- bzw. Rückwärtsprimer für die Amplifikation von mindestens einer NG-Referenztemplatesequenz geeignet, wobei besagte mindestens eine NG-Referenztemplatesequenz ein Fragment bestehend aus den Positionen 101-380 (SEQ ID NO: 42) der NG-Sequenz in SEQ ID NO: 4, oder eines konservierten Subfragments davon ist, welches die Eigenschaft behalten hat, eine geeignete Referenztemplatesequenz zu sein, um auf NG gerichtete Primer zu konstruieren und herzustellen, welche den Echtzeitmehrfachnachweis von NG erlauben,
wobei besagte Bestimmung, ob mindestens ein Amplikon produziert wurde oder wird, mittels mindestens einer Sonde durchgeführt wird, welche dafür gedacht ist, an besagtes mindestens eine Amplikon zu binden, und wobei besagte mindestens eine Sonde umfasst:
- mindestens eine CT-spezifsche Sonde, welche für den Nachweis von CT in Echtzeitmehrfachamplifikation geeignet ist, wobei besagte mindestens eine CT-spezifische Sonde ist:
i. ein Fragment von mindestens 15 Nukleotiden von besagter CT-Referenztemplatesequenz, oder ein Fragment von mindestens 15 Nukleotiden von einer Sequenz, die vollständig komplementär zu besagter CT-Referenztemplatesequenz über die gesamte Länge von besagter Referenztemplatesequenz ist, oder
ii. eine konservierte Variante von einem solchen Fragment, deren Sequenz mindestens 90% Identität mit einem Fragment in i. über die gesamte Länge von besagtem Fragment in i. hat,
und
- mindestens eine MG-spezifische Sonde, welche für den Nachweis von MG in Echtzeitmehrfachamplifikation geeignet ist, wobei besagte mindestens eine MG-spezifische Sonde ist:
iii. ein Fragment von mindestens 15 Nukleotiden von besagter MG-Referenztemplatesequenz, oder ein Fragment von mindestens 15 Nukleotiden einer Sequenz, die vollständig komplementär zu besagter MG-Referenztemplatesequenz über die gesamte Länge von besagter Referenztemplatesequenz ist, oder
iv. eine konservierte Variante von einem solchen Fragment, deren Sequenz mindestens 90% Identität mit einem Fragment in iii. über die gesamte Länge von besagtem Fragment in iii. hat,
und
- mindestens eine NG-spezifische Sonde, welche für den Nachweis von NG in Echtzeitmehrfachamplifikation geeignet ist, wobei besagte mindestens eine NG-spezifische Sonde ist:
v. ein Fragment von mindestens 15 Nukleotiden von besagter NG-Referenztemplatesequenz, oder ein Fragment von mindestens 15 Nukleotiden einer Sequenz, die vollständig komplementär zu besagter NG-Referenztemplatesequenz über die gesamte Länge von besagter Referenztemplatesequenz ist, oder
vi. eine konservierte Variante von einem solchen Fragment, deren Sequenz mindestens 90% Identität mit einem Fragment in v. über die gesamte Länge von besagtem Fragment in v. hat,
besagte CT, MG und NG-Referenztemplatesequenzen teilen die speziellen technischen Eigenschaften von Referenztemplatesequenzen, welche für die Konstruktion und Produktion von auf CT, MG und NG gerichteten Primern geeignet sind, sowie von CT-, MG- und NGspezifischen Sonden, welche zusammen in Mehrfachanwendung in demselben Probenbehälter verwendet werden können, um spezifisch CT und/oder MG und/oder NG, vorteilhafterweise CT und MG und NG, in Echtzeit in besagtem selben Probengefäß nachzuweisen.

2. Das Verfahren zum Nachweis nach Anspruch 1, wobei besagte mindestens eine CT-Referenztemplatesequenz ein Fragment bestehend aus den Positionen 5580-5754 (SEQ ID NO: 6) der CT-Sequenz in SEQ ID NO: 1 ist.

3. Das Verfahren zum Nachweis nach Anspruch 1 oder 2, wobei besagte mindestens eine MG-Referenztemplatesequenz ist:
- das Fragment bestehend aus Positionen 2-259 (SEQ ID NO: 14) der MG-Sequenz in SEQ ID NO: 2, oder
- das Fragment bestehend aus Positionen 1144-1283 (SEQ ID NO: 20) der MG-Sequenz in SEQ ID NO: 3, oder
- das Fragment bestehend aus Positionen 1064-1249 (SEQ ID NO: 26) der MG-Sequenz in SEQ ID NO: 3, oder
- das Fragment bestehend aus Positionen 1527-1704 (SEQ ID NO: 32) der MG-Sequenz in SEQ ID NO: 3, oder
- das Fragment bestehend aus Positionen 1501-1704 (SEQ ID NO: 38) der MG-Sequenz in SEQ ID NO: 3,
besagte MG-Referenztemplatesequenzen teilen die spezifischen technischen Eigenschaften von geeigneten Referenzen, um auf MG gerichtete Primer zu konstruieren und herzustellen, sowie MG-spezifische Sonden, welche zusammen in Mehrfachanwendung in demselben Probengefäß verwendet werden können, um spezifisch CT und/oder MG und/oder NG, vorteilhafterweise CT und MG und NG, in Echtzeit in besagtem selben Probengefäß nachzuweisen.

4. Das Verfahren zum Nachweis nach einem der vorangehenden Ansprüche, wobei besagte mindestens eine NG-Referenztemplatesequenz ein Fragment ist bestehend aus den Positionen 114-365 (SEQ ID NO: 43) der NG-Sequenz in SEQ ID NO: 4.

5. Das Verfahren zum Nachweis nach einem der vorangehenden Ansprüche, wobei besagte mindestens zwei auf CT gerichtete Primer ein Oligonukleotid nach SEQ ID NO: 7 und ein Oligonukleotid nach SEQ ID NO: 12 sind.

6. Das Verfahren zum Nachweis nach einem der vorangehenden Ansprüche, wobei besagte mindestens zwei auf MG gerichtete Primer ein Oligonukleotid nach SEQ ID NO: 15 und ein Oligonukleotid nach SEQ ID NO: 18 sind, oder mindestens ein Oligonukleotid ausgewählt aus der Gruppe bestehend aus SEQ ID NO: 21; 27; 33; 39, und mindestens ein Oligonukleotid ausgewählt aus der Gruppe bestehend aus SEQ ID NO: 24; 30; 36 sind.

7. Das Verfahren zum Nachweis nach Anspruch 6, wobei besagte mindestens zwei auf MG gerichtete Primer ein Oligonukleotid nach SEQ ID NO: 15 und ein Oligonukleotid nach SEQ ID NO: 18 sind.

8. Das Verfahren zum Nachweis nach Anspruch 6, wobei besagte mindestens zwei auf MG gerichtete Primer mindestens ein Oligonukleotid ausgewählt aus der Gruppe bestehend aus SEQ ID NO: 21; 27 und mindestens ein Oligonukleotid ausgewählt aus der Gruppe bestehend aus SEQ ID NO: 24: 30; 36 sind.

9. Das Verfahren zum Nachweis nach Anspruch 8, wobei besagte mindestens zwei auf MG gerichtete Primer ein Oligonukleotid nach SEQ ID NO: 21 und mindestens ein Oligonukleotid ausgewählt aus der Gruppe bestehend aus SEQ ID NO: 24: 36 sind.

10. Das Verfahren zum Nachweis nach Anspruch 9, wobei besagte mindestens zwei auf MG gerichtete Primer ein Oligonukleotid nach SEQ ID NO: 21 und ein Oligonukleotid nach SEQ ID NO: 24 sind.

11. Das Verfahren zum Nachweis nach Anspruch 8, wobei besagte mindestens zwei auf MG gerichtete Primer ein Oligonukleotid nach SEQ ID NO: 27 und mindestens ein Oligonukleotid ausgewählt aus der Gruppe bestehend aus SEQ ID NO: 24; 30; 36 sind.

12. Das Verfahren zum Nachweis nach Anspruch 11, wobei besagte mindestens zwei auf MG gerichtete Primer ein Oligonukleotid nach SEQ ID NO: 27 und ein Oligonukleotid nach SEQ ID NO: 30 sind.

13. Das Verfahren zum Nachweis nach Anspruch 6, wobei besagte mindestens zwei auf MG gerichtete Primer ein Oligonukleotid nach SEQ ID NO: 36 und mindestens ein Oligonukleotid ausgewählt aus der Gruppe bestehend aus SEQ ID NO: 21; 27; 33 und 39 sind.

14. Das Verfahren zum Nachweis nach Anspruch 13, wobei besagte mindestens zwei auf MG gerichtete Primer ein Oligonukleotid nach SEQ ID NO: 33 und ein Oligonukleotid nach SEQ ID NO: 36 sind.

15. Das Verfahren zum Nachweis nach Anspruch 13, wobei besagte mindestens zwei auf MG gerichtete Primer ein Oligonukleotid nach SEQ ID NO: 39 und ein Oligonukleotid nach SEQ ID NO: 36 sind.

16. Das Verfahren zum Nachweis nach einem der vorangehenden Ansprüche, wobei besagte mindestens zwei auf NG gerichtete Primer ein Oligonukleotid nach SEQ ID NO: 44 und ein Oligonukleotid nach SEQ ID NO: 47 sind.

17. Das Verfahren zum Nachweis nach einem der Ansprüche 1-16, wobei besagte mindestens eine CT-spezifische Sonde aus SEQ ID NO: 8 oder 10 ist oder die Sequenz ist, die vollständig komplementär zu einer dieser SEQ ID-Sequenz ist, über die gesamte Länge von dieser SEQ ID-Sequenz, besagte Sonde ist optional verknüpft mit mindestens einem Detektionsmarker und/oder mindestens einem Nukleotidarm, der keinen Bezug zu CT, MG und NG hat und der dafür gedacht ist, einen Quencher oder einen Reporter zu tragen.

18. Das Verfahren zum Nachweis nach Anspruch 17, wobei besagte mindestens eine Sonde mit mindestens einem Beacon-Arm verknüpft ist.

19. Das Verfahren zum Nachweis nach Anspruch 18, wobei besagte mindestens eine CT-spezifische Sonde die Sequenz SEQ ID NO: 9 oder 11 ist, oder die Sequenz, die vollständig komplementär zu dieser SEQ ID-Sequenz ist, über die gesamte Länge von dieser SEQ ID-Sequenz.

20. Das Verfahren zum Nachweis nach einem der Ansprüche 17-19, wobei besagte mindestens zwei auf CT gerichtete Primer ein Oligonukleotid nach SEQ ID NO: 7 und ein
Oligonukleotid nach SEQ ID NO: 12 sind, und besagte mindestens eine CT-spezifische Sonde ausgewählt ist aus der Gruppe bestehend aus SEQ ID NO: 8; 9; 10; 11.

21. Das Verfahren zum Nachweis nach einem der Ansprüche 1-20, wobei besagte mindestens eine MG-spezifische Sonde ausgewählt ist aus der Gruppe bestehend aus SEQ ID NO: 16; 22; 28; 34 und 40, oder die Sequenz ist, die vollständig komplementär zu dieser SEQ ID-Sequenz ist, über die gesamte Länge von dieser SEQ ID-Sequenz, besagte Sonde ist optional verknüpft mit mindestens einem Detektionsmarker und/oder mindestens einem Nukleotidarm, der keinen Bezug zu CT, MG und NG hat und, der dazu gedacht ist, einen Quencher oder einen Reporter zu tragen.

22. Das Verfahren zum Nachweis nach Anspruch 21, wobei besagte mindestens eine Sonde mindestens einen Beacon-Arm hat.

23. Das Verfahren zum Nachweis nach Anspruch 22, wobei besagte mindestens eine MG-spezifische Sonde ausgewählt ist aus der Gruppe bestehend aus SEQ ID NO: 17; 23; 29; 35 und 41 oder die Sequenz ist, die vollständig komplementär zu dieser SEQ ID-Sequenz ist, über die gesamte Länge von dieser SEQ ID-Sequenz.

24. Das Verfahren zum Nachweis nach einem der Ansprüche 21-23, wobei besagte mindestens zwei auf MG gerichtete Primer ein Oligonukleotid nach SEQ ID NO: 15 und ein Oligonukleotid nach SEQ ID NO: 18 sind, und wobei besagte mindestens eine MG-spezifische Sonde aus SEQ ID NO: 16 oder 17 ist, oder die Sequenz ist, die vollständig komplementär zu dieser SEQ ID-Sequenz ist, über die gesamte Länge von dieser SEQ ID-Sequenz.

25. Das Verfahren zum Nachweis nach einem der Ansprüche 21-23, wobei besagte mindestens zwei auf MG gerichtete Primer ein Oligonukleotid nach SEQ ID NO: 21 und ein Oligonukleotid nach SEQ ID NO: 24 sind, und wobei besagte mindestens eine MG-spezifische Sonde aus SEQ ID NO: 22 oder 23 ist, oder die Sequenz ist, die vollständig komplementär zu dieser SEQ ID-Sequenz ist, über die gesamte Länge von dieser SEQ ID-Sequenz.

26. Das Verfahren zum Nachweis nach einem der Ansprüche 21-23, wobei besagte mindestens zwei auf MG gerichtete Primer ein Oligonukleotid nach SEQ ID NO: 27 und ein Oligonukleotid nach SEQ ID NO: 30 sind, und wobei besagte mindestens eine MG-spezifische Sonde aus SEQ ID NO: 28 oder 29 ist, oder die Sequenz ist, die vollständig komplementär zu dieser SEQ ID-Sequenz ist, über die gesamte Länge von dieser SEQ ID-Sequenz.

27. Das Verfahren zum Nachweis nach einem der Ansprüche 21-23, wobei besagte mindestens zwei auf MG gerichtete Primer ein Oligonukleotid nach SEQ ID NO: 33 und ein Oligonukleotid nach SEQ ID NO: 36 sind, und wobei besagte mindestens eine MG-spezifische Sonde aus SEQ ID NO: 34 oder 35 ist oder die Sequenz ist, die vollständig komplementär zu dieser SEQ ID-Sequenz ist, über die gesamte Länge dieser SEQ ID-Sequenz.

28. Das Verfahren zum Nachweis nach einem der Ansprüche 21-23, wobei besagte mindestens zwei auf MG gerichtete Primer ein Oligonukleotid nach SEQ ID NO: 39 und ein Oligonukleotid nach SEQ ID NO: 36 sind, und wobei besagte mindestens eine MG-spezifische Sonde aus SEQ ID NO: 40 oder 41 ist, oder die Sequenz ist, die vollständig komplementär zu dieser SEQ ID-Sequenz ist, über die gesamte Länge von dieser SEQ ID-Sequenz.

29. Das Verfahren zum Nachweis nach einem der Ansprüche 1-28, wobei besagte mindestens eine NG-spezifische Sonde aus SEQ ID NO: 45 ist, oder die Sequenz ist, die vollständig komplementär zu dieser SEQ ID-Sequenz ist, über die gesamte Länge von dieser SEQ ID-Sequenz, besagte Sonde ist optional verknüpft mit mindestens einem Detektionsmarker und/oder mindestens einem Nukleotidarm, der keinen Bezug zu CT, MG und NG hat, und dafür gedacht ist, einen Quencher oder einen Reporter zu tragen.

30. Das Verfahren zum Nachweis nach Anspruch 29, wobei besagte mindestens eine Sonde mindestens einen Beacon-Arm hat.

31. Das Verfahren zum Nachweis nach Anspruch 30, wobei besagte mindestens eine NG-spezifische Sonde aus SEQ ID NO: 46 ist oder die Sequenz ist, die vollständig komplementär zu dieser SEQ ID-Sequenz ist, über die gesamte Länge von dieser SEQ ID-Sequenz.

32. Das Verfahren zum Nachweis nach einem der Ansprüche 29-31, wobei besagte mindestens zwei auf NG gerichtete Primer ein Oligonukleotid nach SEQ ID NO: 44 und ein Oligonukleotid nach SEQ ID NO: 47 sind, und wobei besagte mindestens eine NG-spezifische Sonde aus SEQ ID NO: 45 oder 46 ist, oder die Sequenz ist, die vollständig komplementär zu dieser SEQ ID-Sequenz ist, über die gesamte Länge von dieser SEQ ID-Sequenz.

33. Das Verfahren zum Nachweis nach einem der Ansprüche 1-32, wobei:
- besagte mindestens zwei auf CT gerichtete Primer ein Oligonukleotid nach SEQ ID NO: 7 und ein Oligonukleotid nach SEQ ID NO: 12 sind, und
- besagte mindestens zwei auf MG gerichtete Primer ein Oligonukleotid nach SEQ ID NO: 15 und ein Oligonukleotid nach SEQ ID NO: 18 sind; oder ein Oligonukleotid nach SEQ ID NO: 21 und ein Oligonukleotid nach SEQ ID NO: 24; oder ein Oligonukleotid nach SEQ ID NO: 27 und ein Oligonukleotid nach SEQ ID NO: 30; oder ein Oligonukleotid nach SEQ ID NO: 33 und ein Oligonukleotid nach SEQ ID NO: 36; oder ein Oligonukleotid nach SEQ ID NO: 39 und ein Oligonukleotid nach SEQ ID NO: 36, und
- besagte mindestens zwei auf NG gerichtete Primer ein Oligonukleotid nach SEQ ID NO: 44 und ein Oligonukleotid nach SEQ ID NO: 47 sind.

34. Das Verfahren zum Nachweis nach einem der Ansprüche 1-33, wobei:
- besagte mindestens zwei auf CT gerichtete Primer ein Oligonukleotid nach SEQ ID NO: 7 und ein Oligonukleotid nach SEQ ID NO: 12 sind, und besagte mindestens eine CT-spezifische Sonde aus SEQ ID NO: 8, 9, 10 oder 11 ist, oder eine Sequenz ist, die vollständig komplementär zu dieser SEQ ID-Sequenz ist, über die gesamte Länge von dieser SEQ ID-Sequenz, und
- besagte mindestens zwei auf NG gerichtete Primer ein Oligonukleotid nach SEQ ID NO: 44 und ein Oligonukleotid nach SEQ ID NO: 47 sind, und besagte mindestens eine NG-spezifische Sonde aus SEQ ID NO: 45 oder 46 ist, oder die Sequenz ist, die vollständig komplementär zu dieser SEQ ID-Sequenz ist, über die gesamte Länge von dieser SEQ ID-Sequenz; und
- besagte mindestens zwei auf MG gerichtete Primer und besagte mindestens eine MG-spezifische Sonde sind wie folgt:
- besagte mindestens zwei auf MG gerichtete Primer sind ein Oligonukleotid nach SEQ ID NO: 15 und ein Oligonukleotid nach SEQ ID NO: 18, und besagte mindestens eine MG-spezifische Sonde ist aus SEQ ID NO: 16 oder 17, oder ist die Sequenz, die vollständig komplementär zu dieser SEQ ID-Sequenz ist, über die gesamte Länge von dieser SEQ ID-Sequenz; oder
- besagte mindestens zwei auf MG gerichtete Primer sind ein Oligonukleotid nach SEQ ID NO: 21 und ein Oligonukleotid nach SEQ ID NO: 24, und besagte mindestens eine MG-spezifische Sonde ist aus SEQ ID NO: 22 oder 23, oder ist die Sequenz, die vollständig komplementär zu dieser SEQ ID-Sequenz ist, über die gesamte Länge von dieser SEQ ID-Sequenz; oder
- besagte mindestens zwei auf MG gerichtete Primer sind ein Oligonukleotid nach SEQ ID NO: 27 und ein Oligonukleotid nach SEQ ID NO: 30, und besagte mindestens eine MG-spezifische Sonde ist aus SEQ ID NO: 28 oder 29, oder ist die Sequenz, die vollständig komplementär zu dieser SEQ ID-Sequenz ist, über die gesamte Länge von dieser SEQ ID-Sequenz; oder
- besagte mindestens zwei auf MG gerichtete Primer sind ein Oligonukleotid nach SEQ ID NO: 33 und ein Oligonukleotid nach SEQ ID NO: 36, und besagte mindestens eine MG-spezifische Sonde ist aus SEQ ID NO: 34 oder 35, oder ist die Sequenz, die vollständig komplementär zu dieser SEQ ID-Sequenz ist, über die gesamte Länge von dieser SEQ ID-Sequenz; oder
- besagte mindestens zwei auf MG gerichtete Primer sind ein Oligonukleotid nach SEQ ID NO: 39 und ein Oligonukleotid nach SEQ ID NO: 36, und besagte mindestens eine MG-spezifische Sonde ist aus SEQ ID NO: 40 oder 41, oder ist die Sequenz, die vollständig komplementär zu dieser SEQ ID-Sequenz ist, über die gesamte Länge von dieser SEQ ID-Sequenz.

35. Das Verfahren zum Nachweis nach einem der vorangehenden Ansprüche, wobei besagte mindestens zwei auf CT gerichtete Primer und/oder besagte mindestens zwei auf MG gerichtete Primer und/oder besagte mindestens zwei auf NG gerichtete Primer aus 17-25 Nukleotiden bestehen.

36. Das Verfahren zum Nachweis nach einem der vorangehenden Ansprüche, wobei besagte mindestens eine CT-spezifische Sonde und/oder besagte mindestens eine MG-spezifische Sonde und/oder besagte mindestens eine NG-spezifische Sonde aus 22-27 Nukleotiden besteht oder bestehen.

37. Das Verfahren zum Nachweis nach einem der vorangehenden Ansprüche, welches weiterhin die Amplifikation von einer Internen Kontrolle (Internal Control, IC) umfasst, die eine Sequenz ist, welche nicht mit CT, MG oder NG in Beziehung steht, so dass die IC SEQ ID NO: 48 ist.

38. Das Verfahren zum Nachweis nach einem der vorangehenden Ansprüche, welche eine Echtzeitmehrfachamplifikation ist.

39. Das Verfahren zum Nachweis nach Anspruch 38, worin besagte Amplifikation eine PCR ist.

40. Ein Verfahren für die Herstellung von Primern und Sonden, die in demselben Probengefäß für den Nachweis von CT und MG und NG in Echtzeitmehrfachamplifikation verwendet werden können, welche das Herstellen von mindestens einem Primerpaar und mindestens einer Sonde umfasst,
wobei ein Oligonukleotid von besagtem Primerpaar 14-30 Nukleotide hat, die Sequenz davon ist mindestens 80% identisch zu der Sequenz derselben Länge, die das exakte 5' terminale Ende einer Referenztemplatesequenz ist, und das andere Oligonukleotid von besagtem Primerpaar 14-30 Nukleotide hat, die Sequenz davon ist mindestens 80% identisch zu einer Sequenz derselben Länge, die das exakte 5' terminale Ende von einer Sequenz ist, die vollständig komplementär zu besagter Referenztemplatesequenz über die gesamte Länge von besagter Referenztemplatesequenz ist,
wobei besagte Sonde ist:
i. ein Fragment von mindestens 15 Nukleotiden von besagter Referenztemplatesequenz, oder ein Fragment von mindestens 15 Nukleotiden von einer Sequenz, die vollständig komplementär zu besagter Referenztemplatesequenz über die gesamte Länge von besagter Referenztemplatesequenz ist, oder
ii. eine konservierte Variante oder ein Fragment wie definiert in i., die Sequenz von besagter konservierter Variante ist mindestens 90% identisch zu der Sequenz von besagtem Fragment in i. über die gesamte Länge von besagtem Fragment in i., besagte Sonde ist optional verknüpft mit mindestens einem Detektionsmarker und/oder mindestens einem Beacon-Arm,
wobei besagte Referenztemplatesequenz ist:
- für das Design von CT-Primern und Sonden: ein Fragment bestehend aus den Positionen 5571-5760 (SEQ ID NO: 5) der CT-Sequenz in SEQ ID NO: 1, oder ein konserviertes Subfragment davon, welches die Eigenschaft behalten hat, eine geeignete Referenztemplatesequenz zu sein, zum Konstruieren und Herstellen von auf CT gerichteten Primern, welche einen Echtzeitmehrfachnachweis von CT erlauben, oder eine Sequenz, die vollständig komplementär zu besagtem Fragment oder Subfragment über die gesamte Länge von besagtem Fragment oder Subfragment ist, wobei besagtes konserviertes Subfragment aus den Positionen 5580-5754 (SEQ ID NO: 6) der CT-Sequenz in SEQ ID NO: 1 besteht,
- für das Design von MG-Primern und Sonden: ein Fragment bestehend aus:
- Positionen 1-270 (SEQ ID NO: 13) der MG-Sequenz in SEQ ID NO: 2, oder ein konserviertes Subfragment davon, welches die Eigenschaft behalten hat, eine geeignete Referenztemplatesequenz zu sein, zum Konstruieren und Herstellen von auf MG gerichteten Primern, welche den Echtzeitmehrfachnachweis von MG erlauben, oder eine Sequenz, die vollständig komplementär zu besagtem Fragment oder Subfragment über die gesamte Länge von besagtem Fragment oder Subfragment ist, wobei besagtes konserviertes Subfragment aus den Positionen 2-259 (SEQ ID NO: 14) der MG-Sequenz in SEQ ID NO: 2 besteht,
- Positionen 1140-1290 (SEQ ID NO: 19) der MG-Sequenz in SEQ ID NO: 3, oder ein konserviertes Subfragment davon, welches die Eigenschaft behalten hat, eine geeignete Referenztemplatesequenz zu sein, zum Konstruieren und Herstellen von auf MG gerichteten Primern, welche den Echtzeitmehrfachnachweis von MG erlauben, oder eine Sequenz, die vollständig komplementär zu besagtem Fragment oder Subfragment über die gesamte Länge von besagtem Fragment oder Subfragment ist, wobei besagtes konserviertes Subfragment aus den Positionen 1144-1283 (SEQ ID NO: 20) der MG-Sequenz in SEQ ID NO: 3 besteht,
- Positionen 1060-1250 (SEQ ID NO: 25) der MG-Sequenz in SEQ ID NO: 3, oder ein konserviertes Subfragment davon, welches die Eigenschaft behalten hat, eine geeignete Referenztemplatesequenz zu sein, zum Konstruieren und Herstellen von auf MG gerichteten Primern, welche den Echtzeitmehrfachnachweis von MG erlauben, oder eine Sequenz, die vollständig komplementär zu besagtem Fragment oder Subfragment über die gesamte Länge von besagtem Fragment oder Subfragment ist, wobei besagtes konserviertes Subfragment aus den Positionen 1064-1249 (SEQ ID NO: 26) der MG-Sequenz in SEQ ID NO: 3 besteht,
- Positionen 1520-1710 (SEQ ID NO: 31) der MG-Sequenz in SEQ ID NO: 3, oder ein konserviertes Subfragment davon, welches die Eigenschaft behalten hat, eine geeignete Referenztemplatesequenz zu sein, zum Konstruieren und Herstellen von auf MG gerichteten Primern, welche den Echtzeitmehrfachnachweis von MG erlauben, oder eine Sequenz, die vollständig komplementär zu besagtem Fragment oder Subfragment über die gesamte Länge von besagtem Fragment oder Subfragment ist, wobei besagtes konserviertes Subfragment aus den Positionen 1527-1704 (SEQ ID NO: 32) der MG-Sequenz in SEQ ID NO: 3 besteht,
- Positionen 1500-1710 (SEQ ID NO: 37) der MG-Sequenz in SEQ ID NO: 3, oder ein konserviertes Subfragment davon, welches die Eigenschaft behalten hat, eine geeignete Referenztemplatesequenz zu sein, zum Konstruieren und Herstellen von auf MG gerichteten Primern, welche den Echtzeitmehrfachnachweis von MG erlauben, oder eine Sequenz, die vollständig komplementär zu besagtem Fragment oder Subfragment über die gesamte Länge von besagtem Fragment oder Subfragment ist, wobei besagtes konserviertes Subfragment aus den Positionen 1501-1704 (SEQ ID NO: 38) der MG-Sequenz in SEQ ID NO: 3 besteht,
- für das Design von NG-Primern und Sonden: ein Fragment bestehend aus Positionen 101-380 (SEQ ID NO: 42) der NG-Sequenz in SEQ ID NO: 4 oder ein konserviertes Subfragment davon, welches die Eigenschaft behalten hat, eine geeignete Referenztemplatesequenz zu sein, zum Konstruieren und Herstellen von auf NG gerichteten Primern, welche den Echtzeitmehrfachnachweis von NG erlauben, oder eine Sequenz, die vollständig komplementär zu besagtem Fragment oder Subfragment über die gesamte Länge von besagtem Fragment oder Subfragment ist, wobei besagtes konserviertes Subfragment aus den Positionen 114-365 (SEQ ID NO: 43) der NG-Sequenz in SEQ ID NO: 4 besteht.

41. Ein Primer, welcher speziell für den Nachweis von CT und/oder MG und/oder NG in Echtzeitmehrfachamplifikation angepasst ist, welcher ist:
ein Oligonukleotid von 14-30 Nukleotiden, die Sequenz davon ist mindestens 80% identisch zu einer Sequenz von derselben Länge, die das exakte 5' terminale Ende von einer Referenztemplatesequenz oder von einer Sequenz ist, die vollständig komplementär zu besagter Referenztemplatesequenz über die gesamte Länge von besagter Referenztemplatesequenz ist,
wobei besagte Referenztemplatesequenz ist:
- SEQ ID NO: 6; oder
- SEQ ID NO: 14 oder SEQ ID NO: 20 oder SEQ ID NO: 26 oder SEQ ID NO: 32 oder SEQ ID NO: 38; oder
- SEQ ID NO: 43.

42. Der Primer nach Anspruch 41, welcher ist:
- ein auf CT gerichteter Primer, ausgewählt aus der Gruppe bestehend aus SEQ ID NO: 7; 12, oder
- ein auf MG gerichteter Primer, ausgewählt aus der Gruppe bestehend aus SEQ ID NO: 15; 18; 21; 24; 27; 30; 33; 36; 39, oder
- ein auf NG gerichteter Primer, ausgewählt aus der Gruppe bestehend aus SEQ ID NO: 44; 47.

43. Ein Primer-System, welches speziell für den Nachweis von CT und/oder MG und/oder NG in Echtzeitmehrfachamplifikation angepasst ist, welches umfasst:
mindestens ein Paar von Oligonukleotiden von jeweils 14-30 Nukleotiden,
die Sequenz von einem Oligonukleotid von besagtem Paar ist mindestens 80% identisch zu einer Sequenz derselben Länge, die das exakte 5' terminale Ende einer Referenztemplatesequenz ist, und
die Sequenz des anderen Oligonukleotids von besagtem Paar ist mindestens 80% identisch zu einer Sequenz von derselben Länge, die das exakte 5' terminale Ende einer Sequenz ist, die vollständig komplementär zu besagter Referenztemplatesequenz über die gesamte Länge von besagter Referenztemplatesequenz ist,
wobei besagte Referenztemplatesequenz ist:
- SEQ ID NO: 6; oder
- SEQ ID NO: 14 oder SEQ ID NO: 20 oder SEQ ID NO: 26 oder SEQ ID NO: 32 oder SEQ ID NO: 38; oder
- SEQ ID NO: 43.

44. Das Primer-System nach Anspruch 43, welches umfasst:
- mindestens ein auf CT gerichtetes Primerpaar nach SEQ ID NO: 7 und SEQ ID NO: 12, und/oder
- mindestens ein auf MG gerichtetes Primerpaar ausgewählt aus den folgenden Paaren: SEQ ID NO: 15 und 18; SEQ ID NO: 21 und 24; SEQ ID NO: 27 und 30; SEQ ID NO: 33 und 36; SEQ ID NO: 39 und 36, und/oder
- mindestens ein auf NG gerichtetes Primerpaar nach SEQ ID NO: 44 und 47.

45. Das Primersystem nach Anspruch 43 oder 44, welches drei von besagten Paaren von Oligonukleotiden umfasst, und wobei besagte Referenztemplatesequenz ist:
- für eins von besagten drei Paaren: SEQ ID NO: 6;
- für ein anderes von besagten drei Paaren: SEQ ID NO: 14 oder SEQ ID NO: 20 oder SEQ ID NO: 26 oder SEQ ID NO: 32 oder SEQ ID NO: 38;
- für wieder ein anderes von besagten drei Paaren: SEQ ID NO: 43.

46. Eine Sonde, welche speziell für den Nachweis von CT und/oder MG und/oder NG in Echtzeitmehrfachamplifikation angepasst ist, welche ist:
- eine CT-spezifische Sonde aus SEQ ID NO: 8; oder 10, oder welche aus einer Sequenz besteht, die vollständig komplementär zu dieser SEQ ID-Sequenz ist, über die gesamte Länge von dieser SEQ ID-Sequenz,
- eine MG-spezifische Sonde aus SEQ ID NO: 16; 22; 28: 34 oder 40, oder welche aus einer Sequenz besteht, die vollständig komplementär zu dieser SEQ ID-Sequenz ist, über die gesamte Länge von dieser (SEQ ID-Sequenz,
- eine NG-spezifische Sonde aus SEQ ID NO: 45, oder welche aus einer Sequenz besteht, die vollständig komplementär zu dieser SEQ ID-Sequenz ist, über die gesamte Länge von dieser SEQ ID-Sequenz,
besagte Sonde ist optional verknüpft mit mindestens einem Detektionsmarker und/oder mindestens einem Beacon-Arm.

47. Die Sonde nach Anspruch 46, welche ist:
- eine CT-spezifische Sonde aus SEQ ID NO: 9 oder 11, oder welche aus einer Sequenz besteht, die vollständig komplementär zu dieser SEQ ID-Sequenz ist, über die gesamte Länge von dieser SEQ ID-Sequenz,
- eine MG-spezifische Sonde aus SEQ ID NO: 17; 23; 29; 35 oder 41, oder welche aus einer Sequenz besteht, die vollständig komplementär zu dieser SEQ ID-Sequenz ist, über die gesamte Länge von dieser SEQ ID-Sequenz,
- eine NG-spezifische Sonde aus SEQ ID NO: 45 oder 46, oder welche aus einer Sequenz besteht, die vollständig komplementär zu dieser SEQ ID-Sequenz ist, über die gesamte Länge von dieser SEQ ID-Sequenz.

48. Ein Primer- und Sondensystem, welches speziell für den Nachweis von CT und/oder MG und/oder NG in Echtzeitmehrfachamplifikation angepasst ist, welches mindestens ein Primersystem von einem der Ansprüche 43-45 umfasst, und mindestens eine Sonde nach einem der Ansprüche 46-47.

49. Eine Amplifikationszusammensetzung, umfassend mindestens ein Amplikon erhältlich durch Implementierung des Verfahrens nach einem der Ansprüche 1-39 auf eine CT- und/oder MG- und/oder NG-enthaltende Probe, oder durch Amplifikation von mindestens einer Nukleinsäure von einer CT- und/oder MG- und/oder NG-enthaltenden Probe mittels mindestens einem Primersystem nach einem der Ansprüche 43-45, und weiterhin umfassend:
- die auf CT gerichteten Primer wie definiert in Anspruch 1 und die auf MG gerichteten Primer wie definiert in Anspruch 1 und die auf NG gerichteten Primer wie definiert in Anspruch 1; oder
- das Primersystem nach Anspruch 45.

50. Die Amplifikationszusammensetzung nach Anspruch 49, welche drei Amplikons umfasst, wobei besagte drei Amplikons erhältlich sind:
- durch Implementierung des Verfahrens nach einem der Ansprüche 1-39 auf eine CT- und MG- und NG-enthaltende Probe, oder
- durch Amplifikation von mindestens einer Nukleinsäure von CT, und von mindestens einer Nukleinsäure von MG, und mindestens einer Nukleinsäure von NG mittels des Primersystems nach Anspruch 45.

51. Ein Kit für die Diagnose von einer Infektion durch CT und/oder MG und/oder NG, welches umfasst:
- mindestens ein Primersystem nach einem der Ansprüche 43-45, und/oder
- mindestens eine Sonde nach einem der Ansprüche 46-47,
- optional, Anweisungen für die Verwendung davon und/oder Nukleotide.

52. Das Kit nach Anspruch 50, welches weiterhin ein Internes Kontroll (Internal Control, IC)-Oligonukleotid nach SEQ ID NO: 48 und/oder das IC-Primerpaar nach SEQ ID NO: 49 und NO: 52, und/oder die IC-Sonde nach SEQ ID NO: 50, und/oder die IC-Sonde nach SEQ ID NO: 51 umfasst.

## Revendications

1. Procédé pour la détection de *Chlamydia trachomatis* (CT) et/ou *Mycoplasma genitalium* (MG) et/ou *Neisseria gonorrhoeae* (NG), dans un échantillon, qui est approprié pour ladite détection dans une amplification multiplex en temps réel,
dans lequel ladite détection comprend le fait de déterminer si au moins un amplicon a été, ou est, produit à partir dudit échantillon, ou d'un matériau d'acide nucléique de celui-ci, par une amplification au moyen d'amorces d'amplification,
par lequel une détermination positive indique qu'au moins un CT et/ou au moins un MG et/ou au moins un NG est (sont) présent(s) dans ledit échantillon,
dans lequel lesdites amorces d'amplification comprennent :
- au moins deux amorces, qui sont prévues pour cibler CT, et qui sont appropriées pour la détection de CT dans une amplification multiplex en temps réel,
dans lequel lesdites au moins deux amorces ciblées vers CT sont des oligonucléotides, qui consistent en 14 à 30 nucléotides, dont les séquences sont appropriées pour une utilisation comme amorces sens et antisens, respectivement, dans l'amplification d'au moins une séquence matrice de référence de CT, dans laquelle ladite au moins une séquence matrice de référence de CT est un fragment constitué par les positions 5571 à 5760 (SEQ ID No: 5) de la séquence CT de SEQ ID No: 1, ou un sous-fragment conservateur de celle-ci, qui a conservé la propriété d'être une séquence matrice de référence appropriée, pour construire et produire des amorces ciblées vers CT, qui permettent une détection multiplex en temps réel de CT,
et
- au moins deux amorces, qui sont prévues pour cibler MG, et qui sont appropriées pour la détection de MG dans une amplification multiplex en temps réel,
dans lequel lesdites au moins deux amorces ciblées vers MG sont des oligonucléotides, qui consistent en 14 à 30 nucléotides, dont les séquences sont appropriées pour une utilisation comme amorces sens et antisens, respectivement, dans l'amplification d'au moins une séquence matrice de référence de MG, dans laquelle ladite au moins une séquence matrice de référence de MG est un fragment constitué par :
- les positions 1 à 270 (SEQ ID No: 13) de la séquence MG de SEQ ID No: 2, ou un sous-fragment conservateur de celle-ci, qui a conservé la propriété d'être une séquence matrice de référence appropriée, pour construire et produire des amorces ciblées vers MG, qui permettent une détection multiplex en temps réel de MG,
- les positions 1140 à 1290 (SEQ ID No: 19) de la séquence MG de SEQ ID No: 3, ou un sous-fragment conservateur de celle-ci, qui a conservé la propriété d'être une séquence matrice de référence appropriée, pour construire et produire des amorces ciblées vers MG, qui permettent une détection multiplex en temps réel de MG,
- les positions 1060 à 1250 (SEQ ID No: 25) de la séquence MG de SEQ ID No: 3, ou un sous-fragment conservateur de celle-ci, qui a conservé la propriété d'être une séquence matrice de référence appropriée, pour construire et produire des amorces ciblées vers MG, qui permettent une détection multiplex en temps réel de MG,
- les positions 1520 à 1710 (SEQ ID No: 31) de la séquence MG de SEQ ID No: 3, ou un sous-fragment conservateur de celle-ci, qui a conservé la propriété d'être une séquence matrice de référence appropriée, pour construire et produire des amorces ciblées vers MG, qui permettent une détection multiplex en temps réel de MG,
- les positions 1500 à 1710 (SEQ ID No: 37) de la séquence MG de SEQ ID No: 3, ou un sous-fragment conservateur de celle-ci, qui a conservé la propriété d'être une séquence matrice de référence appropriée, pour construire et produire des amorces ciblées vers MG, qui permettent une détection multiplex en temps réel de MG,
et
- au moins deux amorces, qui sont prévues pour cibler NG, et qui sont appropriées pour la détection de NG dans une amplification multiplex en temps réel,
dans lequel lesdites au moins deux amorces ciblées vers NG sont des oligonucléotides, qui consistent en 14 à 30 nucléotides, dont les séquences sont appropriées pour une utilisation comme amorces sens et antisens, respectivement, dans l'amplification d'au moins une séquence matrice de référence de NG, dans laquelle ladite au moins une séquence matrice de référence de NG est un fragment constitué par les positions 101 à 380 (SEQ ID No: 42) de la séquence NG de SEQ ID No: 4, ou un sous-fragment conservateur de celle-ci, qui a conservé la propriété d'être une séquence matrice de référence appropriée, pour construire et produire des amorces ciblées vers NG, qui permettent une détection multiplex en temps réel de NG,
dans lequel ladite détermination si au moins un amplicon a été ou est produit, est réalisée au moyen d'au moins une sonde, qui est prévue pour s'hybrider audit au moins un amplicon, et dans lequel ladite au moins une sonde comprend :
- au moins une sonde spécifique de CT, qui est appropriée pour la détection de CT dans une amplification multiplex en temps réel, dans laquelle ladite au moins une sonde spécifique de CT est :
i. un fragment d'au moins 15 nucléotides de ladite séquence matrice de référence de CT, ou un fragment d'au moins 15 nucléotides de la séquence qui est entièrement complémentaire de ladite séquence matrice de référence de CT sur la longueur entière de ladite séquence matrice de référence, ou
ii. un variant conservateur d'un tel fragment, dont la séquence a au moins 90 % d'identité avec un fragment de i.
sur la longueur entière dudit fragment de i.,
et
- au moins une sonde spécifique de MG, qui est appropriée pour la détection de MG dans une amplification multiplex en temps réel, dans laquelle ladite au moins une sonde spécifique de MG est :
iii. un fragment d'au moins 15 nucléotides de ladite séquence matrice de référence de MG, ou un fragment d'au moins 15 nucléotides de la séquence qui est entièrement complémentaire de ladite séquence matrice de référence de MG sur la longueur entière de ladite séquence matrice de référence, ou
iv. un variant conservateur d'un tel fragment, dont la séquence a au moins 90 % d'identité avec un fragment de iii. sur la longueur entière dudit fragment de iii.,
et
- au moins une sonde spécifique de NG, qui est appropriée pour la détection de NG dans une amplification multiplex en temps réel, dans laquelle ladite au moins une sonde spécifique de NG est :
v. un fragment d'au moins 15 nucléotides de ladite séquence matrice de référence de NG, ou un fragment d'au moins 15 nucléotides de la séquence qui est entièrement complémentaire de ladite séquence matrice de référence de NG sur la longueur entière de ladite séquence matrice de référence, ou
vi. un variant conservateur d'un tel fragment, dont la séquence a au moins 90 % d'identité avec un fragment de v. sur la longueur entière dudit fragment de v.,
lesdites séquences matrices de référence de CT, MG et NG partageant la caractéristique technique spéciale d'être des séquences matrices de référence, qui sont appropriées pour la construction et la production d'amorces ciblées vers CT, MG et NG, ainsi que de sondes spécifiques de CT, MG et NG, qui peuvent être utilisées ensemble en multiplex dans le même tube pour détecter spécifiquement CT et/ou MG et/ou NG, avantageusement CT et MG et NG, en temps réel dans ledit même tube.

2. Procédé de détection selon la revendication 1, dans lequel ladite au moins une séquence matrice de référence de CT est le fragment constitué des positions 5580 à 5754 (SEQ ID No: 6) de la séquence CT de SEQ ID No: 1.

3. Procédé de détection selon la revendication 1 ou 2, dans lequel ladite au moins une séquence matrice de référence MG est :
- le fragment constitué des positions 2 à 259 (SEQ ID No: 14) de la séquence MG de SEQ ID No: 2, ou
- le fragment constitué des positions 1144 à 1283 (SEQ ID No: 20) de la séquence MG de SEQ ID No: 3, ou
- le fragment constitué des positions 1064 à 1249 (SEQ ID No: 26) de la séquence MG de SEQ ID No: 3, ou
- le fragment constitué des positions 1527 à 1704 (SEQ ID No: 32) de la séquence MG de SEQ ID No: 3, ou
- le fragment constitué des positions 1501 à 1704 (SEQ ID No: 38) de la séquence MG de SEQ ID No: 3,
lesdites séquences matrices de référence de MG partageant la caractéristique technique spécifique d'être des références appropriées pour construire et produire des amorces ciblées vers MG, ainsi que des sondes spécifiques de MG, qui peuvent être utilisées ensemble en multiplex dans le même tube pour détecter spécifiquement CT et/ou MG et/ou NG, avantageusement CT et MG et NG, en temps réel dans ledit même tube.

4. Procédé de détection selon l'une quelconque des revendications précédentes, dans lequel ladite au moins une séquence matrice de référence de NG est le fragment constitué des positions 114 à 365 (SEQ ID No: 43) de la séquence NG de SEQ ID No: 4.

5. Procédé de détection selon l'une quelconque des revendications précédentes, dans lequel lesdites au moins deux amorces ciblées vers CT sont un oligonucléotide de SEQ ID No: 7, et un oligonucléotide de SEQ ID No: 12.

6. Procédé de détection selon l'une quelconque des revendications précédentes, dans lequel lesdites au moins deux amorces ciblées vers MG sont un oligonucléotide de SEQ ID No: 15 et un oligonucléotide de SEQ ID No: 18, ou sont au moins un oligonucléotide choisi dans le groupe constitué de SEQ ID No: 21 ; 27 ; 33 ; 39, et au moins un oligonucléotide choisi dans le groupe constitué de SEQ ID No: 24 ; 30 ; 36.

7. Procédé de détection selon la revendication 6, dans lequel lesdites au moins deux amorces ciblées vers MG sont un oligonucléotide de SEQ ID No: 15 et un oligonucléotide de SEQ ID No: 18.

8. Procédé de détection selon la revendication 6, dans lequel lesdites au moins deux amorces ciblées vers MG sont au moins un oligonucléotide choisi dans le groupe constitué de SEQ ID No: 21 ; 27 et au moins un oligonucléotide choisi dans le groupe constitué de SEQ ID No: 24 ; 30 ; 36.

9. Procédé de détection selon la revendication 8, dans lequel lesdites au moins deux amorces ciblées vers MG sont un oligonucléotide de SEQ ID No: 21 et au moins un oligonucléotide choisi dans le groupe constitué de SEQ ID No: 24 ; 36.

10. Procédé de détection selon la revendication 9, dans lequel lesdites au moins deux amorces ciblées vers MG sont un oligonucléotide de SEQ ID No: 21 et un oligonucléotide de SEQ ID No: 24.

11. Procédé de détection selon la revendication 8, dans lequel lesdites au moins deux amorces ciblées vers MG sont un oligonucléotide de SEQ ID No: 27 et au moins un oligonucléotide choisi dans le groupe constitué de SEQ ID No: 24 ; 30 ; 36.

12. Procédé de détection selon la revendication 11, dans lequel lesdites au moins deux amorces ciblées vers MG sont un oligonucléotide de SEQ ID No: 27 et un oligonucléotide de SEQ ID No: 30.

13. Procédé de détection selon la revendication 6, dans lequel lesdites au moins deux amorces ciblées vers MG sont un oligonucléotide de SEQ ID No: 36, et au moins un oligonucléotide choisi dans le groupe constitué de SEQ ID No: 21 ; 27 ; 33 et 39.

14. Procédé de détection selon la revendication 13, dans lequel lesdites au moins deux amorces ciblées vers MG sont un oligonucléotide de SEQ ID No: 33 et un oligonucléotide de SEQ ID No: 36.

15. Procédé de détection selon la revendication 13, dans lequel lesdites au moins deux amorces ciblées vers MG sont un oligonucléotide de SEQ ID No: 39 et un oligonucléotide de SEQ ID No: 36.

16. Procédé de détection selon l'une quelconque des revendications précédentes, dans lequel lesdites au moins deux amorces ciblées vers NG sont un oligonucléotide de SEQ ID No: 44 et un oligonucléotide de SEQ ID No: 47.

17. Procédé de détection selon l'une quelconque des revendications 1 à 16, dans lequel ladite au moins une sonde spécifique de CT est de SEQ ID No: 8 ou 10, ou est la séquence qui est entièrement complémentaire de cette séquence SEQ ID, sur la longueur entière de cette séquence SEQ ID, ladite sonde étant éventuellement liée à au moins une marque de détection et/ou au moins un bras nucléotidique qui est non apparenté à CT, MG et NG et qui est prévu pour porter un extincteur ou un rapporteur.

18. Procédé de détection selon la revendication 17, dans lequel ladite au moins une sonde est liée à au moins un bras balise.

19. Procédé de détection selon la revendication 18, dans lequel ladite au moins une sonde spécifique de CT est de SEQ ID No: 9 ou 11, ou la séquence qui est entièrement complémentaire de cette séquence SEQ ID, sur la longueur entière de cette séquence SEQ ID.

20. Procédé de détection selon l'une quelconque des revendications 17 à 19, dans lequel lesdites au moins deux amorces ciblées vers CT sont un oligonucléotide de SEQ ID No: 7 et un oligonucléotide de SEQ ID No: 12, et ladite au moins une sonde spécifique de CT est choisie dans le groupe constitué de SEQ ID No: 8 ; 9 ; 10 ; 11.

21. Procédé de détection selon l'une quelconque des revendications 1 à 20, dans lequel ladite au moins une sonde spécifique de MG est choisie dans le groupe constitué de SEQ ID No: 16 ; 22 ; 28 ; 34 et 40, ou est la séquence qui est entièrement complémentaire de cette séquence SEQ ID, sur la longueur entière de cette séquence SEQ ID, ladite sonde étant éventuellement liée à au moins une marque de détection et/ou au moins un bras nucléotidique qui est non apparenté à CT, MG et NG et qui est prévu pour porter un extincteur ou un rapporteur.

22. Procédé de détection selon la revendication 21, dans lequel ladite au moins une sonde a au moins un bras balise.

23. Procédé de détection selon la revendication 22, dans lequel ladite au moins une sonde spécifique de MG est choisie dans le groupe constitué de SEQ ID No: 17 ; 23 ; 29 ; 35 et 41, ou est la séquence qui est entièrement complémentaire de cette séquence SEQ ID, sur la longueur entière de cette séquence SEQ ID.

24. Procédé de détection selon l'une quelconque des revendications 21 à 23, dans lequel lesdites au moins deux amorces ciblées vers MG sont un oligonucléotide de SEQ ID No: 15 et un oligonucléotide de SEQ ID No: 18, et dans lequel ladite au moins une sonde spécifique de MG est de SEQ ID No: 16 ou 17, ou est la séquence qui est entièrement complémentaire de cette séquence SEQ ID, sur la longueur entière de cette séquence SEQ ID.

25. Procédé de détection selon l'une quelconque des revendications 21 à 23, dans lequel lesdites au moins deux amorces ciblées vers MG sont un oligonucléotide de SEQ ID No: 21 et un oligonucléotide de SEQ ID No: 24, et dans lequel ladite au moins une sonde spécifique de MG est de SEQ ID No: 22 ou 23, ou est la séquence qui est entièrement complémentaire de cette séquence SEQ ID, sur la longueur entière de cette séquence SEQ ID.

26. Procédé de détection selon l'une quelconque des revendications 21 à 23, dans lequel lesdites au moins deux amorces ciblées vers MG sont un oligonucléotide de SEQ ID No: 27 et un oligonucléotide de SEQ ID No: 30, et dans lequel ladite au moins une sonde spécifique de MG est de SEQ ID No: 28 ou 29, ou est la séquence qui est entièrement complémentaire de cette séquence SEQ ID, sur la longueur entière de cette séquence SEQ ID.

27. Procédé de détection selon l'une quelconque des revendications 21 à 23, dans lequel lesdites au moins deux amorces ciblées vers MG sont un oligonucléotide de SEQ ID No: 33 et un oligonucléotide de SEQ ID No: 36, et dans lequel ladite au moins une sonde spécifique de MG est de SEQ ID No: 34 ou 35, ou est la séquence qui est entièrement complémentaire de cette séquence SEQ ID, sur la longueur entière de cette séquence SEQ ID.

28. Procédé de détection selon l'une quelconque des revendications 21 à 23, dans lequel lesdites au moins deux amorces ciblées vers MG sont un oligonucléotide de SEQ ID No: 39 et un oligonucléotide de SEQ ID No: 36, et dans lequel ladite au moins une sonde spécifique de MG est de SEQ ID No: 40 ou 41, ou est la séquence qui est entièrement complémentaire de cette séquence SEQ ID, sur la longueur entière de cette séquence SEQ ID.

29. Procédé de détection selon l'une quelconque des revendications 1 à 28, dans lequel ladite au moins une sonde spécifique de NG est de SEQ ID No: 45, ou est la séquence qui est entièrement complémentaire de cette séquence SEQ ID, sur la longueur entière de cette séquence SEQ ID, ladite sonde étant éventuellement liée à au moins une marque de détection et/ou au moins un bras nucléotidique qui est non apparenté à CT, MG et NG et qui est prévu pour porter un extincteur ou un rapporteur.

30. Procédé de détection selon la revendication 29, dans lequel ladite au moins une sonde a au moins un bras balise.

31. Procédé de détection selon la revendication 30, dans lequel ladite au moins une sonde spécifique de NG est de SEQ ID No: 46, ou est la séquence qui est entièrement complémentaire de cette séquence SEQ ID, sur la longueur entière de cette séquence SEQ ID.

32. Procédé de détection selon l'une quelconque des revendications 29 à 31, dans lequel lesdites au moins deux amorces ciblées vers NG sont un oligonucléotide de SEQ ID No: 44 et un oligonucléotide de SEQ ID No: 47, et dans lequel ladite au moins une sonde spécifique de NG est de SEQ ID No: 45 ou 46, ou est la séquence qui est entièrement complémentaire de cette séquence SEQ ID, sur la longueur entière de cette séquence SEQ ID.

33. Procédé de détection selon l'une quelconque des revendications 1 à 32, dans lequel :
- lesdites au moins deux amorces ciblées vers CT sont un oligonucléotide de SEQ ID No: 7 et un oligonucléotide de SEQ ID No: 12, et
- lesdites au moins deux amorces ciblées vers MG sont un oligonucléotide de SEQ ID No: 15 et un oligonucléotide de SEQ ID No: 18 ; ou un oligonucléotide de SEQ ID No: 21 et un oligonucléotide de SEQ ID No: 24 ; ou un oligonucléotide de SEQ ID No: 27 et un oligonucléotide de SEQ ID No: 30 ; ou un oligonucléotide de SEQ ID No: 33 et un oligonucléotide de SEQ ID No: 36 ; ou un oligonucléotide de SEQ ID No: 39 et un oligonucléotide de SEQ ID No: 36, et
- lesdites au moins deux amorces ciblées vers NG sont un oligonucléotide de SEQ ID No: 44 et un oligonucléotide de SEQ ID No: 47.

34. Procédé de détection selon l'une quelconque des revendications 1 à 33, dans lequel :
- lesdites au moins deux amorces ciblées vers CT sont un oligonucléotide de SEQ ID No: 7 et un oligonucléotide de SEQ ID No: 12, et ladite au moins une sonde spécifique de CT est de SEQ ID No: 8, 9, 10 ou 11, ou est la séquence qui est entièrement complémentaire de cette séquence SEQ ID, sur la longueur entière de cette séquence SEQ ID, et
- lesdites au moins deux amorces ciblées vers NG sont un oligonucléotide de SEQ ID No: 44 et un oligonucléotide de SEQ ID No: 47, et ladite au moins une sonde spécifique de NG est de SEQ ID No: 45 ou 46, ou est la séquence qui est entièrement complémentaire de cette séquence SEQ ID, sur la longueur entière de cette séquence SEQ ID ; et
- lesdites au moins deux amorces ciblées vers MG et ladite au moins une sonde spécifique de MG sont comme suit :
- lesdites au moins deux amorces ciblées vers MG sont un oligonucléotide de SEQ ID No: 15 et un oligonucléotide de SEQ ID No: 18, et ladite au moins une sonde spécifique de MG est de SEQ ID No: 16 ou 17, ou est la séquence qui est entièrement complémentaire de cette séquence SEQ ID, sur la longueur entière de cette séquence SEQ ID ; ou
- lesdites au moins deux amorces ciblées vers MG sont un oligonucléotide de SEQ ID No: 21 et un oligonucléotide de SEQ ID No: 24, et ladite au moins une sonde spécifique de MG est de SEQ ID No: 22 ou 23, ou est la séquence qui est entièrement complémentaire de cette séquence SEQ ID, sur la longueur entière de cette séquence SEQ ID ; ou
- lesdites au moins deux amorces ciblées vers MG sont un oligonucléotide de SEQ ID No: 27 et un oligonucléotide de SEQ ID No: 30, et ladite au moins une sonde spécifique de MG est de SEQ ID No: 28 ou 29, ou est la séquence qui est entièrement complémentaire de cette séquence SEQ ID, sur la longueur entière de cette séquence SEQ ID ; ou
- lesdites au moins deux amorces ciblées vers MG sont un oligonucléotide de SEQ ID No: 33 et un oligonucléotide de SEQ ID No: 36, et ladite au moins une sonde spécifique de MG est de SEQ ID No: 34 ou 35, ou est la séquence qui est entièrement complémentaire de cette séquence SEQ ID, sur la longueur entière de cette séquence SEQ ID ; ou
- lesdites au moins deux amorces ciblées vers MG sont un oligonucléotide de SEQ ID No: 39 et un oligonucléotide de SEQ ID No: 36, et ladite au moins une sonde spécifique de MG est de SEQ ID No: 40 ou 41, ou est la séquence qui est entièrement complémentaire de cette séquence SEQ ID, sur la longueur entière de cette séquence SEQ ID.

35. Procédé de détection selon l'une quelconque des revendications précédentes, dans lequel lesdites au moins deux amorces ciblées vers CT et/ou lesdites au moins deux amorces ciblées vers MG et/ou lesdites au moins deux amorces ciblées vers NG consistent en 17 à 25 nucléotides.

36. Procédé de détection selon l'une quelconque des revendications précédentes, dans lequel ladite au moins une sonde spécifique de CT et/ou ladite au moins une sonde spécifique de MG et/ou ladite au moins une sonde spécifique de NG consiste(nt) en 22 à 27 nucléotides.

37. Procédé de détection selon l'une quelconque des revendications précédentes, qui comprend en outre
l'amplification d'un Contrôle Interne (CI), dont la séquence est non apparentée à CT, MG, NG, tel que le CI de SEQ ID No: 48.

38. Procédé de détection selon l'une quelconque des revendications précédentes, qui est une amplification mutliplex en temps réel.

39. Procédé de détection selon la revendication 38, dans lequel ladite amplification est une PCR.

40. Procédé de production d'amorces et sondes qui peuvent être utilisées dans le même tube pour la détection de CT et MG et NG dans une amplification multiplex en temps réel, qui comprend la production d'au moins une paire d'amorces et d'au moins une sonde,
dans lequel un oligonucléotide de ladite paire d'amorces est de 14 à 30 nucléotides, dont la séquence est au moins identique à 80 % à la séquence de la même longueur qui est l'extrémité terminale 5' exacte d'une séquence matrice de référence, et l'autre oligonucléotide de ladite paire d'amorces est de 14 à 30 nucléotides, dont la séquence est au moins identique à 80 % à la séquence de la même longueur qui est l'extrémité terminale 5' exacte de la séquence qui est entièrement complémentaire de ladite séquence matrice de référence sur la longueur entière de ladite séquence matrice de référence, dans lequel ladite sonde est :
i. un fragment d'au moins 15 nucléotides de ladite séquence matrice de référence, ou un fragment d'au moins 15 nucléotides de la séquence qui est entièrement complémentaire de ladite séquence matrice de référence sur la longueur entière de ladite séquence matrice de référence, ou
ii. un variant conservateur d'un fragment tel que défini en i., la séquence dudit variant conservateur étant au moins identique à 90 % à la séquence dudit fragment de i. sur la longueur entière dudit fragment de i.,
ladite sonde étant éventuellement liée à au moins une marque de détection et/ou au moins un bras balise,
dans lequel ladite séquence matrice de référence est :
- pour la conception d'amorces et sondes CT : un fragment constitué des positions 5571 à 5760 (SEQ ID No: 5) de la séquence CT de SEQ ID No: 1, ou un sous-fragment conservateur de celle-ci, qui a conservé la propriété d'être une séquence matrice de référence appropriée, pour construire et produire des amorces ciblées vers CT, qui permettent une détection multiplex en temps réel de CT, ou une séquence qui est entièrement complémentaire dudit fragment ou sous-fragment sur la longueur entière dudit fragment ou sous-fragment, dans lequel ledit sous-fragment conservateur est constitué des positions 5580 à 5754 (SEQ ID No: 6) de la séquence CT de SEQ ID No: 1,
- pour la conception d'amorces et sondes MG : un fragment constitué par :
- les positions 1 à 270 (SEQ ID No: 13) de la séquence MG de SEQ ID No: 2, ou un sous-fragment conservateur de celle-ci, qui a conservé la propriété d'être une séquence matrice de référence appropriée, pour construire et produire des amorces ciblées vers MG, qui permettent une détection multiplex en temps réel de MG, ou une séquence qui est entièrement complémentaire dudit fragment ou sous-fragment sur la longueur entière dudit fragment ou sous-fragment, dans lequel ledit sous-fragment conservateur est constitué des positions 2 à 259 (SEQ ID No: 14) de la séquence MG de SEQ ID No: 2,
- les positions 1140 à 1290 (SEQ ID No: 19) de la séquence MG de SEQ ID No: 3, ou un sous-fragment conservateur de celle-ci, qui a conservé la propriété d'être une séquence matrice de référence appropriée, pour construire et produire des amorces ciblées vers MG, qui permettent une détection multiplex en temps réel de MG, ou une séquence qui est entièrement complémentaire dudit fragment ou sous-fragment sur la longueur entière dudit fragment ou sous-fragment, dans lequel ledit sous-fragment conservateur est constitué des positions 1144 à 1283 (SEQ ID No: 20) de la séquence MG de SEQ ID No: 3,
- les positions 1060 à 1250 (SEQ ID No: 25) de la séquence MG de SEQ ID No: 3, ou un sous-fragment conservateur de celle-ci, qui a conservé la propriété d'être une séquence matrice de référence appropriée, pour construire et produire des amorces ciblées vers MG, qui permettent une détection multiplex en temps réel de MG, ou une séquence qui est entièrement complémentaire dudit fragment ou sous-fragment sur la longueur entière dudit fragment ou sous-fragment, dans lequel ledit sous-fragment conservateur est constitué des positions 1064 à 1249 (SEQ ID No: 26) de la séquence MG de SEQ ID No: 3,
- les positions 1520 à 1710 (SEQ ID No: 31) de la séquence MG de SEQ ID No: 3, ou un sous-fragment conservateur de celle-ci, qui a conservé la propriété d'être une séquence matrice de référence appropriée, pour construire et produire des amorces ciblées vers MG, qui permettent une détection multiplex en temps réel de MG, ou une séquence qui est entièrement complémentaire dudit fragment ou sous-fragment sur la longueur entière dudit fragment ou sous-fragment, dans lequel ledit sous-fragment conservateur est constitué des positions 1527 à 1704 (SEQ ID No: 32) de la séquence MG de SEQ ID No: 3,
- les positions 1500 à 1710 (SEQ ID No: 37) de la séquence MG de SEQ ID No: 3, ou un sous-fragment conservateur de celle-ci, qui a conservé la propriété d'être une séquence matrice de référence appropriée, pour construire et produire des amorces ciblées vers MG, qui permettent une détection multiplex en temps réel de MG, ou une séquence qui est entièrement complémentaire dudit fragment ou sous-fragment sur la longueur entière dudit fragment ou sous-fragment, dans lequel ledit sous-fragment conservateur est constitué des positions 1501 à 1704 (SEQ ID No: 38) de la séquence MG de SEQ ID No: 3,
- pour la conception d'amorces et sondes NG : un fragment constitué des positions 101 à 380 (SEQ ID No: 42) de la séquence NG de SEQ ID No: 4, ou un sous-fragment conservateur de celle-ci, qui a conservé la propriété d'être une séquence matrice de référence appropriée, pour construire et produire des amorces ciblées vers NG, qui permettent une détection multiplex en temps réel de NG, ou une séquence qui est entièrement complémentaire dudit fragment ou sous-fragment sur la longueur entière dudit fragment ou sous-fragment, dans lequel ledit sous-fragment conservateur est constitué des positions 114 à 365 (SEQ ID No: 43) de la séquence NG de SEQ ID No: 4.

41. Amorce, qui est spécialement adaptée à la détection de CT et/ou MG et/ou NG dans une amplification multiplex en temps réel, qui est :
un oligonucléotide de 14 à 30 nucléotides, dont la séquence est au moins identique à 80 % à la séquence de la même longueur qui est l'extrémité terminale 5' exacte d'une séquence matrice de référence ou de la séquence qui est entièrement complémentaire de ladite séquence matrice de référence sur la longueur entière de ladite séquence matrice de référence,
dans laquelle ladite séquence matrice de référence est :
- SEQ ID No: 6, ou
- SEQ ID No: 14 ou SEQ ID No: 20 ou SEQ ID No: 26 ou SEQ ID No: 32 ou SEQ ID No: 38 ; ou
- SEQ ID No: 43.

42. Amorce selon la revendication 41, qui est:
- une amorce ciblée vers CT, choisie dans le groupe constitué de SEQ ID No: 7 ; 12, ou
- une amorce ciblée vers MG, choisie dans le groupe constitué de SEQ ID No: 15 ; 18 ; 21 ; 24 ; 27 ; 30 ; 33 ; 36 ; 39, ou
- une amorce ciblée vers NG, choisie dans le groupe constitué de SEQ ID No: 44 ; 47.

43. Système d'amorces qui est spécialement adapté à la détection de CT et/ou MG et/ou NG dans une amplification multiplex en temps réel, qui comprend :
au moins une paire d'oligonucléotides de 14 à 30 nucléotides chacun,
la séquence d'un oligonucléotide de ladite paire étant au moins identique à 80 % à la séquence de la même longueur qui est l'extrémité terminale 5' exacte d'une séquence matrice de référence, et
la séquence de l'autre oligonucléotide de ladite paire étant au moins identique à 80 % à la séquence de la même longueur qui est l'extrémité terminale 5' exacte de la séquence qui est entièrement complémentaire de ladite séquence matrice de référence sur la longueur entière de ladite séquence matrice de référence,
dans lequel ladite séquence matrice de référence est :
- SEQ ID No: 6, ou
- SEQ ID No: 14 ou SEQ ID No: 20 ou SEQ ID No: 26 ou SEQ ID No: 32 ou SEQ ID No: 38 ; ou
- SEQ ID No: 43.

44. Système d'amorces selon la revendication 43, qui comprend :
- au moins une paire d'amorces ciblées vers CT de SEQ ID No: 7 et SEQ ID No: 12, et/ou
- au moins une paire d'amorces ciblées vers MG choisie parmi les paires suivantes : SEQ ID No: 15 et 18 ; SEQ ID No: 21 et 24 ; SEQ ID No: 27 et 30 ; SEQ ID No: 33 et 36 ; SEQ ID No: 39 et 36, et/ou
- au moins une paire d'amorces ciblées vers NG de SEQ ID No: 44 et 47.

45. Système d'amorces selon la revendication 43 ou 44, qui comprend trois desdites paires d'oligonucléotides, et dans lequel ladite séquence matrice de référence est :
- pour l'une desdites trois paires : SEQ ID No: 6 ;
- pour une autre desdites trois paires : SEQ ID No: 14 ou SEQ ID No: 20 ou SEQ ID No: 26 ou SEQ ID No: 32 ou SEQ ID No: 38 ;
- pour encore une autre desdites trois paires : SEQ ID No: 43.

46. Sonde, qui est spécialement adaptée à la détection de CT et/ou MG et/ou NG dans une amplification multiplex en temps réel, qui est :
- une sonde spécifique de CT de SEQ ID No: 8 ; ou 10, ou qui consiste en une séquence qui est entièrement complémentaire de cette séquence SEQ ID, sur la longueur entière de cette séquence SEQ ID,
- une sonde spécifique de MG de SEQ ID No: 16 ; 22 ; 28 ; 34 ou 40, ou qui consiste en une séquence qui est entièrement complémentaire de cette séquence SEQ ID, sur la longueur entière de cette séquence SEQ ID,
- une sonde spécifique de NG de SEQ ID No: 45, ou qui consiste en une séquence qui est entièrement complémentaire de cette séquence SEQ ID, sur la longueur entière de cette séquence SEQ ID,
ladite sonde étant éventuellement liée à au moins une marque de détection et/ou au moins un bras balise.

47. Sonde selon la revendication 46, qui est :
- une sonde spécifique de CT de SEQ ID No: 9 ou 11, ou qui consiste en une séquence qui est entièrement complémentaire de cette séquence SEQ ID, sur la longueur entière de cette séquence SEQ ID,
- une sonde spécifique de MG de SEQ ID No: 17 ; 23 ; 29 ; 35 ou 41, ou qui consiste en une séquence qui est entièrement complémentaire de cette séquence SEQ ID, sur la longueur entière de cette séquence SEQ ID,
- une sonde spécifique de NG de SEQ ID No: 45 ou 46, ou qui consiste en une séquence qui est entièrement complémentaire de cette séquence SEQ ID, sur la longueur entière de cette séquence SEQ ID.

48. Système d'amorces et de sondes, qui est spécialement adapté à la détection de CT et/ou MG et/ou NG dans une amplification multiplex en temps réel, qui comprend au moins un système d'amorces selon l'une quelconque des revendications 43 à 45, et au moins une sonde selon l'une quelconque des revendications 46 à 47.

49. Composition d'amplification, comprenant au moins un amplicon pouvant être obtenu par la mise en oeuvre du procédé selon l'une quelconque des revendications 1 à 39 sur un échantillon contenant CT et/ou MG et/ou NG, ou par l'amplification d'au moins un acide nucléique provenant d'un échantillon contenant CT et/ou MG et/ou NG au moyen d'au moins un système d'amorces selon l'une quelconque des revendications 43 à 45, et comprenant en outre :
- les amorces ciblées vers CT telles que définies selon la revendication 1 et les amorces ciblées vers MG telles que définies selon la revendication 1 et les amorces ciblées vers NG telles que définies selon la revendication 1 ; ou
- le système d'amorces selon la revendication 45.

50. Composition pour une amplification selon la revendication 49, qui comprend trois amplicons, dans laquelle lesdits trois amplicons peuvent être obtenus :
- par la mise en oeuvre du procédé selon l'une quelconque des revendications 1 à 39 sur un échantillon contenant CT et MG et NG, ou
- par l'amplification d'au moins un acide nucléique de CT, et d'au moins un acide nucléique de MG, et d'au moins un acide nucléique de NG, au moyen du système d'amorces selon la revendication 45.

51. Kit pour le diagnostic d'une infection à CT et/ou MG et/ou NG, qui comprend :
- au moins un système d'amorces selon l'une quelconque des revendications 43 à 45, et/ou
- au moins une sonde selon l'une quelconque des revendications 46 à 47,
- éventuellement, des instructions pour son utilisation et/ou des nucléotides.

52. Kit selon la revendication 50, qui comprend en outre l'oligonucléotide de Contrôle Interne (CI) de SEQ ID No: 48, et/ou la paire d'amorces de CI de SEQ ID No: 49 et No: 52, et/ou la sonde de CI de SEQ ID No: 50, et/ou la sonde de CI de SEQ ID No: 51.
